# DEMANDE DE BREVET EUROPEEN

(11) **EP 1 797 868 A1**
(43) Date de publication de la demande: **20.06.2007**
(21) Numéro de dépôt: 06124534.6
(22) Date de dépôt: 22.11.2006
(51) Int. Cl.: A61K 8/87, A61Q 19/00, A61Q 5/00, A61Q 1/00

(54) **Composition cosmétique comprenant un copolymère (thio)uréthane/ (thio)urée capable de former au moins 3 liaisons hydrogène, et procédé de traitement cosmétique**

(30) Priorité: 16.12.2005 FR 0553913
(71) Demandeur: L'Oréal, 75008 Paris (FR)
(72) Inventeur: Mougin, Nathalie, 75011 Paris (FR); Schultze, Xavier, 77340 Pontault-Combault (FR); Chodorowski-Kimmes, Sandrine, 60300, Senlins (FR)
(74) Mandataire: Dodin, Catherine

(57) **Abrégé**

La présente demande concerne une composition cosmétique comprenant, dans un milieu physiologiquement acceptable, un copolymère ayant un squelette polymérique (thio)uréthane/ (thio)urée, résultant de la réaction :
- d'un premier monomère comportant au moins deux groupes polymérisables, identiques ou différents, choisis parmi -N=C=O et -N=C=S;
- d'un monomère (b1) comportant au moins deux groupes polymérisables à hydrogène labile,
- d'un monomère (b2), différent du monomère (b1), comportant au moins deux groupes polymérisables à hydrogène labile,
- l'un au moins de ces monomères comprenant au moins un groupe de jonction (A), capable de former au moins 3 liaisons H,

L'invention concerne également un procédé de traitement cosmétique des matières kératiniques, comprenant l'application sur lesdites matières d'une composition cosmétique telle que définie ci-dessus.

## Description

La présente invention concerne de manière générale une composition cosmétique de soin, de traitement et/ou de maquillage des matières kératiniques, présentant une persistance accrue d'au moins un effet cosmétique et/ou de soin apporté après application par la composition.

En cosmétique, en effet, on cherche à obtenir un dépôt sur les cheveux, la peau, les cils et les ongles, souvent filmogène et apportant par exemple soit la mise en forme de la coiffure (cheveux); soit de la couleur (cheveux, maquillage); soit de la brillance ou de la brillance et de la couleur (rouge à lèvres, mascaras, eye-liners et vernis à ongles); soit de la couleur et de la matité (fond de teint par exemple); soit du soin, si le dépôt contient un actif de soin tel qu'un hydratant, une protection contre les UV s'il contient des filtres solaires.
On cherche donc pour une persistance de l'effet apporté (mise en forme, couleur, brillance, matité, soin), une grande rémanence du dépôt cosmétique qui doit, en particulier résister aux agressions mécaniques telles que frottements, transferts par contact d'un autre objet; résister à l'eau, à l'humidité, à la sueur, aux larmes, à la pluie, et résister au sébum et aux huiles; tout en restant facilement démaquillable.

Ceci est particulièrement vrai en maquillage pour les rouges à lèvres où l'on recherche la tenue prolongée de la couleur et de la brillance, et le non transfert de la couleur; pour les vernis à ongles où l'on souhaite la brillance et la tenue du film; pour les fonds de teint, fards à paupière et poudres où l'on recherche également la tenue de la couleur apportée, en maintenant le plus longtemps possible la matité de la teinte initiale malgré la sécrétion de sébum et de sueur (la perte de la matité étant due la plupart du temps à l'évolution de la couleur apportée sous l'effet de la sécrétion du sébum et/ou de la sueur, qui font briller la peau), ainsi que le non transfert.
En coiffage, on recherche un maintien de la mise en forme de la chevelure, durant toute la journée.

En cosmétique, les polymères classiquement utilisés sont souvent des copolymères, les comonomères étant reliés les uns aux autres par des liaisons covalentes.
Il est d'usage courant d'utiliser dans de nombreuses formulations cosmétiques, et notamment dans différents produits de maquillage tels que des vernis à ongles, des mascaras et des eye-liners, à titre de résine filmogène, des polyuréthanes. La résine, pour être satisfaisante, doit non seulement présenter de bonnes propriétés filmogènes, mais également de bonnes propriétés de rémanence, c'est-à-dire être difficilement éliminable de son support par simple lavage à l'eau.
En particulier, dans le cas des compositions de maquillage, il serait extrêmement souhaitable de disposer de polymères de nature chimique différente ou identique, facilement solubles ou dispersibles dans les huiles, les solvants cosmétiques et/ou les milieux aqueux, à concentration variable. Il serait également souhaitable que ces compositions, présentant une persistance accrue des effets apportés (notamment couleur, brillance, matité, soin), favorisent l'adhésion sur les matières kératiniques tout en facilitant le démaquillage. Ceci permettrait notamment de disposer de compositions colorées ayant une constance accrue du coloris c'est-à-dire dont le coloris n'est pas détérioré ou affadi par les agressions mécaniques, l'eau, la sueur, les larmes, la pluie, le sébum et les huiles, tout en conservant leurs autres propriétés telles que la tenue de la couleur, la brillance, la matité et le soin.
En coiffage plus particulièrement, la composition doit permettre la mise en forme du cheveu avec une bonne tenue dans le temps et dans les conditions de température et d'humidité de la vie quotidienne.

Pour résoudre ces problèmes, on a proposé l'utilisation dans des compositions cosmétiques de polymères capables d'induire entre eux, une réticulation physique et, notamment des polymères comportant des groupes susceptibles de former des liaisons hydrogène (liaisons H).
Ainsi, la demande de brevet international WO02/098377 décrit une composition cosmétique de soin, de traitement, de maquillage des matières kératiniques comprenant, dans un milieu physiologiquement acceptable, un polymère linéaire, ramifié, cyclique ou dendrimère comportant un squelette polymérique et un groupe de jonction susceptible de former des liaisons hydrogène. Les squelettes polymériques peuvent comporter des motifs poly(oxyde d'éthylène) (POE).
Egalement, la demande de brevet international WO03/032929 décrit une composition de traitement capillaire contenant un polymère comprenant au moins deux groupes susceptibles de former des liaisons hydrogène.
Il a été décrit dans la demande de brevet EP418469, des compositions de vernis à ongles contenant à titre de résine filmogène des dispersions aqueuses de polyuréthanes aliphatiques.
Il a également été décrit dans la demande de brevet EP391322, des vernis à ongles contenant une dispersion aqueuse d'un polyuréthane et/ou d'un copolymère polyuréthane.

L'utilisation de ces résines ne permet pas cependant de conduire à des compositions présentant de bonnes propriétés cosmétiques du fait notamment d'un manque d'adhésion sur le support.
La mise en place de législations visant à diminuer la teneur en solvants volatils, notamment dans les compositions cosmétiques, incite les formulateurs, en particulier des compositions cosmétiques, à utiliser les milieux aqueux, voire l'eau, comme solvant préférentiel pour la formulation des compositions.

Véhiculer dans les milieux aqueux, notamment dans l'eau ou dans un milieu comportant de l'eau, des polymères tout en conservant les propriétés visées des compositions, et, plus particulièrement, celles qu'induisent lesdits polymères ajoutés, s'avère particulièrement difficile. Ceci est particulièrement le cas dans le domaine du coiffage, où un apport suffisant de coiffant par les polymères véhiculés par le milieu aqueux est nécessaire, tout en maintenant une bonne résistance dans le temps de l'effet.
Véhiculer dans les milieux non aqueux, notamment solvants organiques et huiles cosmétiques, des polymères s'avère également difficile, en particulier dans le domaine des produits de maquillage de type fond de teint ou rouge à lèvres, et également de type vernis à ongles.
Par l'expression "véhiculer dans un milieu", aqueux ou non aqueux, un polymère, on entend selon la présente invention que ledit polymère est soluble et/ou dispersible dans ledit milieu, à 25°C, à une concentration d'au moins 1% en poids.

Il existe donc le besoin de compositions cosmétiques qui conduisent après application sur les matières kératiniques à des dépôts permettant de concilier la persistance de l'effet cosmétique et/ou de soin, une bonne adhésion de la composition sur les matières kératiniques et un démaquillage rapide et complet, et qui comprennent des polymères qui sont aisément véhiculables dans le milieu physiologiquement acceptable de la composition, particulièrement dans un milieu comportant de l'eau et pouvant également comporter de l'huile et/ou un solvant organique cosmétique et notamment dans un milieu à base d'eau uniquement.

L'invention a donc pour but de proposer une composition cosmétique, notamment de soin, de traitement cosmétique et/ou de maquillage des matières kératiniques, permettant de pallier les inconvénients des formulations traditionnelles.

La demanderesse a trouvé de manière surprenante et inattendue qu'une composition cosmétique comprenant des copolymères particuliers, ayant un squelette polymérique poly(thio)uréthane, poly(thio)urée ou copoly(thio)uréthane / (thio)urée [ci-après désignée globalement par copolymères (thio)uréthane / (thio)urée] et comprenant au moins un groupe de jonction lié audit squelette et capable d'interagir avec tout groupe de jonction partenaire via la formation d'au moins 3 liaisons H, présentait de bonnes propriétés filmogènes, et permettait d'obtenir des films possédant une bonne solidité.

L'invention a donc pour objet une composition cosmétique comprenant, dans un milieu physiologiquement acceptable, un copolymère ayant un squelette polymérique (thio)uréthane/ (thio)urée, résultant de la réaction :
- d'au moins un premier monomère (a) comportant au moins deux groupes polymérisables, identiques ou différents, choisis parmi -N=C=O et -N=C=S, et/ou leur forme activée ou bloquée;
- d'au moins un monomère (b1), comportant au moins deux groupes polymérisables à hydrogène labile, identiques ou différents, choisis parmi -OH, -SH, - NH₂ et -NHR, avec R représentant un groupe alkyle en C₁-C₆ et
- d'au moins un monomère (b2), différent du monomère (b1), comportant au moins deux groupes polymérisables à hydrogène labile, identiques ou différents, choisis parmi -OH, -SH, -NH₂ et -NHR, avec R représentant un groupe alkyle en C₁-C₆; et
- l'un au moins des monomères (a), (b1) et/ou (b2) comprenant au moins un groupe de jonction (A), capable de former au moins 3 liaisons H, de préférence au moins 4 liaisons H et plus préférentiellement 4 liaisons H.

Les compositions selon l'invention présentent une bonne adhésion sur les matières kératiniques, et permettent un démaquillage rapide, total et sélectif. Elles ne sont pas collantes.
En particulier, les compositions selon l'invention peuvent être filmogènes, et conduire après application à la filmification des compositions au cours du séchage.
Par ailleurs, les copolymères selon l'invention sont facilement véhiculés dans l'eau et les solvants organiques tels que les esters notamment d'alkyle tels que les acétates d'alkyle, les alcools, notamment l'éthanol, les huiles carbonées et/ou siliconées, et leurs mélanges, c'est-à-dire dans les milieux aqueux et/ou huileux et/ou solvant organique usuellement employés en cosmétique.
En outre, le polymère selon l'invention permet d'obtenir une composition cosmétique dont l'élimination est facilitée, par rapport aux compositions de l'art antérieur, notamment décrites dans WO02/98377. Ceci est particulièrement important dans le cas des compositions capillaires, qui doivent être facilement éliminables au shampoing.
Enfin, on a constaté que les compositions comprenant des polymères selon l'invention pouvaient présenter une faible viscosité, bien que comprenant des quantités importantes de polymères; ceci est notamment vrai pour les compositions en milieu aqueux. Ces compositions conservent une bonne sprayabilité dans les formulations de type flacon-pompe, spray ou aérosol, et permettaient l'obtention d'un dépôt filmifiable sur le support.

De plus, les copolymères selon l'invention permettent la préparation de dispersions ou solutions aqueuses très fluides et de plus, particulièrement stables sans utilisation de tensioactifs additionnels, dans le cas particulier où ils comportent en outre des groupes fonctionnels ionisables.

Par "squelette polymérique" aussi appelé -POL-, on entend au sens de l'invention, un copolymère comportant au moins trois motifs de répétition, identiques ou différents, liés de manière covalente et pouvant se répéter plusieurs fois dans le squelette. Ledit copolymère peut être linéaire, cyclique, ramifié, notamment en étoile, hyperbranché, dendrimère ou greffé, ou bien réticulé; il peut s'agir d'un copolymère statistique, alterné, séquencé ou autre, de préférence séquencé.

Par "comportant au moins trois motifs de répétition", on entend selon la présente invention, une unité constitutive d'un polymère résultant de la copolymérisation d'au moins trois motifs monomères ou oligomères identiques ou différents.

Par "groupe de jonction" aussi appelé A, on entend au sens de l'invention, toute entité ou groupe fonctionnel comportant des groupes donneurs ou accepteurs de liaisons H, et capable d'établir au moins 3 liaisons H, de préférence au moins 4 liaisons H et plus préférentiellement 4 liaisons H, avec un groupe de jonction partenaires, de nature chimique identique ou non.
Les groupes de jonction (A) peuvent être latéraux au squelette polymérique (en ramification latérale), et/ou localisés aux extrémités dudit squelette polymérique, et/ou intégrés dans la chaîne formant ledit squelette polymérique. Ils peuvent être répartis de façon aléatoire ou contrôlée dans la chaîne.

Par " groupe de jonction partenaire", on entend au sens de l'invention, tout groupe de jonction d'un copolymère selon l'invention pouvant établir des liaisons H avec un ou plusieurs groupes de jonction d'un même ou d'un autre copolymère selon l'invention. Les groupes de jonction peuvent être de nature chimique identique ou différente. S'ils sont identiques, ils peuvent alors établir des liaisons H entre eux et sont alors appelés groupes de jonction autocomplémentaires. S'ils sont différents, ils sont choisis de telle façon qu'ils soient complémentaires vis-à-vis des interactions H.
L'utilisation de tels copolymères dans une composition cosmétique conduit, après application de cette composition sur les matières kératiniques, à la formation d'un polymère supramoléculaire.

Par "polymère supramoléculaire", on entend au sens de l'invention, une chaîne
ou un réseau polymérique formé de l'assemblage d'un copolymère selon l'invention avec au moins un autre copolymère selon l'invention, identique ou différent, chaque assemblage comprenant au moins une paire de groupes de jonction appariés, identiques ou différents.

Par "paire de groupes de jonction appariés", on entend au sens de l'invention, deux groupes de jonction dont chacun peut être porté ou non par un même copolymère selon l'invention, les deux groupes étant reliés ensemble via au moins trois liaisons H, de préférence au moins 4 liaisons H et plus préférentiellement 4 liaisons H. Les copolymères de l'invention peuvent s'associer par appariement de leurs groupes de jonction avec un groupe de jonction identique chimiquement (ou auto-complémentaire) ou différent chimiquement.

Ainsi le polymère supramoléculaire présentera des points de réticulation physique assurés par les liaisons H entre ces paires de groupes de jonction. La réticulation physique assurera le maintien et la persistance de l'effet cosmétique et/ou de soin de manière analogue à la réticulation chimique, tout en permettant la réversibilité, c'est-à-dire la possibilité d'éliminer totalement le dépôt soit par un démaquillant spécifique, soit par la température, soit par tout autre moyen, ce que ne permet pas la réticulation chimique.

On a constaté que l'utilisation de copolymères selon l'invention, comportant au moins un groupe de jonction, dans une composition cosmétique peut conduire, après application de la composition sur les matières kératiniques, soit à la formation d'un copolymère supramoléculaire sous forme de réseau tridimensionnel réticulé physiquement, se présentant généralement sous forme de film, et ayant une très bonne résistance mécanique, soit à la formation d'un copolymère supramoléculaire sous forme d'une longue chaîne polymère, généralement de masse moléculaire élevée résultant de la connection physique des copolymères de l'invention.

Les copolymères multifonctionnels (possédant plusieurs groupes de jonction) offrent une meilleure probabilité de réticulation, ce qui permet d'utiliser une quantité de polymère plus faible pour une même efficacité de réticulation, et surtout d'accroître les propriétés mécaniques des produits.

Lorsque le copolymère est formulé en présence de solvants volatils, il est possible d'obtenir la filmification de la composition au cours du séchage, lorsque la structure du copolymère le permet.
Par « filmification » on entend, au sens de l'invention la formation d'un dépôt continu se présentant sous forme de films et possédant des propriétés mécaniques analogues à celles de copolymères à haut poids moléculaire ou de réseaux réticulés chimiquement.

Par "radical divalent carboné", on entend selon la présente invention des radicaux comprenant des atomes de carbone et d'hydrogène, et éventuellement un ou plusieurs hétéroatomes; de préférence, les radicaux carbonés divalents sont des radicaux divalents aliphatiques saturés (alkyles) ou non, linéaires ou ramifiés, cycliques ou non, aromatiques ou non, comprenant de préférence 1 à 40 atomes de carbone, substitués ou non, et pouvant éventuellement comporter un ou plusieurs hétéroatomes choisis parmi : O, S, N, P, Si et F, de préférence O, S et N et des combinaisons de ces radicaux.

### Structure générale des copolymères selon l'invention

Les copolymères selon l'invention résultent de la réaction d'au moins un monomère (a), d'au moins un monomère (b1) et d'au moins un monomère (b2), l'un au moins de ces monomères comprenant au moins un groupe de jonction (A).

De préférence, l'un au moins des monomères (a), (b1) et/ou (b2) comprend au moins deux groupes de jonction (A), capables de former au moins 3 liaisons H, de préférence au moins 4 liaisons H et plus préférentiellement 4 liaisons H.

Au final, les copolymères selon l'invention peuvent être schématisés par la formule générale suivante : POL-(A)ᵢ dans laquelle :
- POL désigne le squelette polymérique poly(thio)uréthane / (thio)urée,
- A désigne un groupe de jonction lié audit squelette polymérique et capable d'établir des liaisons H avec un ou plusieurs groupes de jonction partenaires, de nature chimique identique ou différente, chaque appariement d'un groupe de jonction faisant intervenir au moins trois liaisons H, de préférence au moins 4 liaisons H, préférentiellement 4 liaisons H, et
- i est un entier supérieur ou égal à 1, de préférence supérieur ou égal à 2.

Les copolymères selon l'invention peuvent avantageusement se présenter sous l'une des structures suivantes:
1. copolymère linéaire et fonctionnalisé en α, ω avec des groupes de jonction (A);
2. copolymère linéaire comportant plus de deux groupes de jonction, situés dans la chaîne et/ou à l'une ou aux deux extrémités et/ou en ramifications;
3. copolymère ramifié avec des groupes de jonction intégrés dans la chaîne et/ou en ramification et/ou à l'une ou aux deux extrémités;

Il est clair que les copolymères selon l'invention peuvent présenter une seule de ces structures, ou un mélange de ces structures, en toutes proportions.

### Définition générale des groupes de jonction A

Selon la présente invention, un groupe de jonction A est un groupe chimique capable de former au moins 3 liaisons H, de préférence au moins 4 liaisons H, et mieux quatre liaisons H, et comportant de préférence au moins 3 hétéroatomes, identiques ou différents, de préférence 4 hétéroatomes, identiques ou différents, choisis dans le groupe constitué de O, N, S, P et F, de préférence parmi O, S et N.
Ces groupes de jonction peuvent comprendre par exemple au moins 3 groupes fonctionnels, de préférence au moins 4, choisis parmi : 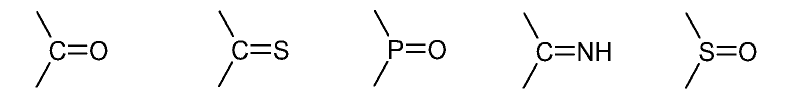 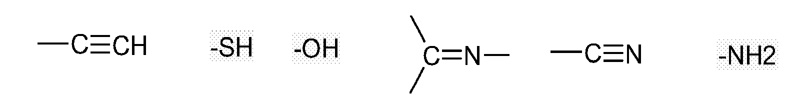 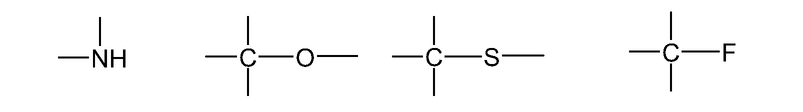 Ces groupes fonctionnels peuvent être classés en deux catégories :
- les groupes fonctionnels donneurs de liaisons H tels que les groupes : 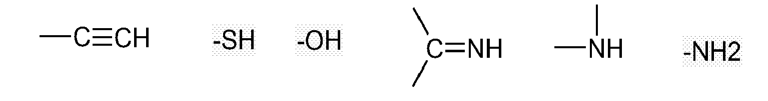
- et les groupes fonctionnels accepteurs de liaisons H tels que les groupes : 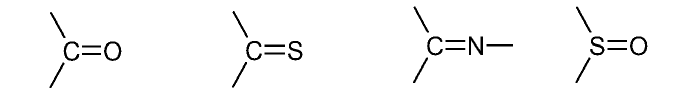 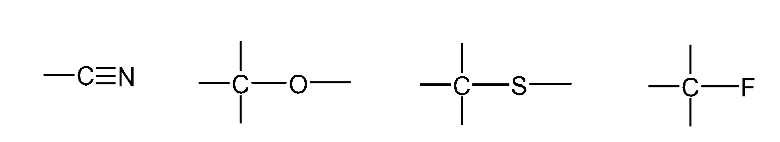
Les groupes de jonction A forment un élément structural de base comportant au moins 3 groupes, de préférence au moins 4 groupes fonctionnels et plus préférentiellement 4 groupes fonctionnels, capables d'établir des liaisons H. Les éléments structuraux de base capables d'établir 3 ou 4 liaisons H peuvent être schématisés de la manière suivante : 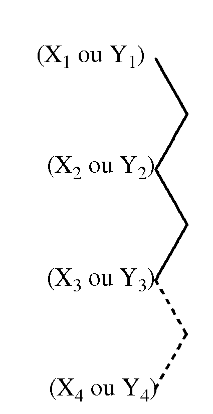 où Xᵢ (i entier naturel) est un groupe fonctionnel accepteur de liaisons H et Yᵢ est un groupe fonctionnel donneur de liaisons H.
Ainsi, chaque élément structural doit pouvoir établir des liaisons H avec un ou plusieurs éléments structuraux partenaires, identiques (c'est-à-dire autocomplémentaires) ou différents, de telle sorte que chaque appariement de deux éléments structuraux partenaires se fasse par formation d'au moins trois liaisons H, de préférence au moins quatre liaisons H, et plus préférentiellement 4 liaisons H.
Un accepteur de protons X s'appariera avec un donneur de protons Y.
Plusieurs possibilités sont offertes, par exemple:
Appariement de :
   XXXX avec YYYY ;
   XXXY avec YYYX ;
   XXYX avec YYXY ;
   XYYX avec YXXY ;
   XXYY avec YYXX auto-complémentaire ou non ;
   XYXY avec YXYX auto-complémentaire ou non.
De préférence les groupes de jonction A peuvent établir 4 liaisons H avec un groupe partenaire identique (ou autocomplémentaire) parmi lesquelles 2 liaisons donneur (par exemple NH) et 2 liaisons accepteur (par exemple CO et - C=N-).
De préférence, les groupes de jonction comportent des cycles à 5 ou 6 atomes (cycles aromatiques ou hétérocycles insaturés) très souvent constitués d'atomes de C et/ou N et avec des doubles liaisons conjuguées pour stabiliser et diriger les interactions H.
De préférence encore, les groupes de jonction sont engagés dans des cycles à 6 atomes comprenant des C et/ou N et avec des doubles liaisons conjuguées pour stabiliser et diriger les interactions H.

Dans un mode de réalisation préféré, les groupes de jonction (A) capables de former 3 ou 4 liaisons H peuvent être choisis parmi les familles suivantes, étant entendu que toutes les formes tautomériques sont inclues :
- (i) les aminopyrimidones de formule : 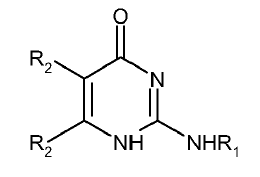
- (ii) les ureïdopyrimidones de formule : 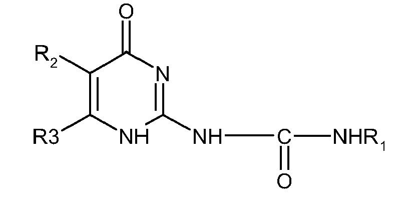
- (iii) les acylaminopyridines et notamment :

- les monoacylaminopyridines de structure : 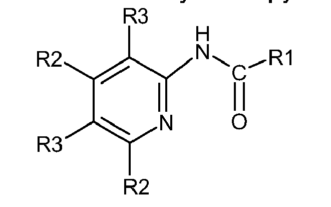
- les di(acylamino)pyridines et plus particulièrement les 2,6-di(acylamino)pyridines de structure : 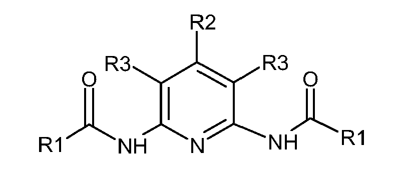
- (iv) les aminopyrimidines, et notamment :

- les composés aminopyrimidines : 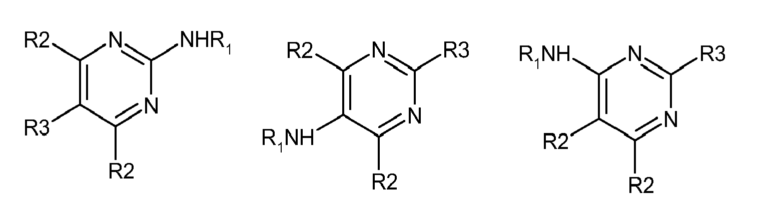
- les composés diaminopyrimidines de structure : 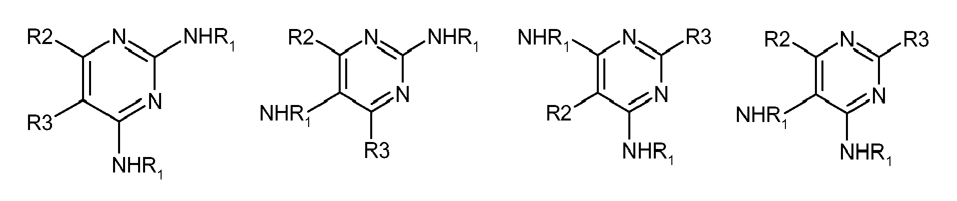
- les composés triaminopyrimidines;
- (v) les uréïdotriazines, et notamment les mono-, di- et tri-uréïdotriazines, et en particulier les uréïdoaminotriazines de structure : 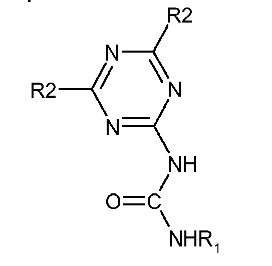
- (vi) les (acylamino)triazines, et notamment les mono-, di- et tri-acylamino triazines, éventuellement amino (mono-, di- ou tri-amino) et en particulier :

- les di(acylamino)triazines de structure : 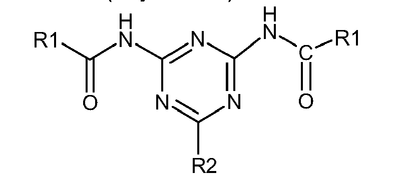
- les acylamino, amino-triazines, (mono ou di- acylamino, et mono- ou di-amino) et notamment les composés de structure : 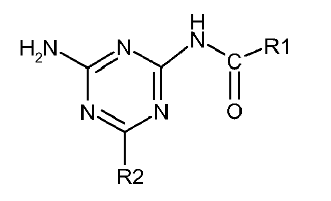
- les acylaminotriazines de structure : 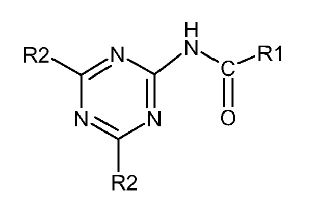
- les tri-acylamino triazines,
- (vii) les aminotriazines et notamment :

- les monoaminotriazines,
- les 2,6-diamino-s-triazines de structure : 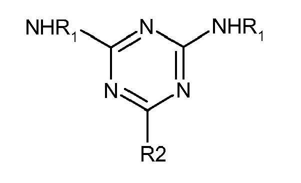
- les composés triamino-s-triazines de structure : 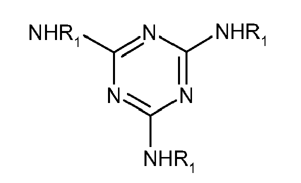
- (viii) les acylaminotriazoles de structure : 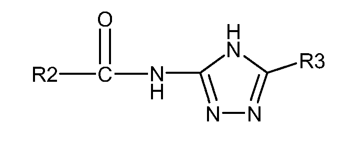
- (ix) les composés de la famille de l'acide urazoyl benzoïque de structure : 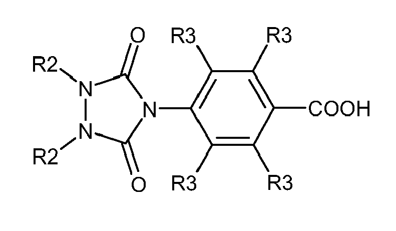
- (x) les phtalhydrazides de structure : 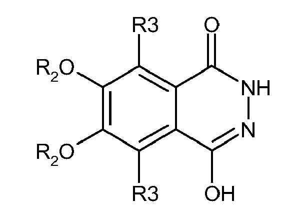
- (xi) les uraciles de structure : 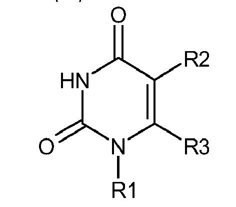
- (xii) les thymines de structure : 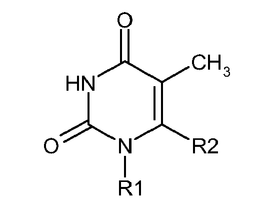
- (xiii) les succinimides de structure : 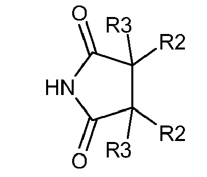
- (xiv) les glutarimides de structure : 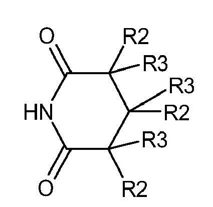
- (xv) les composés de la famille de l'acide cyanurique de structure : 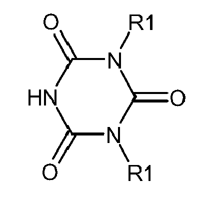
- (xvi) les maléimides : 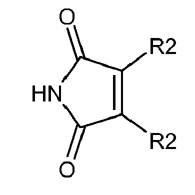
- (xvii) les composés de la famille de l'acide barbiturique, de structure : 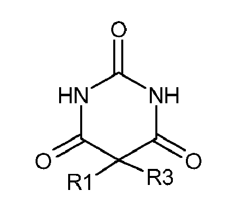
- (xviii) les composés de structure : 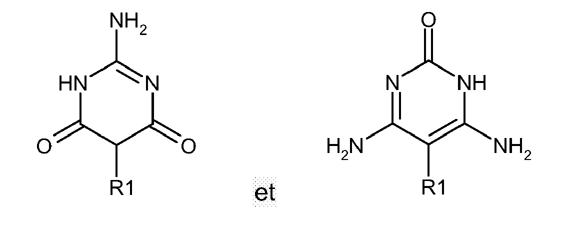
- (xix) les composés de la famille de l'acide triméllitique, de formule : 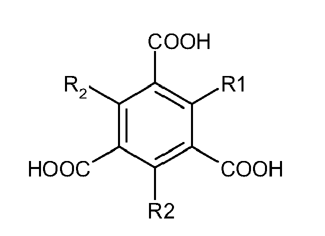
- (xx) les uréïdopyridines, notamment les mono- ou di-uréïdopyridines, et en particulier ceux de formule : 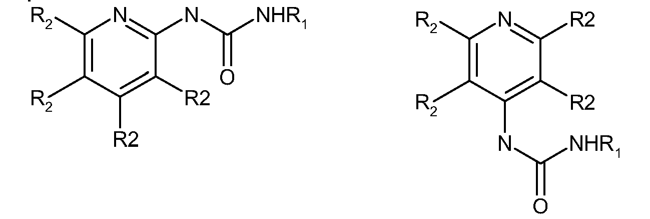 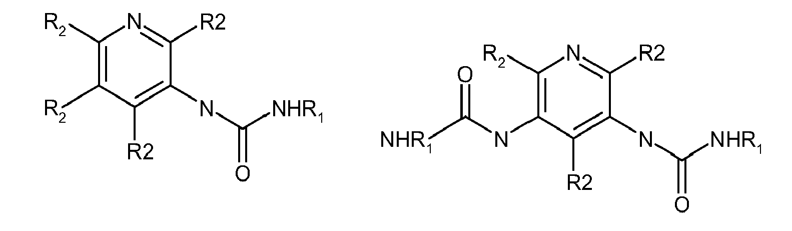 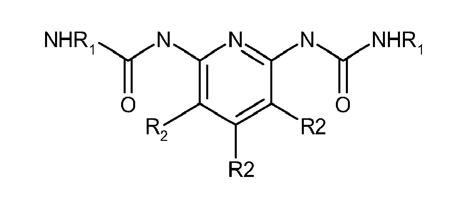
- (xxi) les carbamoylpyridines, de formule : 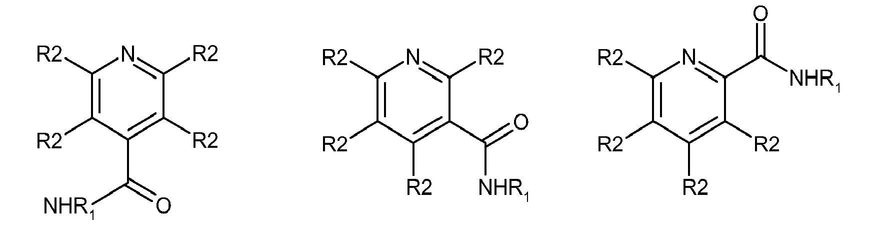
- (xxii) les adénines de formule : 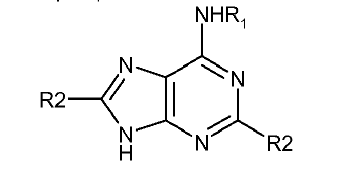
- (xxiii) les guanines de formule : 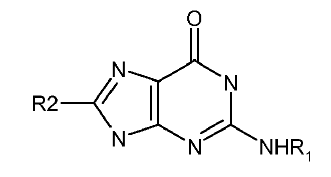
- (xxiv) les cytidines de formule :

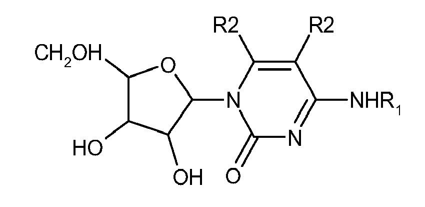

Dans l'ensemble de ces formules, la signification des radicaux est la suivante :
- (a) les radicaux R¹, identiques ou différents, représentent H, halogène et/ou un groupe carboné (notamment alkyle) monovalent, linéaire, ramifié ou cyclique, en C₁-C₆₀₀₀, saturé ou non, éventuellement aromatique, pouvant contenir un ou plusieurs hétéroatomes tels que O, S, N, P, Cl, Br, Cl, Br, F; ou une combinaison de ces significations.
   Le radical R¹ peut notamment être un groupe cycloalkyle en C₄-C₁₂; un groupe alkyle linéaire ou ramifié en C₁-C₃₀ ou un groupe aryle en C₄-C₁₂; éventuellement substitués par une fonction amino, ester et/ou hydroxy.
   De préférence, R₁ est un groupement : -C₄H₉, -phényle ; 1,4-nitrophényle, ou 1,2-éthylène, 1,6-héxylène, 1,4-butylène, 1,6-(2,4,4-triméthylhexylène), 1,4-(4-méthylpentylène), 1,5-(5-méthylhexylène), 1,6-(6-méthylheptylène), 1,5-(2,2,5-triméthylhexylène) ou 1,7-(3,7-diméthyloctylène); -isophorone-, 4,4'-méthylène biscyclohexylène, tolylène , 2-méthyl-1,3-phénylène, 4-méthyl-1,3-phénylène, 4,4-biphénylèneméthylène,
   et de préférence : -isophorone-, -(CH₂)₂-, -(CH₂)₆-, -CH₂CH(CH₃)-CH₂-C(CH₃)₂-CH₂-CH₂, 4,4'-méthylène biscyclohexylène, 2-méthyl-1,3-phénylène .
- (b) les radicaux R2, identiques ou différents au sein d'une même formule, représentent H, halogène (-BR, -Cl, -F), -OH, -N(R)2 (avec R étant H ou un radical alkyle, linéaire ou ramifié en C1-C12, de préférence en C1-C4 et mieux un radical méthyle ou éthyle); ou un groupe hydrocarboné monovalent, linéaire, ramifié ou cyclique, en C1-C6000, saturé ou non, éventuellement aromatique, pouvant contenir un ou plusieurs hétéroatomes tels que O, S, N, P, F; ou une combinaison de ces significations;
   Les radicaux R² peuvent notamment être H, CN, NH₂ ou bien :
   - un groupe alkyle en C₁-C₃₀ ;
   - un groupe cycloalkyle en C₄-C₁₂ ;
   - un groupe aryle en C₄-C₁₂ ;
   - un groupe aryl(C₄-C₁₂) alkyle en C₁-C₃₀
   - un groupe alcoxy en C₁-C₄;
   - un groupe arylalcoxy, en particulier un groupe aryle (C₁-C₄) alcoxy ;
   - un hétérocycle en C₄-C₁₂
   - un groupe thioalcoxy,
   - un groupe sulfoxy,
   ou leurs mélanges, ces groupes étant éventuellement substitués par une fonction amino, ester et/ou hydroxy.
   De préférence R² représente H, CH₃, C₁₃H₂₇, C₇H₁₅ ou phényle.
- (c) les radicaux R³, identiques ou différents au sein d'une même formule, représentent H ou un groupe hydrocarboné monovalent, linéaire, ramifié ou cyclique, en C₁-C₆₀₀₀, saturé ou non, éventuellement aromatique, pouvant contenir un ou plusieurs hétéroatomes tels que O, S, N, P, F; ou une combinaison de ces significations;
   Le radical R³ peut notamment être un groupe cycloalkyle en C₄-C₁₂; un groupe alkyle linéaire ou ramifié en C₁-C₃₀ ou un groupe aryle en C₄-C₁₂; éventuellement substitués par une fonction amino, ester et/ou hydroxy.

Dans l'ensemble de ces formules, il est bien entendu qu'au moins un, notamment un ou deux, des groupes R¹ et/ou R² est le point d'accroche du groupe de jonction A sur le squelette polymérique -POL-.
De préférence, ledit point d'accroche est porté par R¹ et/ou R² et de préférence lorsque le point d'accroche est unique, il est porté par le groupe R¹.

Les groupes de jonction (A) peuvent notamment être choisis parmi:
(a) les groupes de jonction (A) complémentaires et identiques c'est-à-dire autocomplémentaires, et notamment :
   - les aminopyrimidones, les uréïdopyrimidones,
   - les composés de la famille de l'acide triméllitique, ou de l'acide urazoyl benzoïque,
   - les acylaminopyridines, les uréïdopyridines, les carbamoylpyridines,
   - les acylaminotriazines, les uréidotriazines et notamment les uréïdoaminotriazines, les diamino-triazines,
   - les acylaminotriazoles,
   - les phtalhydrazides,
   - les composés de formule : 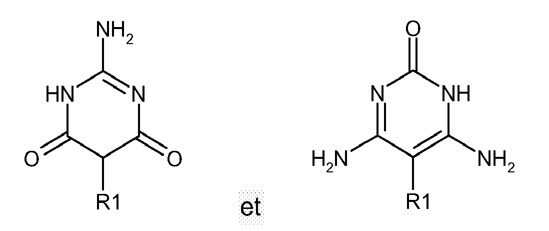
   dans lesquelles R¹ est H ou un groupe hydrocarboné monovalent, linéaire, ramifié ou cyclique, en C₁-C₆₀₀₀, saturé ou non, éventuellement aromatique, pouvant contenir un ou plusieurs hétéroatomes tels que O, S, N, P, F.
(b) les groupes de jonction (A) complémentaires mais différents, et notamment:
   - adénine complémentaire de guanine,
   - cytidine complémentaire de thymine,
   - triamino-s-triazine complémentaire de uracile ou de succinimide ou de glutarimide ou d'acide cyanurique ou de thymine ou de maléimide ou de (di)aminopyrimidine ou d'acide barbiturique;
   - acylamino-amino-s-triazine complémentaire de uracile ou de succinimide ou de glutarimide ou d'acide cyanurique ou de thymine ou de maléimide ou de (di)aminopyrimidine ou d'acide barbiturique.

De manière préférée, les groupes de jonction A sont choisis parmi les groupes capables d'établir au moins trois liaisons H avec eux-mêmes (autocomplémentaire), notamment au moins quatre liaisons H avec eux-mêmes. Parmi ces groupes, on peut en particulier citer :
- les uréïdopyrimidones;
- les uréïdopyridines, les carbamoylpyridines;
- les acylamino-s-triazines et notamment les acyl-diamino-s-triazines;
- les uréidotriazines ;
- les phtalhydrazides;
- les composés de formule :

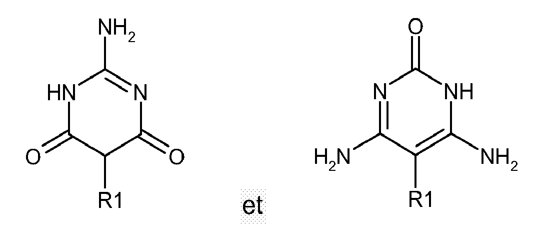

Dans lesquels les radicaux R1, R2 et R3 ont les significations données ci-dessus, en particulier les significations données en préférence.

Encore mieux, on peut citer à titre d'exemple préféré de groupes de jonction capables d'établir au moins 3 liaisons H avec eux mêmes, les groupes suivants:
- la 2-uréïdopyrimidone;
- la 6-méthyl, 2-uréïdopyrimidone;
- la diacyl de 2,6-diamino-s-triazine;
- l'uréido-s-triazine;
- les composés de formule : 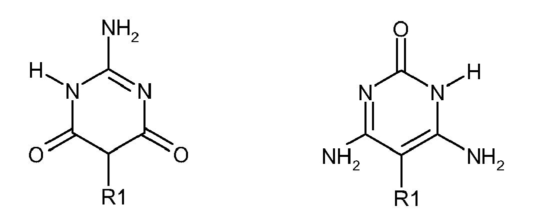
dans lesquels les radicaux R1, R2 et R3 ont les significations données ci-dessus, en particulier les significations données en préférence.

Ainsi qu'il sera indiqué plus loin, dans un mode particulier de réalisation de l'invention, l'un au moins des groupes de jonction A peut porter au moins un groupe ionisable tel que défini ci-après.
En particulier, l'un au moins des radicaux R¹, R² et/ou R³ des composés ci-dessus, peut comporter un groupe ionisable tel que défini ci-après.

Les copolymères préférés selon l'invention sont les copolymères comportant au moins deux groupes de jonction (A), de préférence au moins quatre groupes et mieux quatre ou six groupes de jonction, par chaîne de copolymères.

Dans un mode de réalisation préféré, le monomère qui comporte le ou les groupes de jonction (A) est le monomère (a) et/ou (b2), préférentiellement le monomère (a).

Ainsi qu'il a été mentionné plus haut, le copolymère selon l'invention résulte de la réaction :
- d'au moins un monomère (a) qui comporte au moins deux groupes polymérisables, identiques ou différents, choisis parmi -N=C=O et -N=C=S, ou leur forme activée ou bloquée;
- d'au moins un monomère (b1), comportant au moins deux groupes polymérisables à hydrogène labile, identiques ou différents, choisis parmi -OH, -SH, - NH₂ et -NHR, avec R représentant un groupe alkyle en C₁-C₆ ; et
- d'au moins un monomère (b2), différent du monomère (b1), comportant au moins deux groupes polymérisables à hydrogène labile, identiques ou différents, choisis parmi -OH, -SH, -NH₂ et -NHR, avec R représentant un groupe alkyle en C₁-C₆;
- l'un au moins des monomères (a), (b1) et/ou (b2) comprenant au moins un groupe de jonction (A), capable de former au moins 3 liaisons H, de préférence au moins 4 liaisons H et plus préférentiellement 4 liaisons H.

### Monomère (a)

Le monomère (a) peut donc être de formule Y=C=N-R^{a}-N=C=Y' avec Y, Y', identique ou différent, représentant O ou S; de préférence Y=Y' et encore mieux Y=Y'=O.
Lorsqu'il est sous forme activée ou bloquée, le monomère (a) peut également être de formule B-C(O)-NH-R^{a}-NH-C(O)-B' ainsi que cela est décrit dans "Comprehensive Polymer Science", vol 5 : step polymerization p421, Pergamon press (1989).
Ainsi, B et B' peuvent être, indépendamment l'un de l'autre, choisis parmi : 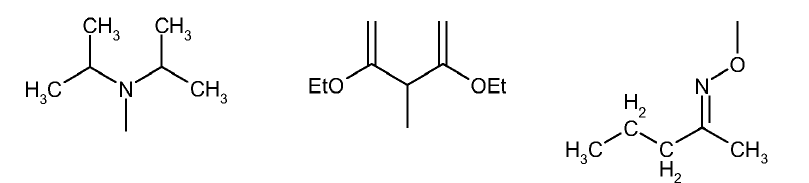 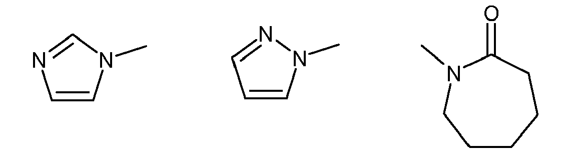 Une première famille (a1) préférée de monomères (a) est celle dans laquelle R^{a} est un radical divalent, aliphatique, saturé ou non, linéaire ou ramifié, cyclique
ou non, aromatique ou non, et qui comprend 1 à 40 atomes de carbone, comprenant éventuellement un ou plusieurs hétéroatomes choisis parmi O, S et/ou N, et/ou éventuellement substitué par un ou plusieurs atomes de fluor et/ou radicaux hydroxyle, et leurs mélanges.
Le radical R^{a} peut notamment être un groupe alkyle linéaire ou ramifié en C₁-C₃₀ ou un groupe cycloalkyle en C₄-C₁₂ ou un groupe aryle en C₄-C₁₂; éventuellement substitués par une fonction ester et/ou amide.
Il peut par exemple être de structure :
- (CH₂)_{c}-, - (CRH)_{c} et -(CRR')_{c} dans lesquels R et R', identiques ou différents, représentent un groupe alkyle linéaire ou ramifié en C₁-C₃₀ et c est un entier de 1 à 20, de préférence 1 à 12,
ou bien de structure : 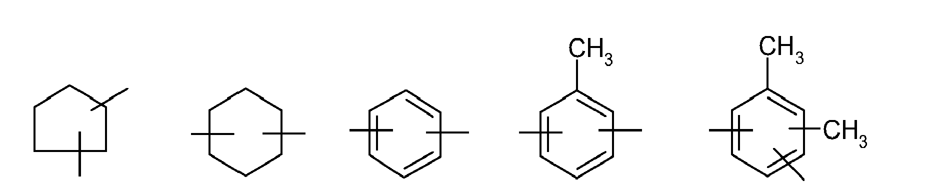 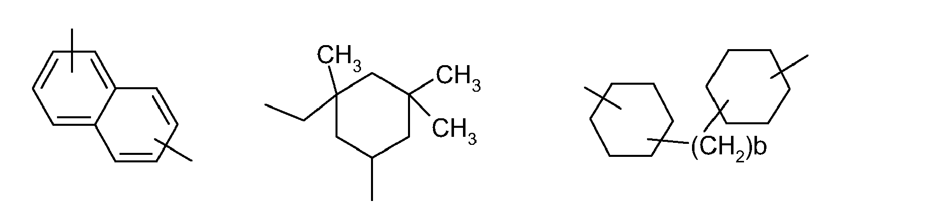 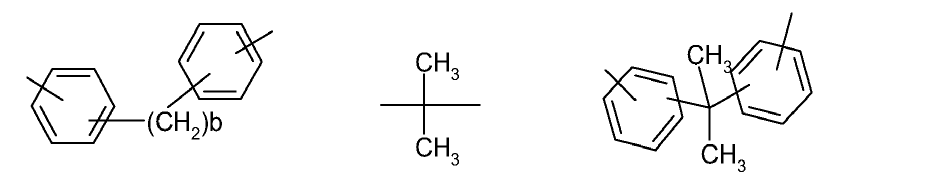 dans lesquelles b est un entier compris entre 0 et 3;
ainsi que toutes les combinaisons de ces structures.

Parmi les radicaux divalents R^{a} particulièrement préférés, on peut citer les radicaux suivants: 1,2-éthylène, 1,6-héxylène, 1,4-butylène, 1,6-(2,4,4-triméthylhexylène), 1,4-(4-méthylpentylène), 1,5-(5-méthylhexylène), 1,6-(6-méthylheptylène), 1,5-(2,2,5-triméthylhexylène), 1,7-(3,7-diméthyloctylène); 4,4'- methylenebis(cyclohexyl 1,4-cyclohexylène ; 2,4-tolylène, 2,6-tolylène; 1,5-naphtylène; 4,4'-methylenebis(phenyl); tétraméthyl xylylène; le radical divalent dérivé de l'isophorone.

Les monomères (a) particulièrement préférés peuvent être choisis parmi les composés suivants :
1,4-diisocyanatobutane;
1,6-hexamethylenediisocyanate ou 1,6-diisocyanatohexane
1,5-diisocyanato-2-méthyl-pentane
1,4-diisocyanato-4-méthyl-pentane,
1,6-diisocyanato-2,2,4-triméthylhexane,
1,6-diisocyanato-2,4,4-triméthylhexane,
1,5-diisocyanato-5-méthylhexane,
3(4)-isocyanatométhyl-1-méthylcyclohexyl-isocyanate
1,6-diisocyanato-6-méthyl-heptane,
1,5-diisocyanato-2,2,5-triméthylhexane,
1,7-diisocyanato-3,7-diméthyloctane,
1-isocyanato-1,2,2-triméthyl-3-(2-isocyanato-éthyl)-cyclopentane,
1-isocyanato-*n*-butyl-3-(4-isocyanatobut-1-yl)-cyclopentane ;
1-isocyanato-1,2-diméthyl-3-éthyl-3-isocyanatométhylcyclopentane;
1-isocyanato-1-méthyl-4-(4-isocyanatobut-2-yl)-cyclohexane,
1-isocyanato-1,4-diméthyl-4-isocyanatométhyl-cyclohexane,
1-isocyanato-1,3-diméthyl-3-isocyanatométhyl-cyclohexane,
1,3-bis(isocyanatomethyl)cyclohexane
isophoronediisocyanate;
4,4'- methylenebis(cyclohexyl isocyanate)
1,4-diphenylene diisocyanate ; tolylène 2,4-diisocyanate; tolylène 2,6-diisocyanate;
1,3-bis(isocyanatomethyl)benzène
4,4'-methylenebis(phényl isocyanate)
naphtalènediisocyanate;
tetramethyl-1,3-xylylenediisocyanate.

Ces diisocyanates peuvent bien entendu être utilisés seuls ou sous forme de mélange de deux ou plusieurs diisocyanates.

Une seconde famille (a2) préférée de monomères (a) est celle dans laquelle le radical divalent R^{a} est un radical polymérique, notamment de type homopolymère ou copolymère, comme par exemple choisi parmi :
- les copolymères éthyléniques tels que les polyoléfines comportant des motifs choisis parmi 1,2-butadiène; 1,4-butadiène; isoprène; éthylène; propylène; 1,2-butylène, 1,4-butylène; isobutylène; les copolymères (méth)acryliques, les copolymères (méth)acrylamides, les copolymères vinyliques, les copolymères allyliques et leurs mélanges.
   Ainsi conviennent à l'invention les copolymères vinyliques / (méth)acrylates, vinyliques / (méth)acrylamides, vinyliques / (méth)acrylates / méthacrylamides, oléfiniques / vinyliques et les (méth)acrylates /(méth)acrylamides;
- les polyéthers, perfluorés ou non, de type polyoxyde d'éthylène, polyoxyde de propylène, et leurs copolymères polyoxyde d'éthylène/polyoxyde de propylène, les polyoxydes de tétraméthylène et les perfluoropolyéthers ; les polythioéthers;
- les polyesters et notamment les polyesters à base d'acide adipique ou d'acide téréphtalique; la polycaprolactone; le poly(2-méthyl-1,3-propylène-adipate), le poly-(2-méthyl-1,3-propylène)-glutarate; les polyesters sulfoniques ;
- les polylactides ;
- les polyamides;
- les polyoxazolines tels que la poly(2-méthyloxazoline) ou la poly(2-éthyloxazoline) ;
- les copolymères de siloxane, tels que par exemple les polysiloxanes constitués de motifs -Si(R⁴)(R⁵)O- où (R⁴) et (R⁵), identiques ou différents, représentent H ou un radical carboné, linéaire ou ramifié, cyclique ou non, saturé ou non, ou aromatique, de préférence un alkyle en C₁-C₁₂ pouvant éventuellement comporter un ou plusieurs, de préférence 1 à 5 hétéroatomes, identiques ou différents, choisis parmi : O, N, S, P, F et Si, de préférence O, N et S, et notamment les polydiméthylsiloxanes (PDMS), et les poly(méthylphénylsiloxanes) ;
- les copolymères de ces différents types de polymères comme par exemple les copolymères polysiloxane / polyoxyde d'éthylène,
- les polyacétals ;
- les polycarbonates perfluorés ou non ;
- et leurs mélanges.

Lorsque R^{a} est un radical polymérique, il a de préférence une masse molaire moyenne en poids (Mw) comprise entre 500 et 30 000, plus particulièrement entre 700 et 25 000 et mieux de 800 à 15 000.

De préférence, R^{a} peut être choisi parmi les polymères fonctionnels de type:
- polyesters, plus particulièrement à base d'acide adipique et/ou d'acide téréphtalique; le poly(2-méthyl-1,3-propylène-adipate) et le poly-(2-méthyl-1,3-propylène)glutarate;
- polyéthers et notamment les polyoxydes de tétraméthylène,
- les copolymères de siloxane; et
- les poly(éthylène-butylène) et les polybutadiènes.

Lorsque R^{a} correspond à un mélange de différents polymères, le pourcentage desdits différents polymères peut être choisi par l'homme du métier en fonction des propriétés recherchées.

De préférence, les monomères (a) sont de type (a1), seuls ou en mélange entre eux. Toutefois, on peut également avoir un mélange de monomères (a1), seuls
ou en mélange, et de monomères (a2) seuls ou en mélange.

Lorsque le monomère (a) comprend au moins un groupe de jonction (A), il peut être de formule : 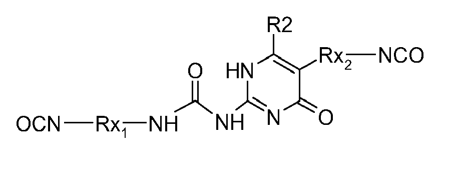 dans laquelle Rx₁, Rx₂, identiques ou différents, représentent un radical carboné divalent choisi parmi un groupe alkyle linéaire ou ramifié en C₁-C₃₀ ou un groupe cycloalkyle en C₄-C₁₂ ou un groupe aryle en C₄-C₁₂; comprenant éventuellement 1 à 8 hétéroatomes choisis parmi O, N, S, F, Si et P; éventuellement substitués par une fonction ester, amide, ou par un radical alkyle en C₁-C₁₂ ; ou un mélange de ces groupes;
R2 étant tel que défini précédemment pour le groupe de jonction (A).

De manière plus préférée, le monomère (a) peut être de formule : 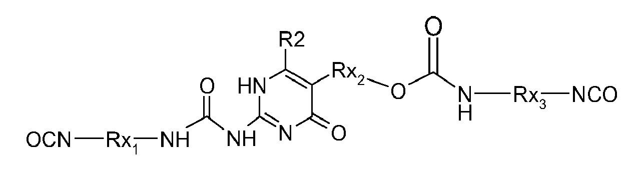 dans laquelle Rx₁, Rx₂, Rx₃ identiques ou différents, représentent un radical carboné divalent choisi parmi un groupe alkyle linéaire ou ramifié en C₁-C₃₀ ou un groupe cycloalkyle en C₄-C₁₂ ou un groupe aryle en C₄-C₁₂; ou leur mélanges,
et R2 est un radical alkyle en C1-C32.

Encore plus préférentiellement, le monomère (a) peut être de formule :

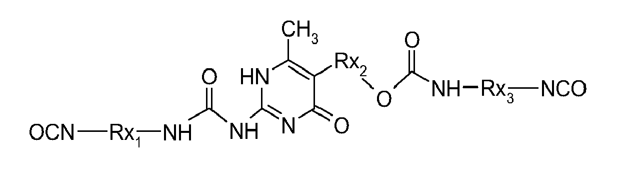

Les radicaux Rx1, Rx2 et Rx3, indépendamment l'un de l'autre peuvent être préférentiellement choisis parmi les radicaux suivants : méthylène, 1,2-éthylène, 1,6-héxylène, 1,4-butylène, 1,6-(2,4,4-triméthylhexylène), 1,4-(4-méthylpentylène), 1,5-(5-méthylhexylène); 1,6-(6-méthylheptylène); 1,5-(2,2,5-triméthylhexylène), 1,7-(3,7-diméthyloctylène); 4,4'-méthylènebiscyclohexylène; 2-méthyl-1,3-phénylène; 4-méthyl-1,3-phénylène ; 4,4'-biphénylèneméthylène, 1,2-tolylène, 1,4-tolylène, 2,4-tolylène, 2,6-tolylène; 1,5-naphtylène; 4,4'-methylenebis(phényl); tétraméthyl xylylène; le radical divalent dérivé de l'isophorone.

De façon particulièrement préférée, Rx₁, Rx₂ et Rx₃ représentent, indépendamment l'un de l'autre, -(CH₂)₂-, -(CH₂)₆-, - CH₂CH(CH₃)CH₂C(CH₃)(CH₃)CH₂CH₂-, ou un radical -isophorone- ; et mieux encore R_{X1} et Rx₃ représentent un radical -isophorone- et Rx₂ représente -(CH₂)₂-, ce qui conduit au monomère (a) suivant :

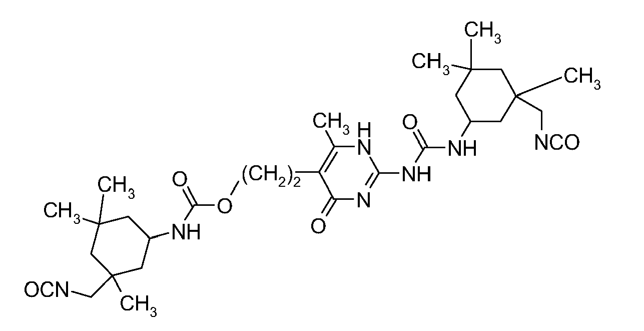

### Monomères (b) : HX-R^{b}-X'H

Le copolymère selon l'invention comprend en outre au moins deux monomères (b1) et (b2), différent l'un de l'autre, et qui comprennent tous deux au moins deux groupes polymérisables, identiques ou différents, choisis parmi OH, SH, NH₂ et NHR avec R = alkyle en C1-C6.

Le monomère (b1) peut donc être de formule HX-R^{b1}-X'H, avec X et X', identiques ou différents, choisis parmi O, S, NH ou NR, R représentant un groupe alkyle en C₁-C₆.
De préférence, dans (b1), X et/ou X' = O et préférentiellement X=X'=O.

Le radical divalent R^{b1} représente de préférence un radical divalent polymérique, notamment de type homopolymère ou copolymère, comme par exemple choisi parmi les copolymères fonctionnels dithiol, diamines, diols, aminoalcool, les fonctions alcool, amine et/ou thiol étant portées aux extrémités de la chaîne ou le long du squelette; on peut notamment citer comme radical polymérique, les radicaux de type :
- polyéthers, perfluorés ou non, de type polyoxyde d'éthylène, polyoxyde de propylène, et leurs copolymères polyoxyde d'éthylène/polyoxyde de propylène, les polyoxydes de tétraméthylène; les perfluoropolyéthers et les polythioéthers;
- polylactides;
- polyesters, notamment à base d'acide adipique ou d'acide téréphtalique; en particulier la polycaprolactone; le poly(2-méthyl-1,3-propylène-adipate), le poly-(2-méthyl-1,3-propylène)-glutarate; les polyesters sulfoniques;
- polyamides ;
- polyoxazolines tels que la poly(2-méthyloxazoline), la poly(2-éthyloxazoline);
- les copolymères de siloxane, tels que par exemple les polysiloxanes constitués de motifs - Si(R⁴)(R⁵)O - où (R⁴) et (R⁵), identiques ou différents, représentent H ou un radical carboné, linéaire ou ramifié, cyclique ou non, saturé ou non, ou aromatique, de préférence un alkyle en C₁ - C₁₂ pouvant éventuellement comporter un ou plusieurs, de préférence 1 à 5 hétéroatomes, identiques ou différents, choisis parmi : O, N, S, P, F et Si, de préférence O, N et S, et notamment les polydiméthylsiloxanes (PDMS), et les poly(méthylphénylsiloxanes) ;
- les polyacétals ;
- les copolymères éthyléniques et notamment les copolymères (méth)acryliques, les copolymères (méth)acrylamides, les copolymères vinyliques, les copolymères allyliques; ainsi conviennent à l'invention les copolymères vinyliques/(méth)acrylates, vinyliques/(méth)acrylamides, vinyliques/(méth)acrylates/méthacrylamides, oléfiniques/vinyliques et les (méth)acrylates /(méth)acrylamides;
- les polyoléfines comportant des motifs choisis parmi 1,2-butadiène; 1,4-butadiène; isoprène; éthylène; propylène; 1,2-butylène, 1,4-butylène; isobutylène;
- les polycarbonates perfluorés ou non ;
- les copolymères de ces différents types de polymères comme par exemple les copolymères polysiloxane / polyoxyde d'éthylène,
- et leurs mélanges.

Le monomère (b1) a de préférence une masse molaire moyenne en poids (Mw) comprise entre 500 et 30 000, plus particulièrement entre 700 et 25 000 et mieux de 800 à 15 000.

Lorsque R^{b1} correspond à un mélange de différents polymères, le pourcentage desdits différents polymères peut être choisi par l'homme du métier en fonction des propriétés recherchées.

De préférence, R^{b1} peut être choisi parmi les polymères fonctionnels de type:
- polyesters, plus particulièrement à base d'acide adipique et/ou d'acide téréphtalique; et notamment le poly(2-méthyl-1,3-propylène-adipate) et le poly-(2-méthyl-1,3-propylène)glutarate;
- polyéthers et notamment les polyoxydes de tétraméthylène,
- les copolymères de siloxane; et
- les poly(éthylène-butylène) et les polybutadiènes.

Le monomère (b2) peut donc être de formule HX-R^{b2}-X'H, avec X et X', identiques ou différents, choisis parmi O, S, NH ou NR, R représentant un groupe alkyle en C₁-C₆.
De préférence, dans (b2), X et/ou X' = O et préférentiellement X=X'=O.

Le radical divalent R^{b2} représente de préférence un radical carboné divalent ayant de 1 à 40 atomes de carbone, ramifié ou non, cyclique ou non, saturé ou non, aromatique ou non, comprenant éventuellement un ou plusieurs hétéroatomes choisi(s) parmi O, S, P et N, et/ou éventuellement substitué par un ou plusieurs atomes de fluor et/ou silicium.

Le ou les hétéroatomes, quand ils sont présents, peuvent être intercalés dans la chaîne dudit radical, ou bien ledit radical peut être substitué par un ou plusieurs groupes les comprenant tels que les groupes hydroxyle ou amino (NH₂, NHR' ou NR'R" avec R' et R" identiques ou différents représentant un alkyle linéaire ou ramifié en C₁-C₂₂, comprenant éventuellement 1 à 12 hétéroatomes choisis parmi O, N, S, F, Si et P, notamment méthyle ou éthyle).

Ainsi, R^{b2} peut comprendre :
- un radical alkylène ayant 1 à 40 atomes de carbone ou cycloalkylène ayant 3 à 16 atomes de carbone, éventuellement substitué, par un radical alkyle en C₁-C₁₂ comprenant éventuellement 1 à 8 hétéroatomes choisis parmi O, N, S, F, Si et P tel que méthylène, éthylène, propylène, n-butylène, isobutylène, tertiobutylène, n-hexylène, n-octylène, n-dodécylène, n-octadécylène, n-tétradécylène, n-docosanylène; éthyl-2-hexylène, cyclohexylène, cyclohexyleméthylène, isophorone;
- un radical arylène en C₁ à C₃₀ tel qu'un radical phénylène -C₆H₄-(ortho, méta
ou para) éventuellement substitué par un radical alkyle en C₁-C₁₂ comprenant éventuellement 1 à 25 hétéroatomes choisis parmi O, N, S, F, Si et P;
- un radical benzylène -C₆H₄-CH₂- éventuellement substitué par un radical alkyle en C₁-C₁₂ comprenant éventuellement 1 à 8 hétéroatomes choisis parmi O, N, S, F, Si et P;
- un radical alkylarylène ou arylalkylène en C₁ à C₃₀, de préférence en C₂ à C₁₂,
- un radical de formule : -O-CO-O-, -CO-O-, -OCO -, -O-CO-NH-, anhydride, - NH-CO-NH-, NHCO;
- un radical -Si(R⁴)(R⁵)O- où R⁴ et R⁵, identiques ou différents, représentent H ou un radical hydrocarboné, linéaire ou ramifié, cyclique ou non, saturé ou non, ou aromatique, de préférence un alkyle en C₁-C₁₂ pouvant éventuellement comporter un ou plusieurs, de préférence 1 à 5, hétéroatomes identiques ou différents choisis parmi O, N, S, P, F et Si, de préférence O, N et S ;
- un radical oxyalkylène ou aminoalkylène, notamment un radical oxyde d'alkylène de formule -(R"'O)yR^{iv} avec R"' représentant un alkyle linéaire ou ramifié en C₂-C₄, R^{iv} est l'hydrogène ou un radical alkyle, linéaire ou ramifié, en C₁à C₃₀ et y est compris entre 1 et 500 inclus, de préférence de 1 à 250 ;
- un mélange de ces radicaux.

Notamment R^{b2} peut être
- un radical alkylène ayant 1 à 40 atomes de carbone ou cycloalkylène ayant 3 à 16 atomes de carbone, éventuellement substitué, par un radical alkyle en C₁-C₁₂ comprenant éventuellement 1 à 8 hétéroatomes choisis parmi O, N, S, F, Si et P tel que méthylène, éthylène, propylène, n-butylène, n-pentylène, isobutylène, tertiobutylène, n-hexylène, n-octylène, n-dodécylène, n-octadécylène, n-tétradécylène, n-docosanylène; éthyl-2-hexylène, cyclohexylène, cyclohexyleméthylène, isophorone;
- un radical arylène en C₁ à C₃₀ tel qu'un radical phénylène -C₆H₄-(ortho, méta
ou para) éventuellement substitué par un radical alkyle en C₁-C₁₂ comprenant éventuellement 1 à 25 hétéroatomes choisis parmi O, N, S, F, Si et P;
- un radical alkylarylène ou arylalkylène en C₁ à C₃₀, de préférence en C₂ à C₁₂, et notamment un radical benzylène -C₆H₄-CH₂- éventuellement substitué par un radical alkyle en C₁-C₁₂ comprenant éventuellement 1 à 8 hétéroatomes choisis parmi O, N, S, F, Si et P;
- un radical oxyalkylène ou aminoalkylène, notamment un radical oxyde d'alkylène de formule -(R'''O)_{y}R^{iv} avec R"' représentant un alkyle linéaire ou ramifié en C₂-C₄, R^{iv} est l'hydrogène ou un radical alkyle, linéaire ou ramifié, en C₁à C₃₀ et y est compris entre 1 et 500 inclus, de préférence de 1 à 250 ;
- un mélange de ces radicaux.

De préférence, R^{b2} peut être :
- un radical alkylène ayant 1 à 12 atomes de carbone ou cycloalkylène ayant 3 à 6 atomes de carbone, éventuellement substitué, par un radical alkyle en C₁-C₁₂ tel que méthylène, éthylène, propylène, n-butylène, isobutylène, tertiobutylène, n-pentylène, n-hexylène, n-octylène, n-dodécylène, n-octadécylène, cyclohexylène, cyclohexyleméthylène, isophorone;
- un radical alkylarylène ou arylalkylène en C₁ à C₃₀, de préférence en C₂ à C₁₂, éventuellement substitué par un radical alkyle en C₁-C₁₂ tel que par exemple un radical benzylène -C₆H₄-CH₂- ou benzylène diméthylène.

Le monomère (b2) a de préférence une masse molaire moyenne en poids (Mw) comprise entre 60 et 1000, plus particulièrement entre 70 et 700 et mieux de 80 à 500.

Parmi les monomères (b2) particulièrement préférés, on peut citer :
- l'aminoéthanol, l'aminopropanol; le 4-aminobutanol; le 1-éthylaminobutan-2-ol; l'amino-2-méthyl-2-propanol; le méthyl-4-amino-4-pentan-2-ol ;
- la 1,2-éthylènediamine; la 1,2-propylènediamine; la 1,3-propylènediamine; le 1,4-diaminobutane; le 1,5-diaminopentane; le 1,6-diaminohexane; la 2,6-toluènediamine;
- le 1,4-butanediol; le 1,6-hexanediol; le 1,2-éthanediol; le 1,2-propanediol; le 1,3-propanediol;
- le néopentylglycol; le di(éthylèneglycol) de formule HO-(CH₂CH₂O)₂H; les poly(oxyde d'éthylène)dihydroxylés ; les poly(oxyde de propylène) dihydroxylés; les copolymères poly(oxyde d'éthylène /oxyde de propylène) dihydroxylés ;
- le 1,2-benzènediméthanol, le 1,4-benzènediméthanol; le 1,4-diméthylolcyclohexane ;
- le 1,2-benzènethiol;
- leurs mélanges.

Lorsque le monomère (b2) comprend au moins un groupe de jonction (A), il peut être choisi parmi les composés de formule : 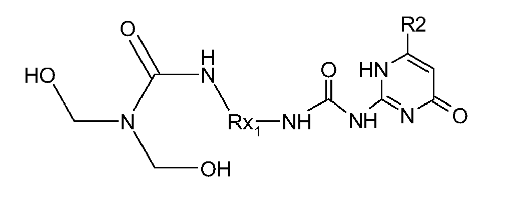 dans laquelle R2 et Rx1 sont tels que définis précédemment.

### Groupe ionisable

Dans un mode de réalisation particulier de l'invention, le copolymère peut comprendre au moins un groupe ionisable, qui peut être présent, de préférence, sur au moins une partie des monomères de type (b2) et/ou sur au moins une partie des groupes de jonction (A), préférentiellement sur au moins une partie des monomères (b2).

Par "groupe ionisable" selon l'invention, on entend tout groupe qui, soit par sa nature chimique propre, soit en fonction du milieu et/ou du pH du milieu dans lequel il se trouve, peut être sous forme ionique. Selon sa nature chimique, il peut être cationisable, anionisable ou amphotère. Ceci inclut donc les groupes ioniques tels que les groupes ammonium quaternaire tétra-N-substitués.

Les copolymères (thio)uréthane / (thio)urée selon l'invention peuvent donc comporter un ou plusieurs groupes ionisables, choisis parmi les groupes cationisables, anionisables, amphotères et leurs mélanges, favorisant leur dispersion ou solubilisation dans les milieux aqueux.

Parmi les groupes ionisables préférés, on peut citer :

-i) les groupes anionisables et leurs sels, tels que les groupes comportant une fonction acide choisie parmi :
- le radical carboxylique : -COOH,
- le radical sulfonique : -SO₃H,
- le radical : -OSO₃H
- le radical phosphonique: -(O)P(OH)₂
- le radical phosphorique : -OP(O)(OH)₂
- ou leurs formes salifiées organiques ou minérales.

La neutralisation des groupes acides peut être effectuée par une base minérale, telle que LiOH, NaOH, KOH, Ca(OH)₂, NH₄OH, Mg(OH)₂ ou Zn(OH)₂; ou par une base organique telle qu'une alkylamine primaire, secondaire ou tertiaire, notamment la triéthylamine ou la butylamine. Cette alkylamine primaire, secondaire ou tertiaire peut comporter un ou plusieurs atomes d'azote et/ou d'oxygène et peut donc comporter par exemple une ou plusieurs fonctions alcool; on peut notamment citer l'amino-2-méthyl-2-propanol, la triéthanolamine et le dimethylamino-2-propanol. On peut encore citer la lysine ou la 3-(diméthylamino)propylamine.

-ii) les groupes cationisables et leurs sels, tels que les groupes comportant une fonction choisie parmi :
-a) les radicaux amine de formule -N(R^{v})(R^{vi}) ou leurs sels organiques ou minéraux, avec R^{v} et R^{vi} représentant, indépendamment l'un de l'autre :
   (i) un atome d'hydrogène,
   (ii) un groupement alkyle linéaire, ramifié ou cyclique, saturé ou insaturé, éventuellement aromatique, comprenant de 1 à 30 atomes de carbone et pouvant comprendre 1 à 10 hétéroatomes choisis parmi O, N, S, et P, notamment un groupement méthyle, éthyle, propyle, isopropyle, n-butyle, t-butyle, isobutyle, lauryle, stéaryle;
   (iii) un groupement oxyde d'alkylène de formule -(R^{vii})ᵣR^{vii} avec R^{vii} représentant un alkyle linéaire ou ramifié en C₂-C₄, R^{viii} est l'hydrogène ou un radical alkyle, linéaire ou ramifié, en C₁ à C₃₀ et r est compris entre 1 et 250 inclus ;
   (iv) R^{v} et R^{vi} peuvent former avec l'atome d'azote un cycle saturé ou insaturé, éventuellement aromatique, comprenant au total 5, 6, 7 ou 8 atomes, et notamment 4, 5 ou 6 atomes de carbone et/ou 2 à 4 hétéroatomes choisi parmi O, S et N; ledit cycle pouvant en outre être fusionné avec un ou plusieurs autres cycles, saturés ou insaturés, éventuellement aromatiques, comprenant chacun 5, 6 ou 7 atomes, et notamment 4, 5, 6 ou 7 atomes de carbone et/ou 2 à 4 hétéroatomes choisi parmi O, S et N ;
-b) un groupement -R^{ix}-N-R^{x}- dans lequel R^{ix} et R^{x} forment avec l'atome d'azote un cycle, saturé ou insaturé, éventuellement aromatique, comprenant au total 5, 6, 7 ou 8 atomes, et notamment 4, 5 ou 6 atomes de carbone et/ou 2 à 4 hétéroatomes choisi parmi O, S et N; ledit cycle pouvant être fusionné avec un ou plusieurs autres cycles, saturés ou insaturés, éventuellement aromatiques, comprenant chacun 5, 6 ou 7 atomes, et notamment 4, 5, 6, 7 ou 8 atomes de carbone et/ou 2 à 4 hétéroatomes choisi parmi O, S et N, par exemple, le groupe ionisable peut constituer un cycle aromatique ou non comportant un groupement amine tertiaire cationisable ou peut représenter un hétérocycle aromatique ou non, contenant un azote tertiaire, cationisable.
-c) un groupement guanidino ou amidino respectivement de formule : 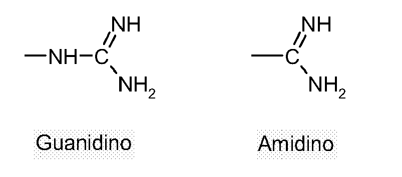
-d) les groupements ammonium quaternaire de formule : 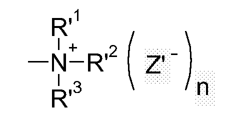 dans laquelle :
   les groupes R'¹, R'² et R'³ désignent, indépendamment l'un de l'autre, un groupe alkyle linéaire ou ramifié en C₁ à C₂₀,
   n désigne un nombre allant de 0 à 1,
   et Z'- représente un ion halogénure tel que Br ou Cl⁻ (n=1), ou encore un ion CH₃SO₃⁻ ou un ion sulfate SO₄²⁻ (n = ½) ou leurs mélanges;
- e) et leurs mélanges.

Parmi ces radicaux préférés, on peut citer les radicaux pyridinyle, indolyle, isoindolinyle, imidazolyle, imidazolinyle, piperidinyle, pyrazolyl, quinoléine, pyrazolinyl, piperazinyl, pyrrolidinyl, quinidinyl, thiazolinyl, morpholine, guanidino, amidino, et leurs combinaisons.

Les motifs amines peuvent éventuellement être neutralisés. Parmi les sels, on peut citer les sels d'acides minéraux, tels que l'acide sulfurique, l'acide chlorhydrique, l'acide phosphorique. On peut également citer les sels d'acides organiques, qui peuvent comporter un ou plusieurs groupes acide carboxylique, sulfonique, ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyle. On peut notamment citer l'acide propionique, l'acide acétique, l'acide téréphtalique, l'acide citrique, l'acide tartrique et l'acide lactique.

Il est à noter que la neutralisation des motifs acides ou amines, de même que la quaternisation, peut être totale ou partielle; elle peut notamment être comprise entre 5 et 100%, notamment entre 20 et 95%.

Plus particulièrement, ces groupes ionisables peuvent être choisis parmi:
- les groupes anionisables suivants: les groupes monovalents -COOH, - CH₂COOH, -(CH₂)₂COOH, -(CH₂)₃COOH, -(CH₂)SO₃H, -(CH₂)₂SO₃H, - (CH₂)₃SO₃H, -O(CH₂)₃SO₃H; les groupes divalents -C(COOH)(CH₃)- et -CH₂-C(COOH)(CH₃)-CH₂- ; pour lesquels les neutralisants peuvent être choisis parmi NaOH, KOH, Ca(OH)₂, NH₄OH, la triéthylamine, la butylamine, l'amino-2-méthyl-2-propanol, la triéthanolamine, la diméthylamino-2-propanol, la lysine ou la 3-(diméthylamino)propylamine;
- les groupes cationisables suivants: les groupes monovalents -(CH₂)₂-N(CH₃)₂, -N(CH₃)₂, -(CH₂)₃-N(CH₃)₂, -O-(CH₂)₃-N(CH₂CH₃)₂, -(CH₂)₂-N(CH₂CH₃)₂; les groupes divalents -(CH₂)₂-N(CH₃)-(CH₂)₂- et -(CH₂)₃-N(CH₃)-(CH₂)₃- ; pour lesquels les neutralisants peuvent être choisis parmi HCl, l'acide propionique, l'acide acétique, l'acide citrique et l'acide tartrique.

Ainsi qu'il a été mentionné plus haut, l'un au moins des groupes de jonction (A) peut porter au moins un groupe ionisable tel que défini ci-dessus.
En particulier, l'un au moins des radicaux R¹, R² et/ou R³ desdits groupes de jonction (A) peut comporter un groupe ionisable tel que défini ci-dessus.

Ledit groupe ionisable peut également être porté par le monomère (b1); dans ce cas, ledit monomère (b1) peut être choisi parmi les polyesters sulfoniques ou les copolymères éthyléniques à base d'anhydride maléique.

Dans un mode de réalisation particulier, le groupe ionisable peut être porté par tout ou partie des monomères (b2) tels que définis ci-dessus.

Parmi les monomères (b2) anionisables préférés, on peut citer les monomères comportant des fonctions carboxylique (-COOH), et/ou sulfonique (-SO₃H), notamment ceux de formule : 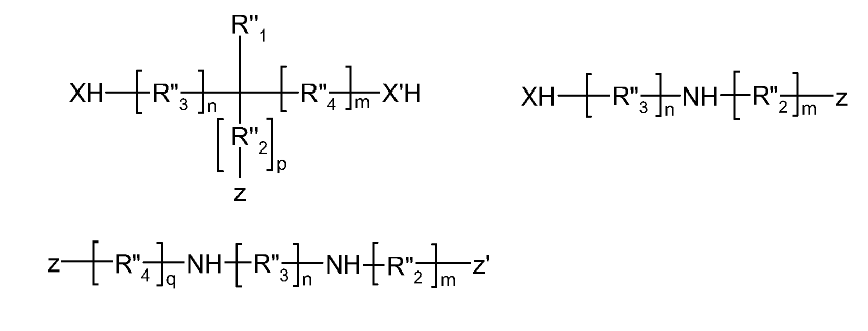 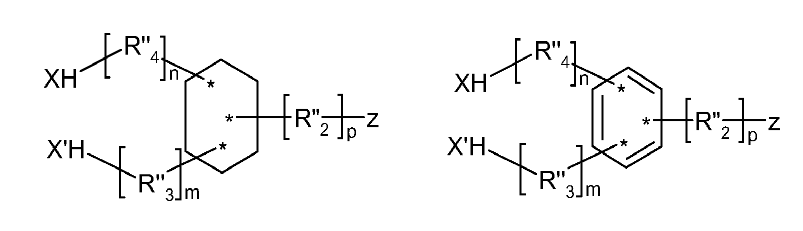 dans lesquelles :
- R"₁ représente H ou un groupe alkyle ayant de 1 à 40 atomes de carbone, ramifié ou non, cyclique ou non, saturé ou non, aromatique ou non, et/ou comprenant éventuellement un ou plusieurs hétéroatomes choisi(s) parmi O, S, P et N, et/ou éventuellement substitué par un ou plusieurs atomes de fluor ou silicium ; le ou les hétéroatomes, quand ils sont présents, peuvent être intercalés dans la chaîne dudit groupe, ou bien ledit groupe peut être substitué par un ou plusieurs groupes les comprenant tels que les groupes hydroxyle ou amino (NH₂, NHR' ou NR'R" avec R' et R" identiques ou différents représentant un alkyle linéaire ou ramifié en C₁-C₂₂, notamment méthyle ou éthyle);
- R"₂, R"₃ et R"₄, identiques ou différents, représentent un groupe alkylène (alkyle divalent) ayant de 1 à 40 atomes de carbone, ramifié ou non, cyclique ou non, saturé ou non, aromatique ou non, et/ou comprenant éventuellement un ou plusieurs hétéroatomes choisi(s) parmi O, S, P et N, et/ou éventuellement substitué par un ou plusieurs atomes de fluor ou silicium ; le ou les hétéroatomes, quand ils sont présents, peuvent être intercalés dans la chaîne dudit groupe, ou bien ledit groupe peut être substitué par un ou plusieurs groupes les comprenant tels que les groupes ester, amide, hydroxyle ou amino (NH₂, NHR' ou NR'R" avec R' et R" identiques ou différents représentant un alkyle linéaire ou ramifié en C₁-C₂₂, notamment méthyle ou éthyle).
- X et X', identiques ou différents, représentent O, S, NH, NR où R est un radical alkyle linéaire ou ramifié, en C₁-C₆; de préférence X et X' représentent O ;
- Z, Z', identiques ou différents, représentent une fonction acide carboxylique (-COOH) ou acide sulfonique (-SO₃H).
- n, p, m et q sont, indépendamment l'un de l'autre, égal à 0 ou 1.

De préférence, les monomères anionisables (b2) sont choisis parmi les composés de formule: 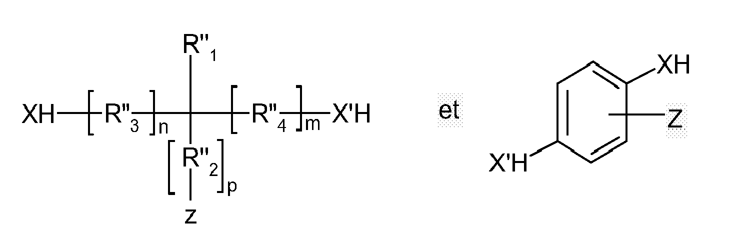 dans lesquelles X, X', R"1, R"2, R"3, R"4, m, n, p et Z sont tels que définis ci-dessus, et plus particulièrement les composés de formule : 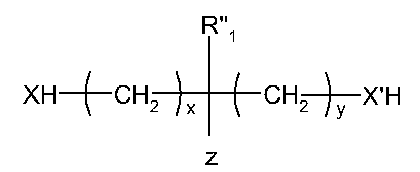 pour lesquels R"1 représente un groupe alkyle ayant de 1 à 22 atomes de carbone, de préférence CH₃, et x et y identiques ou différents, sont compris entre 1 et 5 inclus.

Parmi les monomères anionisables (b2) préférés, on peut citer l'acide diméthylolpropionique, l'acide diméthylaminopropionique, la N-éthylsulfonique-diméthanolamine, la N-éthylsulfonique-diéthanolamine, l'acide diolbenzène sulfonique.

Il est clair que ces groupes anioniques peuvent être neutralisés comme indiqués ci-dessus.

Parmi les monomères (b2) cationisables ou amphotères, on peut citer tout particulièrement les monomères porteurs de fonctions amines tertiaires de formules : 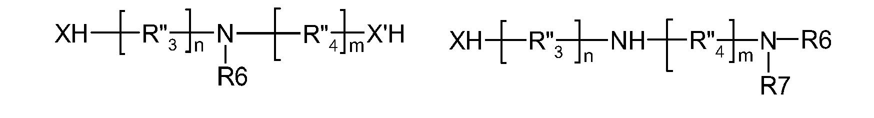 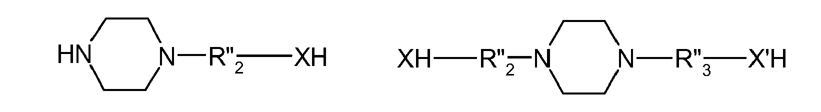 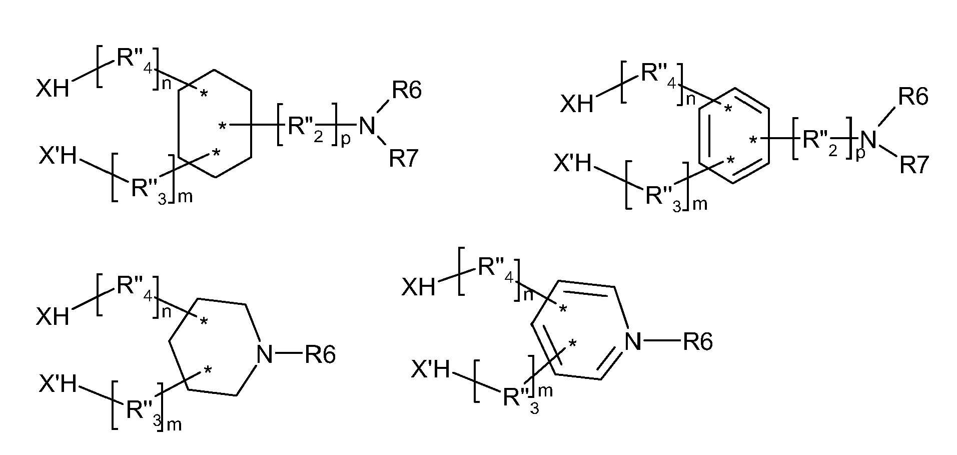 dans lesquelles :
- R"₂, R"₃ et R"₄, identiques ou différents, représentent un groupe alkylène (alkyle divalent) ayant de 1 à 40 atomes de carbone, ramifié ou non, cyclique ou non, saturé ou non, aromatique ou non, et/ou comprenant éventuellement un ou plusieurs hétéroatomes choisi(s) parmi O, S, P et N, et/ou éventuellement substitué par un ou plusieurs atomes de fluor ou silicium ; le ou les hétéroatomes, quand ils sont présents, peuvent être intercalés dans la chaîne dudit groupe, ou bien ledit groupe peut être substitué par un ou plusieurs groupes les comprenant tels que les groupes ester, amide, hydroxyle ou amino (NH₂, NHR' ou NR'R" avec R' et R" identiques ou différents représentant un alkyle linéaire ou ramifié en C₁-C₂₂, notamment méthyle ou éthyle).
- X et X', identiques ou différents, représentent O, S, NH, NR où R est un radical alkyle linéaire ou ramifié, en C₁-C₆; de préférence X et X' représentent O;
- n, p et m sont, indépendamment l'un de l'autre, égal à 0 ou 1;
- R₆ et R₇ représentent un groupe alkyle en C₁ à C₂₂, linéaire ou ramifié, de préférence méthyl, éthyl, lauryl, béhényl.

On peut notamment citer les monomères de formule : 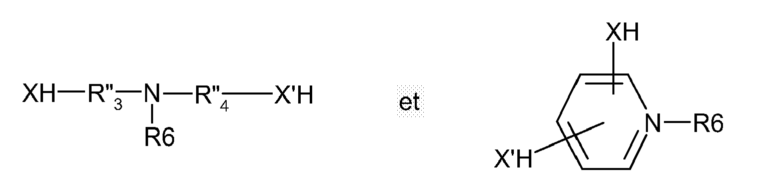 dans lesquelles X, X', R"3, R"4 et R6 sont définis tels que précédemment.

On peut notamment citer la N-méthyldiéthanolamine, la N-tert-butyldiéthanolamine, la N-éthyldiéthanolamine et la diaminopyridine.

Il est clair que ces groupes cationiques peuvent être neutralisés comme indiqués ci-dessus.

On peut encore citer comme monomères (b2) cationiques ou amphotères, ceux dans lesquels la fonction amine est sous une forme quaternaire, de formule : 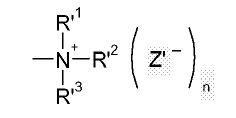 telle que définie ci-dessus.

La quaternisation des groupes amines tertiaires peut être effectuée par des composés à halogène mobile, notamment des halogénures d'alkyle tels que des chlorures ou des bromures d'alkyles en C₁-C₁₂, et par exemple le bromure de méthyle ou le chlorure d'éthyle.
Ces groupes peuvent encore être quaternisés par des composés à halogène mobile comportant des fonctions acides carboxyliques ou sulfoniques, notamment le chloroacétate de sodium; ou par des sulfones cycliques, par exemple la propanesulfone. On obtient ainsi des monomères amphotères (ou bétaïnes, ayant au moins une charge (+) et au moins une charge (-) portées par le même monomère).
La quaternisation peut être effectuée sur le polymère déjà synthétisé ou sur les monomères de départ, avant polymérisation.

Le groupe ionisable, quand il est présent, peut représenter 0,1 à 50% en poids du poids total du copolymère selon l'invention, de préférence 0,5 à 35% en poids, et notamment 1 à 15% en poids, par rapport au poids total du polymère.
Ainsi, le ou les monomères portant le/les groupes ionisables peuvent représenter 3 à 20% en poids, de préférence 6 à 17% en poids, voire 8 à 15% en poids, du poids total du copolymère final.

Le copolymère selon l'invention peut également comprendre, à titre optionnel, des monomères de structure YCN-R₁₀ avec Y représentant O ou S; de préférence O; et R₁₀ représentant un radical carboné, notamment alkyle, ayant de 1 à 40 atomes de carbone, linéaire ou ramifié, cyclique ou non, saturé ou insaturé, aromatique ou non, comprenant éventuellement un ou plusieurs hétéroatomes choisi(s) parmi O, S, P et N, et/ou éventuellement substitué par un ou plusieurs atomes de fluor ou silicium; le ou les hétéroatomes, quand ils sont présents, peuvent être intercalés dans la chaîne dudit radical, ou bien ledit radical pouvant être substitué par un ou plusieurs groupes les comprenant tels que les groupes esters et/ou amides.

Dans un mode de réalisation particulier, R₁₀ peut également porter au moins un groupe de jonction (A).
Notamment, YCN-R₁₀ peut répondre à la formule : 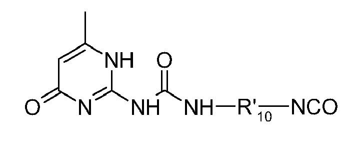 dans laquelle R'10 peut représenter un radical carboné divalent choisi parmi un groupe alkyle linéaire ou ramifié en C₁-C₃₀, un groupe cycloalkyle en C₄-C₁₂ ou un groupe aryle en C₄-C₁₂; comprenant éventuellement mais non préférentiellement, 1 à 8 hétéroatomes choisis parmi O, N, S, F, Si et P; ainsi que les mélanges de ces significations.

Le copolymère selon l'invention peut également comprendre, à titre optionnel, des monomères de structure HX-R₁₁ avec :
- X représentant O, S, NH ou NR, R représentant un groupe alkyle en C₁-C₆; de préférence, X = O; et
- R₁₁ représentant un radical carboné, notamment alkyle, ayant de 1 à 40 atomes de carbone, linéaire ou ramifié, cyclique ou non, saturé ou insaturé, aromatique ou non, comprenant éventuellement un ou plusieurs hétéroatomes choisi(s) parmi O, S, P et N, et/ou éventuellement substitué par un ou plusieurs atomes de fluor ou silicium; le ou les hétéroatomes, quand ils sont présents, peuvent être intercalés dans la chaîne dudit radical, ou bien ledit radical pouvant être substitué par un ou plusieurs groupes les comprenant tels que les groupes esters et/ou amides.

### Structure des copolymères selon l'invention

De manière avantageuse, les copolymères de l'invention ont un squelette polymérique ayant un degré de polymérisation allant de 3 à 20000, et de préférence de 5 à 10000 et mieux de 10 à 5000.

Les copolymères selon l'invention se caractérisent par la présence de séquences polyuréthanes et/ou polyurées et/ou polythiouréthanes et/ou polythiourées répondant de préférence à la formule générale suivante :

-[X-R^{b}-X'-C(Y)NH-R^{a}-NHC(Y')-]ₓ-

dans laquelle :
X et X' proviennent des monomères (b), à savoir des monomères (b1) et(b2), et sont tels que définis ci-dessus,
Y et Y' proviennent des monomères (a), qu'ils soient de type (a1) et/ou (a2), et sont tels que définis ci-dessus,
les radicaux R^{b} proviennent des monomères (b), à savoir (b1) et (b2), c'est-à-dire qu'ils représentent, statistiquement, Rb1 et Rb2 tels que définis ci-dessus;
les radicaux R^{a} proviennent des monomères (a), et sont tels que définis ci-dessus; notamment ils peuvent représenter, statistiquement, les monomères R^{a} de type radical divalent aliphatique (a1) et les monomères R^{a} de type polymérique (a2);
étant donné qu'au moins un des radicaux Ra et/ou Rb, comprend au moins un groupe de jonction A capable de former au moins 3 liaisons H;
x est un nombre entier ≥ 2.

La masse moléculaire moyenne en nombre Mn du copolymère selon l'invention est de préférence comprise entre 1 000 et 3 000 000, de préférence entre 5000 et 1 000 000, mieux entre 8 000 et 500 000.

De préférence, le rapport molaire entre la totalité des monomères de formule YCN-Ra-NCY' et la totalité des monomères de formule HX-Rb-X'H (à savoir les monomères (b1) et (b2)) peut être de 0,5 à 2, de préférence de 0,6 à 1,5 et mieux de 0,7 à 1,3; préférentiellement, ce rapport est de 1.

De préférence, les monomères de type HX-R^{b1}-X'H(polymère) représentent 10 à 95% en poids, notamment 12 à 85% en poids, préférentiellement 15 à 80% en poids, du poids total du polymère final.

De préférence, les monomères de type HX-R^{b2}-X'H (petit diol) représentent 1 à 30% en poids, notamment 2 à 25% en poids, préférentiellement 3 à 20% en poids, du poids total du polymère final.

De préférence, le copolymère selon l'invention comporte 1 à 12 groupes de jonction (A), notamment 2 à 10, en particulier 3 à 8, voire 4 à 6, groupes de jonction (A), par chaîne polymérique.

Les copolymères selon l'invention peuvent présenter deux températures de transition vitreuse, de préférence une première température de transition vitreuse, Tg1, inférieure ou égale à 0°C, et une deuxième température de transition vitreuse, Tg2, supérieure ou égale à 20°C.

De préférence encore, les copolymères de l'invention comportent des motifs provenant de monomères polymériques (b1) portant à leurs extrémités des fonctions réactives à hydrogène labile (XH) et qui ont une température de transition vitreuse (Tg) inférieure à 20°C, de préférence inférieure à 10°C, mieux inférieure à 0°C.

### Synthèse des copolymères selon l'invention :

Les copolymères selon l'invention, notamment les copolymères (thio)uréthane/(thio)urée peuvent être préparés selon des méthodes de polycondensation usuelles et connues de l'homme du métier. Ces méthodes sont notamment décrites dans les ouvrages suivants :
- 60 Years of PUR - J.E. Kresta, E.W. Eldred Ed. Technomic Publishing, 1998,
- Waterborne and Solvent Based Surface Coating Resins and Their Application, Série Surface Coating Technology, Vol. 3, Polyurethanes, Paul Thomas, John Wiley and Sons, 1998.

A titre d'illustration, on donne ci-après un schéma général de synthèse au quel l'homme du métier pourra se référer :
- on fait réagir, dans un solvant organique, le ou les monomères à hydrogène labile (b1) et (b2) avec le ou les monomères (a), en présence ou non d'un catalyseur;
- le solvant organique utilisé est de préférence choisi pour être inerte vis-à-vis des groupes isocyanates. Il est de préférence choisi dans le groupe constitué par l'acétone, la méthyléthylcétone, l'acétate d'éthyle ou l'acétate de butyle, le tétrahydrofurane, le 1,2-dichloroéthane et leurs mélanges ;
- le catalyseur éventuel peut être généralement choisi parmi les dérivés d'étain
ou de titane, et de préférence choisi parmi le 2-éthyl-hexanoate d'étain et le dibutyl dilaurate d'étain;
- la polymérisation s'effectue à une température généralement comprise entre 20°C et 120°C et de préférence entre 50 et 100 °C;
- le copolymère de l'invention ainsi obtenu peut être ensuite éventuellement purifié, par exemple par précipitation dans un non-solvant.

Lorsque le copolymère est destiné à être utilisé dans les milieux hydrophiles et en particulier aqueux, une étape de mise en solution aqueuse peut être nécessaire; dans ce cas le polymère comporte de préférence des groupes ionisables au sens de la présente invention.

Il est possible de préparer une solution ou une dispersion aqueuse du copolymère selon l'invention de la manière suivante :
- le copolymère peut être solubilisé par simple addition d'eau, notamment s'il comporte des groupes ionisables en quantité suffisante c'est-à-dire par exemple au moins 3 à 20% en poids de monomères portant de tels groupes, de préférence 6 à 17% en poids, voire 8 à 15% en poids, par rapport au poids total du copolymère.
- le copolymère, notamment lorsqu'il comporte des groupes ionisables, peut être véhiculé dans l'eau par simple addition de neutralisant en solution aqueuse. Dans ce cas, le polymère peut être obtenu sous forme soluble ou dispersible dans l'eau.
- le copolymère, notamment lorsqu'il comporte des groupes ionisables, peut également être véhiculé dans l'eau par une méthode de solubilisation dans un solvant organique volatil, puis addition d'eau et de neutralisant, et enfin évaporation du solvant organique. Dans ce cas, le polymère est généralement obtenu sous forme de dispersion aqueuse.
   On peut, selon une variante de procédé, opérer la neutralisation des fonctions ionisables du copolymère lors de la formation même du polymère .
   Après l'obtention du copolymère dans le solvant organique, on procède alors à la préparation d'une émulsion aqueuse en versant, sous agitation, à la solution organique obtenue, une quantité appropriée d'eau contenant un neutralisant et éventuellement un agent anti-mousse dont le rôle sera de faciliter l'évaporation ultérieure de la phase organique.
   Lors de la formation de l'émulsion, l'agitation est de préférence réalisée à l'aide d'un disperseur cisaillant du type Moritz ou Ultraturax ou Raineri équipé de pâles défloculantes.
   L'émulsion ainsi obtenue est particulièrement stable sans qu'il soit nécessaire d'employer un agent tensioactif dans la mesure où les groupes ioniques du copolymère se placent à l'interface avec l'eau et protègent les gouttelettes de la coalescence par répulsion électrostatique.
   Après formation de l'émulsion à une température comprise entre la température ambiante (20-25°C) et 70°C environ, on procède alors à l'évaporation sous pression réduite du solvant organique jusqu'à son élimination totale, ladite évaporation étant de préférence réalisée sous léger chauffage.
   On obtient ainsi finalement une dispersion aqueuse de particules du copolymère, par exemple du polyuréthane, qui est exempte de tout tensioactif ou de tout autre stabilisant hydrophile, tout en étant très stable.

La taille moyenne des particules ainsi que leur polydispersité peuvent être réglées en faisant varier, lors de la préparation de la dite dispersion, les proportions respectives entre le copolymère, le solvant organique et l'eau, ou en faisant varier le taux de neutralisation ou la nature de l'agent neutralisant.
Selon un mode particulier de réalisation de la dispersion, la taille moyenne des particules est comprise entre 5 et 400 nanomètres, de préférence entre 10 et 250 nanomètres, mesurée par diffusion quasi-élastique de lumière.
La polydispersité en taille desdites particules, mesurée par diffusion quasi-élastique de la lumière, est, quant à elle, généralement inférieure à 0,5, et de préférence inférieure à 0,3.

Il est entendu que la formation de solution ou de dispersion dépend de la nature du copolymère et /ou du neutralisant utilisé.

Les copolymères, par exemple les polyuréthanes, selon l'invention peuvent être plastifiés pour améliorer la formation des films à température ambiante. La plastification peut être réalisée à l'aide de plastifiants classiques non polymériques. Il est alors préférable que le plastifiant soit un bon solvant du copolymère, par exemple du polyuréthane, selon l'invention et soit de préférence insoluble dans l'eau. Parmi les plastifiants hydrophobes, on peut citer notamment :
- les phtalates et adipates de diéthyle, de dibutyle et de 2-diéthylhexyle,
- les tartrates de diéthyle et de dibutyle,
- les phosphates de diéthyle, de dibutyle et de 2-diéthylhexyle,
- les dérivés de propylèneglycol choisis parmi le propylèneglycol phényléther, le propylèneglycol diacétate, le dipropylèneglycol butyléther, le tripropylèneglycol butyléther,
- les esters de glycérol tels que le triacétate de glycérol (triacétine),
- le monophényléther de propylèneglycol vendu sous la dénomination de "Dowanol PPH" par Dow Chemical et le dipropylèneglycol *n*-butyléther vendu sous la dénomination de "Dowanol DPnB" par Dow Chemical.
   Le plastifiant peut être incorporé soit après la réalisation de la dispersion, soit pendant la réalisation de la dite dispersion : le plastifiant étant alors incorporé dans la phase organique.

Les copolymères, notamment les polyuréthanes, selon l'invention permettent donc la préparation de dispersions ou de solutions aqueuses très fluides et de plus, particulièrement stables sans utilisation de tensioactifs additionnels, dans la mesure où ils comportent en outre des fonctions ioniques.

Les copolymères de la présente invention peuvent également être véhiculés dans les esters, les alcools, les huiles carbonées et/ou siliconées et leur mélange.

L'invention a également pour objet l'utilisation d'au moins un copolymère selon l'invention dans des compositions cosmétiques en vue d'améliorer les propriétés visco-élastiques des dépôts et films cosmétiques obtenus à partir de ces compositions.

Les compositions cosmétiques selon l'invention comprennent de préférence les copolymères selon l'invention, seuls ou en mélange, en une quantité de 0,01% à 90% en poids, de préférence de 0,1 à 85% en poids, et mieux de 0,5 à 80% en poids, par rapport au poids total de la composition.

Les compositions selon l'invention peuvent se présenter sous toutes les formes galéniques classiquement utilisées pour une application topique et notamment sous forme d'une solution ou suspension aqueuse, alcoolique ou hydroalcoolique, ou d'une solution ou suspension huileuse, ou d'une solution ou d'une dispersion du type lotion ou sérum, d'une émulsion de consistance liquide ou semi-liquide du type lait, obtenues par dispersion d'une phase grasse dans une phase aqueuse (H/E) ou inversement (E/H), ou d'une suspension ou émulsion de consistance molle de type crème (H/E) ou (E/H), ou d'un gel aqueux ou anhydre, d'un onguent, d'une poudre libre ou compactée à utiliser telle quelle ou à incorporer dans un excipient, ou de toute autre forme cosmétique.

Les compositions cosmétiques ou pharmaceutiques selon l'invention comprennent, outre lesdits copolymères, un milieu physiologiquement acceptable, notamment cosmétiquement ou pharmaceutiquement, notamment dermatologiquement, acceptable, c'est-à-dire un milieu compatible avec les tissus cutanés comme la peau du visage ou du corps, et les matières kératiniques telles que les cheveux, les cils, les sourcils et les ongles.
Le milieu physiologiquement acceptable est avantageusement un milieu ne nuisant pas aux propriétés de persistance accrue d'au moins un effet cosmétique et/ou de soin, d'adhésion sur les matières kératiniques et de facilité de démaquillage apportées par la composition après application.

De préférence, le milieu physiologiquement acceptable comprend un milieu solvant des copolymères selon l'invention, qui peut comprendre au moins un composé choisi parmi l'eau, les alcools, les polyols, les esters, les huiles carbonées, les huiles de silicone, les huiles de silicone fluorées, et leurs mélanges. Les huiles peuvent être polaires ou apolaires.

Ainsi, le milieu solvant des compositions selon l'invention peut comprendre de l'eau ou un mélange d'eau et de solvant(s) organique(s) hydrophile(s) comme les alcools et notamment les monoalcools, linéaires ou ramifiés en C₁-C₆, comme l'éthanol, le tert-butanol, le n-butanol, l'isopropanol ou le n-propanol, ou le 2-butoxy-éthanol; et les polyols comme la glycérine, la diglycérine, l'éthylène glycol, le propylène glycol, le sorbitol, le pentylène glycol, et les polyéthylène glycols, ou bien encore les éthers de glycols notamment en C₂ tel que le monoéthyléther et le monométhyléther de diéthylèneglycol, et les aldéhydes en C₂-C₄ hydrophiles.

Le milieu solvant peut comprendre des alcools incorporant un groupe aromatique comme l'alcool benzylique ou le phénoxyéthanol (alcool aromatique), leurs dérivés; des esters carboxyliques, et leurs mélanges.
Parmi les esters carboxyliques, on peut citer ceux comprenant 2 à 8 atomes de carbone, notamment l'acétate d'éthyle et l'acétate de butyle, l'acétate de méthyle, l'acétate de propyle, l'acétate de n-butyle, l'acétate d'isopentyle.
Le milieu solvant peut également comprendre des solvants organiques, physiologiquement acceptables, choisis parmi les cétones liquides à température ambiante tels que la méthyléthylcétone, la méthylisobutylcétone, la diisobutylcétone, l'isophorone, la cyclohexanone, l'acétone; les éthers de propylène glycol liquides à température ambiante tels que le monométhyléther de propylène glycol, l'acétate de monométhyléther de propylène glycol, le mono n-butyl éther de dipropylène glycol; les éthers liquides à 25°C tels que le diéthyléther, le diméthyléther ou le dichlorodiéthyléther; les alcanes liquides à 25°C tels que le décane, l'heptane, le dodécane, l'isododécane, le cyclohexane; les composés cycliques aromatiques liquides à 25°C tels que le toluène et le xylène ; les aldéhydes liquides à 25°C tels que le benzaldéhyde, l'acétaldéhyde et leurs mélanges.

Le milieu solvant peut également comprendre des huiles cosmétiques, polaires ou apolaires, carbonées ou siliconées, d'origine animale, végétale, minérale ou synthétique.
Parmi les huiles polaires, on peut citer les huiles carbonées pouvant comporter des fonctions ester, éther, acide et/ou alcool, telles que par exemple :
- les huiles végétales carbonées à forte teneur en triglycérides constitués d'esters d'acides gras et de glycérol dont les acides gras peuvent avoir des longueurs de chaîne variées, ces dernières pouvant être linéaires ou ramifiées, saturées ou insaturées; ces huiles sont notamment les huiles de germe de blé, de maïs, de tournesol, de karité, de ricin, d'amandes douces, de macadamia, d'abricot, de soja, de colza, de coton, de luzerne, de pavot, de potimarron, de sésame, de courge, d'avocat, de noisette, de pépins de raisin ou de cassis, d'onagre, de millet, d'orge, de quinoa, d'olive, de seigle, de carthame, de bancoulier, de passiflore, de rosier muscat; ou encore les triglycérides des acides caprylique/caprique comme ceux vendus par la société Stearineries Dubois ou ceux vendus sous les dénominations Miglyol 810, 812 et 818 par la société Dynamit Nobel;
- les huiles de synthèse de formule R¹¹COOR¹² dans laquelle R¹¹ représente le reste d'un acide gras, linéaire ou ramifié, comportant de 7 à 19 atomes de carbone, et R¹² représente une chaîne hydrocarbonée ramifiée contenant de 3 à 20 atomes de carbone, comme par exemple l'huile de Purcellin (octanoate de cétostéaryle), l'isononanoate d'isononyle, les benzoates d'alkyle en C₁₂-C₁₅.
- les esters et les éthers de synthèse comme le myristate d'isopropyle, le palmitate d'éthyl-2-hexyle, les octanoates, les décanoates ou les ricinoléates d'alcools ou de polyalcools,
- les esters hydroxylés comme le lactate d'isostéaryle, le malate de diisostéaryle, et les esters du pentaérythritol;
- les alcools gras en C₈ à C₂₆ comme l'alcool oléique; et
- leurs mélanges.
   Parmi les huiles apolaires, on peut citer :

- les huiles de silicone volatiles ou non, linéaires ou cycliques, liquides à température ambiante, telles que les polydiméthylsiloxanes (PDMS) comportant des groupements alkyle, alcoxy ou phényle, pendants et/ou en bout de chaîne siliconée et ayant de 2 à 24 atomes de carbone; les silicones phénylées, comme les phényl triméthicones, les phényl diméthicones, les phényl triméthylsiloxydiphénylsiloxanes, les diphényl diméthicones, les diphényl méthyltrisiloxanes, les 2-phényléthyl triméthylsiloxysilicates,
- les hydrocarbures et les hydrocarbures fluorés ou fluorocarbures, linéaires ou ramifiés d'origine synthétique ou minérale, comme les huiles volatiles, telles que les huiles de paraffine (par exemple les isoparaffines et notamment l'isododécane), ou non volatiles et leurs dérivés, tels que la vaseline, les polydécènes, le polyisobutène hydrogéné tel que le parléam, le squalane, et leurs mélanges.
   Le milieu solvant peut être présent de préférence en une quantité de 1 à 90% en poids, en particulier de 5 à 70% en poids par rapport au poids total de la composition.

La solubilité des copolymères selon l'invention peut être contrôlée par le choix des squelettes polymériques -POL- et/ou des groupes de jonction (A).

Les copolymères de l'invention peuvent dans certains cas interagir entre eux physiquement (en établissant un réseau d'interactions H) dans certains solvants ou mélanges de solvants. Cela dépend notamment de la nature et des proportions de solvants ou mélanges de solvants utilisés. Ceci peut provoquer une augmentation indésirable de la viscosité de la composition et éventuellement gêner son application (par exemple lotion, aérosol, etc....).
Pour s'affranchir de ce problème de viscosité, il est possible:
- de solubiliser le copolymère selon l'invention dans un solvant volatil pouvant établir des interactions H avec des groupes de jonction (A), par exemple en utilisant des alcools courts en C₁-C₄, des polyols volatils, de l'eau et/ou un mélange de ces solvants, ou
- d'utiliser un milieu solvant biphasique, comme par exemple une émulsion eau-dans-huile (E/H) ou huile-dans-eau (H/E) et un couple de copolymères selon l'invention sélectifs dont les groupes de jonction et les squelettes polymériques sont de nature chimique différentes, chaque polymère étant dissous dans une phase différente de l'autre (l'un dans l'eau, l'autre dans l'huile).
   Dans ce dernier cas, le couple de copolymères A₁-POL₁-A₁ et A₂-POL₂-A₂ peut être avantageusement choisi de telle manière que :

- chacun des groupes de jonction (A₁) n'établit pas d'interaction H avec lui-même mais seulement avec (A₂),
- chacun des groupes de jonction (A₂) n'établit pas d'interaction H avec lui-même mais seulement avec (A₁),
- les groupes de jonction (A₁) et (A₂) n'établissent des interactions H que lorsqu'ils sont mis en contact, et
- les squelettes polymériques -POL₁- et -POL₂- sont choisis de manière telle que les copolymères A₁-POL₁- A₁et A₂-POL₂- A₂ peuvent être chacun véhiculés dans une phase distincte de l'émulsion, de sorte qu'ils ne puissent pas réagir dans l'émulsion.
   L'interaction entre les deux copolymères ne se fera qu'à l'application, à condition toutefois que les milieux solvants soient des solvants volatils ou puissent pénétrer dans le support kératinique.

La composition de l'invention peut en outre comprendre les adjuvants habituellement employés dans les domaines cosmétique ou pharmaceutique, dans la mesure où l'adjuvant n'altère pas les propriétés recherchées pour la composition de l'invention, tels que les cires, les gommes, les tensioactifs, les épaississants, les gélifiants hydrophiles ou lipophiles, les actifs cosmétiques hydrophiles ou lipophiles, les conservateurs, les antioxydants, les parfums, les agents nacrants, les charges, les neutralisants, les copolymères autres que ceux définis précédemment, les émulsionnants et les co-émulsionnants, les pigments, les matières colorantes.
Bien entendu l'homme du métier veillera à choisir ce ou ces éventuels adjuvants et/ou leur quantité, de manière telle que les propriétés avantageuses de la composition selon l'invention ne soient pas, ou substantiellement pas, altérées par l'adjonction envisagée.
Les quantités de ces différents adjuvants sont celles classiquement utilisées dans les domaines considérés, par exemple de 0,001 à 30% du poids total de la composition. Ces adjuvants, selon leur nature, peuvent être introduits dans la phase grasse, dans la phase aqueuse, dans les vésicules lipidiques et/ou dans les nanoparticules.

Par "cire" au sens de la présente invention, on entend un composé lipophile, solide à température ambiante (25°C), à changement d'état solide/liquide réversible, ayant un point de fusion supérieur ou égal à 25°C pouvant aller jusqu'à 120°C. En portant la cire à l'état liquide (fusion), il est possible de la rendre miscible aux huiles éventuellement présentes et de former un mélange homogène microscopiquement, mais en ramenant la température du mélange à la température ambiante, on obtient une recristallisation de la cire dans les huiles du mélange. Le point de fusion de la cire peut être mesuré à l'aide d'un calorimètre à balayage différentiel (D.S.C.), par exemple le calorimètre vendu sous la dénomination DSC 30 par la société METTLER.
Les cires peuvent être hydrocarbonées, fluorées et/ou siliconées et être d'origine végétale, minérale, animale et/ou synthétique. En particulier, les cires présentent un point de fusion supérieur à 30 °C et mieux supérieur à 45°C. Comme cire utilisable dans la composition de l'invention, on peut citer la cire d'abeilles, la cire de Carnauba ou de Candellila, la paraffine, les cires microcristallines, la cérésine ou l'ozokérite; les cires synthétiques comme les cires de polyéthylène ou de Fischer Tropsch, les cires de silicones comme les alkyl ou alkoxy-diméthicone ayant de 16 à 45 atomes de carbone.
Les gommes sont généralement des polydiméthylsiloxanes (PDMS) à haut poids moléculaire ou des gommes de cellulose ou des polysaccharides et les corps pâteux sont généralement des composés hydrocarbonés comme les lanolines et leurs dérivés ou encore des PDMS.
La nature et la quantité des cires et/ou gommes sont fonctions des propriétés mécaniques et des textures recherchées. A titre indicatif, la composition peut comprendre 0,1 à 50% en poids de cires, par rapport au poids total de la composition et mieux de 1 à 30% en poids.

La composition selon l'invention peut en outre comprendre, dans une phase particulaire, des pigments et/ou des nacres et/ou des charges habituellement utilisés dans les compositions cosmétiques.
La composition peut également comprendre d'autres matières colorantes que les pigments et nacres, choisies parmi les colorants hydrosolubles ou les colorants liposolubles bien connus de l'homme du métier. Toutes ces matières colorantes peuvent être employées en mélanges.
Par pigments, il faut comprendre des particules de toute forme, blanches ou colorées, minérales ou organiques, insolubles dans le milieu physiologique, destinées à colorer la composition.
Par charges, il faut comprendre des particules incolores ou blanches, minérales ou de synthèse, lamellaires ou non lamellaires, destinées à donner du corps ou de la rigidité à la composition, et/ou de la douceur, de la matité et de l'uniformité au maquillage.
Par nacres, il faut comprendre des particules de toute forme irisées, notamment produites par certains mollusques dans leur coquille ou bien synthétisées.
Les pigments peuvent être présents dans la composition à raison de 0,01 à 25% en poids de la composition finale, et de préférence à raison de 3 à 10% en poids. Ils peuvent être blancs ou colorés, minéraux ou organiques. On peut citer les oxydes de titane, de zirconium ou de cérium, ainsi que les oxydes de zinc, de fer ou de chrome, le bleu ferrique, l'hydrate de chrome, le noir de carbone, les outremers (polysulfures d'aluminosilicates), le pyrophosphate de manganèse et certaines poudres métalliques telles que celles d'argent ou d'aluminium. On peut encore citer les pigments D&C et les laques couramment employées pour conférer aux lèvres et à la peau un effet de maquillage, qui sont des sels de calcium, de baryum, d'aluminium, de strontium ou de zirconium.
Les nacres peuvent être présentes dans la composition à raison de 0,01 à 20% en poids, de préférence à un taux de l'ordre de 3 à 10% en poids. Parmi les nacres envisageables, on peut citer la nacre naturelle, le mica recouvert d'oxyde de titane, d'oxyde de fer, de pigment naturel ou d'oxychlorure de bismuth ainsi que le mica titane coloré.
Parmi les colorants, liposolubles ou hydrosolubles, qui peuvent être présents dans la composition, seul ou en mélange, à raison de 0,001 à 15% en poids, de préférence 0,01 à 5% en poids et notamment de 0,1 à 2% en poids, par rapport au poids total de la composition, on peut citer le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle, le bleu de méthylène, le carmin de cochenille, les colorants halogéno-acides, azoïques, anthraquinoniques, le sulfate de cuivre ou de fer, le brun Soudan, le rouge Soudan et le rocou, ainsi que le jus de betterave et le carotène.
La composition selon l'invention peut comprendre en outre en outre une ou plusieurs charges, notamment en une teneur allant de 0,01 % à 50% en poids, par rapport au poids total de la composition, de préférence allant de 0,02 % à 30 % en poids. Les charges peuvent être minérales ou organiques de toute forme, plaquettaires, sphériques ou oblongues. On peut citer le talc, le mica, la silice, le kaolin, les poudres de polyamide (Nylon^{®}), de poly-β-alanine et de polyéthylène, les poudres de polymères de tétrafluoroéthylène (Téflon^{®}), la lauroyllysine, l'amidon, le nitrure de bore, les microsphères creuses polymériques telles que celles de chlorure de polyvinylidène/acrylonitrile comme l'Expancel^{®} (Nobel Industrie), de copolymères d'acide acrylique (Polytrap^{®} de la société Dow Corning) et les microbilles de résine de silicone (Tospearls^{®} de Toshiba, par exemple), les particules de polyorganosiloxanes élastomères, le carbonate de calcium précipité, le carbonate et l'hydrocarbonate de magnésium, l'hydroxyapatite, les microsphères de silice creuses (Silica Beads^{®} de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

La composition peut comprendre en outre un polymère additionnel tel qu'un polymère filmogène. Selon la présente invention, on entend par "polymère filmogène", un polymère apte à former à lui seul ou en présence d'un agent auxiliaire de filmification, un film continu et adhérent sur un support, notamment sur les matières kératiniques. Parmi les polymères filmogènes susceptibles d'être utilisés dans la composition de la présente invention, on peut citer les polymères synthétiques, de type radicalaire ou de type polycondensat, les polymères d'origine naturelle et leurs mélanges, en particulier les polymères acryliques, les polyuréthanes, les polyesters, les polyamides, les polyurées, les polymères cellulosiques comme la nitrocellulose.
En particulier, les (co)polyméres de l'invention peuvent être utilisés en mélange avec un ou plus des copolymères décrits dans la demande WO 02/098377.

Bien que de préférence elle n'en comprenne pas, la composition peut également comprendre en outre au moins un agent tensioactif qui est généralement présent en une quantité comprise entre 0,01% et 50% en poids, de préférence entre 0,1% et 40% et encore plus préférentiellement entre 0,5% et 30%, par rapport au poids total de la composition.
Cet agent tensioactif peut être choisi parmi les agents tensioactifs anioniques, amphotères, non ioniques, cationiques ou leurs mélanges.

Parmi les tensioactifs anioniques susceptibles d'être utilisés, on peut citer, seuls ou mélanges, les sels (en particulier sels de métaux alcalins, notamment de sodium, sels d'ammonium, sels d'amines, sels d'aminoalcools ou sels de magnésium) des composés suivants : les alkylsulfates, les alkyléthersulfates, alkylamidoéther-sulfates, alkylarylpolyéther-sulfates, monoglycérides sulfates, les alkylsulfonates, alkylphosphates, alkylamidesulfonates, alkylarylsulfonates, α-oléfine-sulfonates, paraffine-sulfonates; les alkylsulfosuccinates, les alkyléthersulfosuccinates, les alkylamide-sulfosuccinates; les alkylsulfosuccinamates; les alkylsulfoacétates ; les alkylétherphosphates; les acylsarcosinates; les acyliséthionates et les N-acyltaurates, le radical alkyle ou acyle de tous ces différents composés comportant de préférence de 8 à 24 atomes de carbone, et le radical aryle désignant de préférence un groupement phényle ou benzyle.
On peut également citer les sels d'acides gras tels que les sels des acides oléique, ricinoléique, palmitique, stéarique, les acides d'huile de coprah ou d'huile de coprah hydrogénée; les acyl-lactylates dont le radical acyle comporte 8 à 20 atomes de carbone; les acides d'alkyl D galactoside uroniques et leurs sels ainsi que les acides alkyl (C₆-C₂₄) éther carboxyliques polyoxyalkylénés, les acides alkyl(C₆-C₂₄)aryl éther carboxyliques polyoxyalkylénés, les acides alkyl(C₆-C₂₄) amido éther carboxyliques polyoxyalkylénés et leurs sels, en particulier ceux comportant de 2 à 50 groupements oxyde d'éthylène, et leurs mélanges.

Parmi les tensioactifs non ioniques susceptibles d'être utilisés, on peut citer, seuls ou mélanges, les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30.
On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les esters d'acides gras du sorbitane oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀-C₁₄) amines ou les oxydes de N-acylaminopropylmorpholine.

Parmi les tensioactifs amphotères susceptibles d'être utilisés, on peut citer, seuls ou mélanges, les dérivés d'amines secondaires ou tertiaires aliphatiques,
dans lesquels le radical aliphatique est une chaîne linéaire ou ramifiée comportant 8 à 22 atomes de carbone et comprenant au moins un groupe anionique hydrosolubilisant (par exemple carboxylate, sulfonate, sulfate, phosphate ou phosphonate); on peut citer encore les alkyl (C₈-C₂₀) bétaïnes, les sulfobétaïnes, les alkyl (C₈-C₂₀) amidoalkyl (C₁-C₆) bétaïnes telles que la cocoamidopropylbétaïne ou les alkyl (C₈-C₂₀) amidoalkyl (C₁-C₆) sulfobétaïnes.

Parmi les tensioactifs cationiques susceptibles d'être utilisés, on peut citer, seuls ou en mélange, :
- les sels d'ammonium quaternaires de la formule générale suivante: 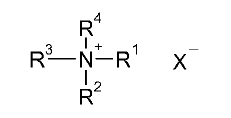 dans laquelle X est un anion choisi parmi les halogénures (chlorure, bromure ou iodure) ou alkyl(C₂-C₆)sulfates plus particulièrement méthylsulfate, des phosphates, des alkyl-ou-alkylarylsulfonates, des anions dérivés d'acide organique tel que l'acétate ou le lactate, et
   a) les radicaux R¹ à R³, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide,
      R⁴ désigne un radical alkyle, linéaire ou ramifié, comportant de 16 à 30 atomes de carbone.
      De préférence le tensioactif cationique est un sel (par exemple chlorure) de béhényl triméthyl ammonium.
   b) les radicaux R¹ et R², qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide et hydroxyalkyle, comportant environ de 1 à 4 atomes de carbone;
      R³ et R⁴, identiques ou différents, désignent un radical alkyle, linéaire ou ramifié, comportant de 12 à 30 atomes de carbone, ledit radical comprenant au moins une fonction ester ou amide.
      R³ et R⁴ sont notamment choisis parmi les radicaux alkyl (C₁₂-C₂₂)amido alkyle(C₂-C₆), alkyl(C₁₂-C₂₂)acétate ;
      De préférence le tensioactif cationique est un sel (par exemple chlorure) de stéaramidopropyl diméthyl (myristylacétate) ammonium.
- les sels d'ammonium quaternaire de type imidazolinium, comme par exemple ceux de formule suivante : 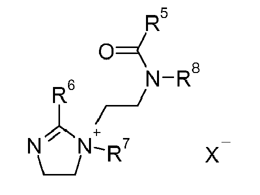 dans laquelle R⁵ représente un radical alcényle ou alkyle comportant de 8 à 30 atomes de carbone par exemple dérivés des acides gras du suif, R⁶ représente un atome d'hydrogène, un radical alkyle en C₁-C₄ ou un radical alcényle ou alkyle comportant de 8 à 30 atomes de carbone, R⁷ représente un radical alkyle en C₁-C₄ , R⁸ représente un atome d'hydrogène, un radical alkyle en C₁-C₄, X est un anion choisi dans le groupe des halogénures, phosphates, acétates, lactates, alkylsulfates, alkyl-ou-alkylarylsulfonates. De préférence, R⁵ et R⁶ désignent un mélange de radicaux alcényle ou alkyle comportant de 12 à 21 atomes de carbone par exemple dérivés des acides gras du suif, R⁷ désigne méthyle, R⁸ désigne hydrogène. Un tel produit est par exemple le Quaternium-27(CTFA 1997) ou le Quaternium-83 (CTFA 1997) commercialisés sous les dénominations "REWOQUAT" W 75, W90, W75PG, W75HPG par la société WITCO,
- les sels de diammonium quaternaire de formule suivante : 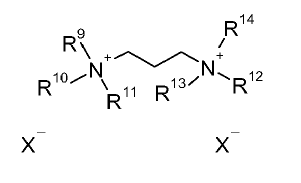 dans laquelle R⁹ désigne un radical aliphatique comportant environ de 16 à 30 atomes de carbone, R¹⁰, R¹¹, R¹², R¹³ et R¹⁴, identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium.
- les sels d'ammonium quaternaire comprenant au moins une fonction ester de formule suivante : 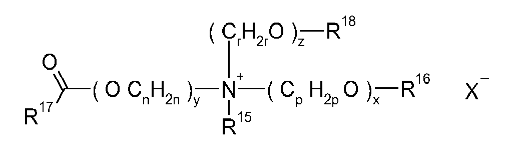 dans laquelle :
   - R¹⁵ est choisi parmi les radicaux alkyles en C₁-C₆ et les radicaux hydroxyalkyles ou dihydroxyalkyles en C₁-C₆ ;
   - R¹⁶ est choisi parmi l'hydrogène, le radical R¹⁹-C(O)- et les radicaux R₂₀ hydrocarbonés en C₁-C₂₂ linéaires ou ramifiés, saturés ou insaturés,
   - R¹⁸ est choisi parmi l'hydrogène, le radical R²¹-C(O)- et les radicaux R₂₂ hydrocarbonés en C₁-C₂₂ linéaires ou ramifiés, saturés ou insaturés:
   - R¹⁷, R¹⁹ et R²¹, identiques ou différents, sont choisis parmi les radicaux hydrocarbonés en C₇-C₂₂, linéaires ou ramifiés, saturés ou insaturés ;
   - n, p et r, identiques ou différents, sont des entiers valant de 2 à 6 ;
   - y est un entier valant de 1 à 10 ;
   - x et z, identiques ou différents, sont des entiers valant de 0 à 10 ;
   - X⁻ est un anion simple ou complexe, organique ou inorganique ;
      sous réserve que la somme x + y + z vaut de 1 à 15, et lorsque x vaut 0 alors R¹⁶ désigne R²⁰ et lorsque z vaut 0 alors R¹⁸ désigne R²².

Parmi les émulsionnants et les co-émulsionnants éventuellement utilisés dans la composition notamment sous forme d'émulsion, on peut citer, selon le sens de l'émulsion (E/H ou H/E), les esters d'acide gras et de polyol tels que le stéarate de PEG-100, le stéarate de PEG-50 et le stéarate de PEG-40 ; le tristéarate de sorbitane, les stéarates de sorbitane oxyéthylénés comprenant par exemple de 20 à 100 OE, et par exemple ceux disponibles sous les dénominations commerciales Tween^{®} 20 ou Tween^{®} 60, et leurs mélanges tels que le mélange de monostéarate de glycéryle et de stéarate de polyéthylène glycol (100 OE) commercialisé sous la dénomination SIMULSOL 165 par la société SEPPIC.
On peut citer aussi les émulsionnants siliconés tels que les diméthicone copolyols et les alkyl diméthicone copolyols. On peut citer par exemple comme diméthicone copolyol, le mélange de diméthicone copolyol, de cyclométhicone et d'eau (10/88/2), commercialisé par la société Dow Corning sous la dénomination DC3225C ou DC2-5225C, et comme alkyl diméthicone copolyol, ceux ayant un radical alkyle comportant de 10 à 22 atomes de carbone, tels que le cétyl diméthicone copolyol comme le produit commercialisé sous la dénomination Abil EM-90 par la société Goldschmidt et le mélange de diméthicone copolyol et de cyclopentasiloxane (85/15) commercialisé sous la dénomination Abil EM-97 par la société Goldschmidt ; le lauryl diméthicone copolyol et par exemple le mélange d'environ 91% de lauryl diméthicone copolyol et d'environ 9% d'alcool isostéarylique, commercialisé sous la dénomination Q2-5200 par la société Dow Corning, et leurs mélanges.
Ces émulsionnants et co-émulsionnants sont de préférence présents, dans la composition, en une proportion allant de 0,3 à 30% en poids, et de préférence de 0,5 à 20% en poids par rapport au poids total de la composition.

Parmi les gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles.
Comme gélifiants lipophiles, on peut citer les argiles modifiées comme les bentones, les sels métalliques d'acides gras et la silice hydrophobe.

Comme actifs cosmétiques, on peut utiliser notamment les dépigmentants, les émollients, les hydratants, les oligo-éléments, les anti-séborrhéiques, les anti-acnéiques, les agents favorisant la repousse des cheveux, les agents kératolytiques et/ou desquamants, les agents antirides et tenseurs, les agents anti-irritants, les agents apaisants, les vitamines, les filtres UV, les absorbeurs d'odeur, les anti-oxydants, les agents anti-chutes des cheveux, les agents antipelliculaires, les agents propulseurs, les céramides, et leurs mélanges.

Les compositions selon l'invention peuvent se présenter sous toutes les formes galéniques classiquement utilisées pour une application topique et notamment sous forme d'une solution, dispersion ou suspension aqueuse, alcoolique ou hydroalcoolique ou d'une solution huileuse, éventuellement épaissies ou gélifiées; d'une émulsion huile-dans-eau, eau-dans-huile, ou multiple, de consistance liquide ou semi liquide du type lait ou bien de consistance molle de type crème; d'un gel aqueux ou anhydre, d'une mousse; d'un gel huileux ou émulsionné; d'une dispersion de vésicules notamment lipidiques; d'une lotion biphase ou multiphase; d'un spray; ou de toute autre forme cosmétique.
L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

La composition cosmétique selon l'invention peut se présenter sous la forme d'un produit de soin, de nettoyage et/ou de maquillage de la peau du corps ou du visage, des lèvres, des cils, des ongles et des cheveux, d'un produit solaire ou autobronzant, d'un produit d'hygiène corporelle, d'un produit capillaire, notamment de soin, de nettoyage, de coiffage ou de coloration des cheveux.
Elle trouve notamment une application particulièrement intéressante dans le domaine capillaire, notamment pour le maintien de la coiffure ou la mise en forme des cheveux, ou encore le nettoyage des cheveux. Les compositions capillaires sont de préférence des shampooings, des après-shampooings, des gels de coiffage ou de soin, des lotions ou crèmes de soin, des conditionneurs, des lotions de mise en plis, des lotions pour le brushing, des compositions de fixation et de coiffage telles que les laques ou spray. Les lotions peuvent être conditionnées sous diverses formes, notamment dans des vaporisateurs, des flacons-pompe ou dans des récipients aérosol afin d'assurer une application de la composition sous forme vaporisée ou sous forme de mousse.
Elle peut notamment se présenter sous la forme d'un produit de coloration capillaire; ou sous forme de composition pour permanente, défrisage, ou décoloration, ou encore sous forme de compositions à rincer, à appliquer avant ou après une coloration, une décoloration, une permanente ou un défrisage ou encore entre les deux étapes d'une permanente ou d'un défrisage.
La composition selon l'invention peut également se présenter sous la forme d'une composition de soin, notamment hydratant, pour la peau, les lèvres et/ou les phanères, ou sous forme d'une composition de nettoyage de la peau, par exemple un produit démaquillant ou un gel pour le bain ou la douche.
Elle peut aussi se présenter sous forme d'un produit de soin, non coloré, destiné à traiter la peau et notamment à l'hydrater, la lisser, la dépigmenter, la nourrir, la protéger des rayons solaires, ou lui conférer un traitement spécifique. A cet effet, elle contient avantageusement au moins un actif de soin choisi parmi les dépigmentants, les émollients, les hydratants, les anti-séborrhéiques, les anti-acnéiques, les agents favorisant la repousse des cheveux, les agents kératolytiques et/ou desquamants, les agents antirides et tenseurs, les agents antiirritants, les agents apaisants, les vitamines, les filtres, les absorbeurs d'odeur et leurs mélanges.
Elle peut également se présenter sous forme d'une composition d'hygiène corporelle notamment sous forme de produit déodorant, anti-transpirant, ou encore sous forme d'une composition dépilatoire.
Elle peut encore se présenter sous la forme d'un produit de maquillage, en particulier coloré, de la peau du corps ou du visage, ou des cheveux, en particulier un fond de teint, présentant éventuellement des propriétés de soin, un blush, un fard à joues ou à paupières, un produit anti-cerne, un eye-liner; un produit de maquillage des lèvres comme un rouge à lèvres, présentant éventuellement des propriétés de soin, un brillant à lèvres, les crayons à lèvres; un produit de maquillage des phanères comme les ongles, les cils en particulier sous forme d'un mascara pain, les sourcils et les cheveux; un produit de tatouage temporaire de la peau du corps.

L'invention a aussi pour objet un procédé de traitement cosmétique, notamment de maquillage, de soin, de nettoyage ou de coloration, des matières kératiniques, notamment de la peau du corps ou du visage, des ongles, des cheveux, des poils et/ou des cils, comprenant l'application sur lesdites matières d'une composition cosmétique telle que définie précédemment.
L'application peut éventuellement être suivie d'un rinçage à l'eau. Ainsi, ce procédé selon l'invention permet le maintien de la coiffure, le traitement cosmétique, le soin, le maquillage, le lavage ou le démaquillage, entre autre, de la peau, des cheveux et/ou de toute autre matière kératinique.

L'invention a également pour objet un procédé pour améliorer à la fois la persistance d'au moins un effet apporté après dépôt par une composition cosmétique et l'adhésion de la composition appliquée sur les matières kératiniques, et pour permettre également une élimination rapide, totale et sélective du dépôt, qui consiste à ajouter à la composition une quantité efficace d'au moins un copolymère selon l'invention tel que défini précédemment.
L'élimination du dépôt (ou film) peut consister notamment en le rinçage d'une composition nettoyante ou en un démaquillage d'un dépôt de maquillage (rouge à lèvres, fond de teint, mascara, eye-liner notamment). Cette élimination peut être réalisée de manière sélective en utilisant un solvant contenant un rupteur des interactions hydrogène et pouvant pénétrer dans le dépôt.

L'invention est illustrée plus en détail dans les exemples suivants, dans lesquels les quantités sont données en pourcentage en poids. Les équivalents (equiv) sont des équivalents molaires.

### Détermination des masses molaires moyennes en poids et en nombre

On détermine les masses molaires moyennes en poids (Mw) et en nombre (Mn) par chromatographie liquide par perméation de gel ou GPC (solvant THF, courbe d'étalonnage établie avec des étalons de polystyrène linéaire, détecteur réfractométrique).

La GPC est réalisée avec des colonnes STYRAGEL HR4 / 7,8 x 300 mm, vendues par Waters WAT044225.
La détection est effectuée avec un réfractomètre WATERS 410.
L'éluant est le tétrahydrofurane (THF), à un débit de 1 ml/minute.
Le volume injecté est de 50 microlitres, à 25°C.

### Détermination de la température de transition vitreuse (Tg)

La température de transition vitreuse est mesurée par analyse calorimétrique différentielle (DSC) dans les conditions suivantes :
on réalise un film ayant une épaisseur d'environ 150 mm du polymère à tester en déposant une solution ou une dispersion aqueuse du polymère dans une matrice circulaire en téflon de 40 mm de diamètre et en laissant sécher le dépôt. Le film est mis à sécher dans une étuve à une température d'environ 23°C sous une humidité relative de 45%, jusqu'à ce que le poids ne varie plus. On prélève environ 5 à 15 mg du film, que l'on place dans un creuset qui est introduit ensuite dans l'analyseur. L'analyseur thermique est un modèle DSC-2920 de la société TA INSTRUMENTS. Les températures initiales et finales du balayage en température sont choisies de manière à encadrer la température de transition vitreuse recherchée. Le balayage en température se fait à une vitesse de 10 °C/minute.
   Cette analyse est effectuée selon la norme ASTM D 3418-97 aux modifications ci-dessus près.

### Exemple 1 : Préparation d'un monomère uréidopyrimidone fonctionnalisé diisocyanate, de formule

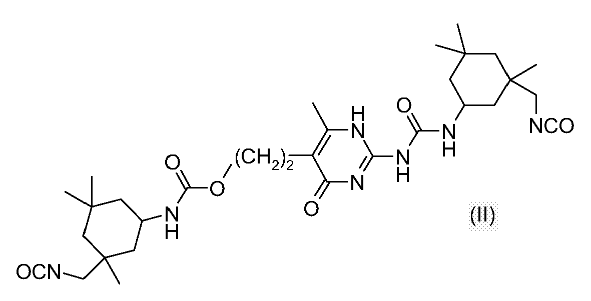

On met en suspension 12 g de 5-(2-hydroxyéthyl)-6-méthyl-isocytosine dans 150 ml de diisocyanate d'isophorone; le mélange est agité sous argon pendant 12 heures à 90°C. La solution limpide résultante est refroidie et précipitée dans l'hexane. Le précipité est isolé par filtration, lavé à l'hexane puis séché. On obtient 46 g de solide, soit un rendement de 93%.

### Exemple 2 : Préparation d'un copolymère polyuréthane-polyurée-polyester portant une fonction ionisable

Réactifs mis en jeu 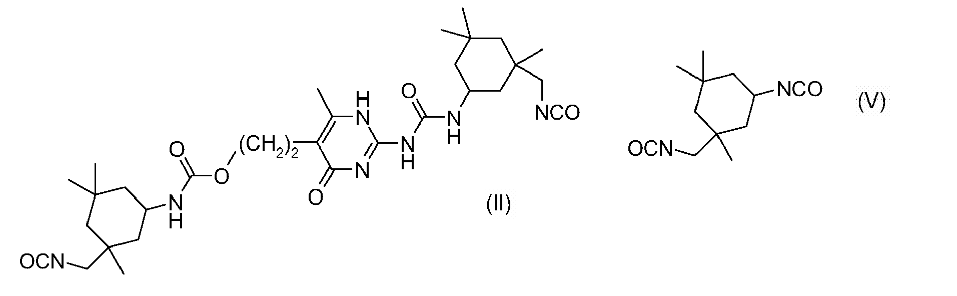 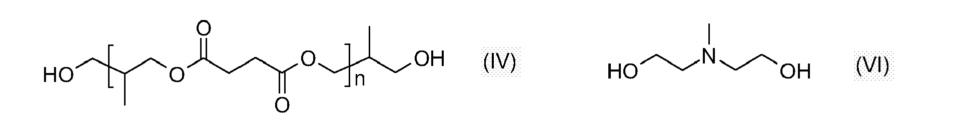 On mélange dans 250 ml de chloroforme:
- 16,3 g (1 equiv) de poly(2-méthyl-1,3-propylène-glutarate) de poids moléculaire moyen Mn = 1,0 kDa (monomère (b1) polyester diol téléchélique fonctionnalisé OH de formule (IV)),
- 7,1 g (1 equiv) de diisocyanate d'isophorone de formule (V),
- 8,4 g (6/7 equiv) de monomère de formule (II) préparé à l'exemple 1, et
- 3,6 g (1 equiv) de N-méthyl-diéthanolamine de formule (VI) (monomère (b2)).

On ajoute quelques gouttes de dilaurate de dibutyle étain (catalyseur). Le mélange est chauffé à 60°C pendant 16 heures. Le polymère est précipité dans l'hexane puis séché sous pression réduite.

Le polymère désiré est obtenu avec un rendement de 96%.
La masse en nombre (Mn) du polymère obtenu est d'environ 13 000 Da.

### Exemple 3 : composition de vernis à ongles

On solubilise 4,8 g de polymère obtenu à l'exemple 2 dans 30 ml de THF, auxquels sont ajoutés 22 g d'acétate de butyle, puis le THF est évaporé.
On obtient ainsi une solution visqueuse contenant 17,9% en poids de polymère. La viscosité peut être diminuée si nécessaire par addition de 2 g d'éthanol.
Après application sur l'ongle de cette solution, on obtient un film transparent et brillant.

### Exemple 4 : composition de spray capillaire

On met en dispersion 16 g de polymère obtenu à l'exemple 2 dans l'eau via le procédé suivant : 16 g de polymère sont dissous dans 64 g de THF, le milieu est visqueux. 10 g d'éthanol sont ajoutés, puis 14,4 g de HCl 1 N afin de neutraliser la totalité des groupes issus de la N-méthyl-diéthanolamine. On ajoute ensuite 76,3 g d'eau sous agitation au moyen d'un barreau magnétique, à température ambiante. Le THF et l'éthanol sont alors évaporés à 50°C, à l'évaporateur rotatif.
On obtient une solution transparente de faible viscosité légèrement jaunâtre.
Extrait sec final : 18,4 % (en masse).
Taille de particule mesurée par diffusion de lumière après forte dilution (via un appareil Coulter N4-SD): 30 nm
pH=2,3

Le polymère en dispersion dans l'eau ainsi obtenu est ensuite placé dans un flacon pompe et vaporisé sur les cheveux. Le produit conduit à une fixation de la chevelure qui tient dans le temps. La chevelure est brillante.

### Exemple 5 : Préparation d'un copolymère polyuréthane-polyurée-poly(éthylènebutylène) portant une fonction ionisable

On dissout dans un mélange séché de 120ml de toluène et de 20ml de pyridine, 26,2 g de 1,2-polybutadiène hydrogéné di-OH (G13000 de Nisso-PB, Mn= 3100) en présence de 3,81 g de N-methyldiethylethanolamine, 7,12 g d'isophorone diisocyanate (V), et de 3 gouttes de catalyseur (dilaurate de dibutyle étain). Le mélange réactionnel est chauffé à 80°C sous argon pendant 2 heures.
A cette solution est alors additionné le monomère (II) préparé à l'exemple 1. Le mélange est chauffé durant 16 heures supplémentaires.
Le mélange réactionnel est concentré à 50% du volume initial par évaporation sous vide, on ajoute 50 ml de toluène et on évapore à nouveau, l'opération étant répétée deux fois.
Le mélange est alors dissous dans un mélange chloroforme/méthanol (9 volume/1 volume), et le polymère désiré est obtenu par précipitation dans un large volume de méthanol (excès de 10 volumes). Le précipité est filtré et séché sous vide.
On obtient 40,2 g de polymère recherché, caractérisé par GPC (THF): sa masse est de Mn = 9,6 kDa avec un indice de dispersité de 1,7.

### Exemple 6 : composition capillaire

On met en solution 15,4 g du polymère de l'exemple 5 dans 46,2 g de THF puis on ajoute 11,6 g de HCl 1N et 87,3 g d'eau. On évapore la dispersion au rotavapor à 50% sous vide pour obtenir une dispersion aqueuse.
Extrait sec final : 13.8% (en poids)
Taille de particule mesurée par diffusion de la lumière (via un appareil Coulter N4-SD) (après forte dilution) : 300à 500 nm
pH=2,7

La solution peut ensuite être placée dans un flacon pompe et vaporisée sur les cheveux. On observe une fixation de la chevelure, qui tient dans le temps; la chevelure est brillante.

### Exemple 7 : Préparation d'un copolymère polyuréthane-polyurée-polyester portant une fonction ionisable acide carboxylique

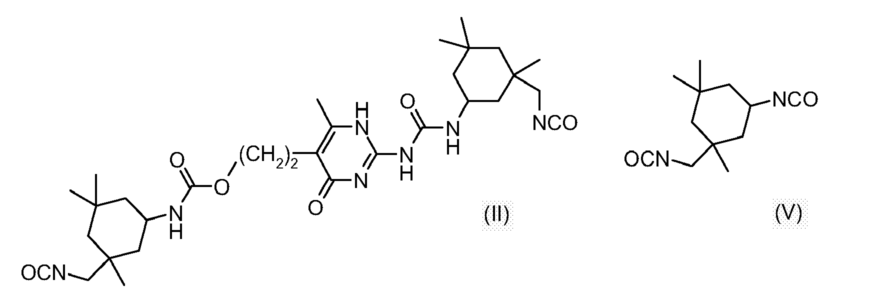

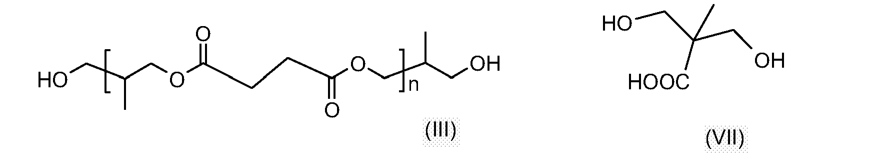

Un mélange de 13 g de poly(2-méthyl-1,3-propylène-glutarate) (III) de Mn=1kDa et de 3,42 g de 2,2-bis(hydroxymethyl)propionic acid (VII) est séché sous vide à 110°C pendant deux heures avant d'être mis en solution dans un mélange de solvant au préalable séché (40ml de toluène + 40ml de methylethylcétone) en présence de 5,66 g d'isophorone diisocyanate (V) et une quantité catalytique de dilaurate de dibutyle étain (3 gouttes).
Le mélange réactionnel est ensuite chauffé à 80°C, sous argon pendant 2 heures, suivi de l'addition de 6,70 g de monomère (II).
Le mélange ainsi obtenu est chauffé durant 16 heures supplémentaires, sous argon. On ajoute 10 ml de méthanol au mélange réactionnel, suivi d'une précipitation du polymère désiré dans un large excès d'hexane. On filtre puis on sèche sous pression réduite et l'on obtient 28,3 g du polymère recherché, caractérisé par GPC (THF): sa masse est de Mn = 5,3 kDa avec un indice de dispersité de 2,5.

### Exemple 8: Composition de vernis à ongles

On met en solution 9,3 g du polymère obtenu dans l'exemple 7 dans 40 g d'acétate d'éthyle en présence de 9 g d'éthanol, et après un chauffage à 50°C pendant 15 heures. Cette solution permet d'obtenir des films brillants après application sur la surface d'un ongle.

### Exemple 9 : Préparation d'un copolymère polyuréthane-polyester-polyester ne portant pas de fonction ionisable

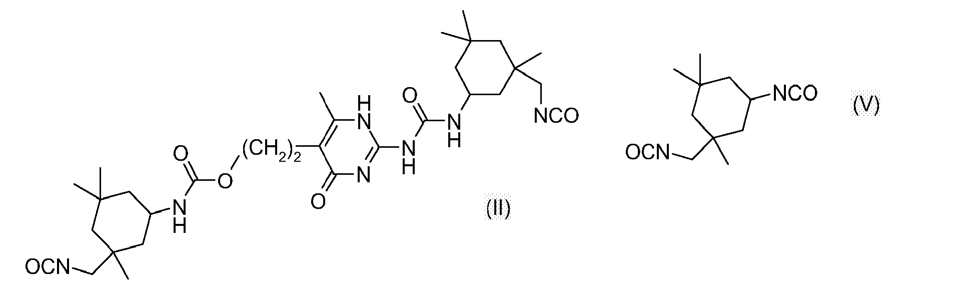

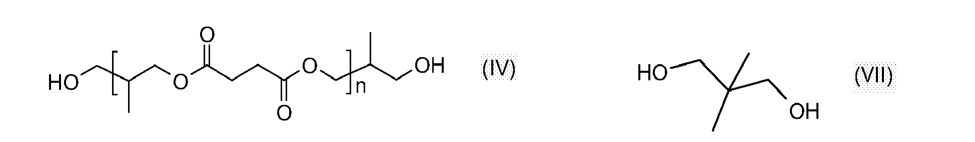

Un mélange de 14,8 g de poly(2-méthyle-1,3-propylène glutarate) Mn=1kDa (IV) et de 3,03 g de 2,2-dimethyl-1,3-propanediol (VII) est séché sous vide à 110°C pendant deux heures avant d'être mis en solution dans un mélange de 60 ml de toluène sec + 40 ml de méthyléthylcétone, en présence de 6,74 g d'isophorone diisocyanate (V) et une quantité catalytique de dilaurate de dibutyle étain (3 gouttes).
Le mélange réactionnel est ensuite chauffé à 80°C, sous argon pendant 2 heures, suivi de l'addition de 7,65 g de monomère (II).
Le mélange ainsi obtenu est chauffé durant 16 heures supplémentaires, sous argon. On ajoute 10 ml d'éthanol au mélange réactionnel, suivi d'une précipitation du polymère dans un large excès d'hexane. On filtre et on sèche sous vide et l'on obtient 31,4 g du polymère recherché, caractérisé par GPC (THF): sa masse est de Mn = 9,3 kDa avec un indice de dispersité de 2,5.

### Exemple 10 : Composition de vernis à ongles

On met en solution 9,18 g du polymère de l'exemple 9 dans 40 g d'acétate d'éthyle en présence de 9 g d'éthanol, après agitation et un chauffage à 50°C pendant 15 heures. Cette solution permet d'obtenir des films brillants après application sur la surface d'un ongle.

## Revendications

1. Composition cosmétique comprenant, dans un milieu physiologiquement acceptable, un copolymère ayant un squelette polymérique (thio)uréthane/(thio)urée, résultant de la réaction :
- d'au moins un premier monomère (a) comportant au moins deux groupes polymérisables, identiques ou différents, choisis parmi -N=C=O et -N=C=S, et/ou leur forme activée ou bloquée; de préférence deux groupes -N=C=O ;
- d'au moins un monomère (b1), comportant au moins deux groupes polymérisables à hydrogène labile, identiques ou différents, choisis parmi -OH, -SH, - NH₂ et -NHR, avec R représentant un groupe alkyle en C₁-C₆ ; et
- d'au moins un monomère (b2), différent du monomère (b1), comportant au moins deux groupes polymérisables à hydrogène labile, identiques ou différents, choisis parmi -OH, -SH, -NH₂ et -NHR, avec R représentant un groupe alkyle en C₁-C₆ ; et
- l'un au moins des monomères (a), (b1) et/ou (b2) comprenant au moins un groupe de jonction (A), capable de former au moins 3 liaisons H, de préférence au moins 4 liaisons H et plus préférentiellement 4 liaisons H.

2. Composition selon la revendication 1, dans laquelle le groupe de jonction (A) est un groupe chimique comportant au moins 3 hétéroatomes, identiques ou différents, de préférence 4 hétéroatomes, identiques ou différents, choisis dans le groupe constitué de O, N, S, P et F, de préférence parmi O, S et N.

3. Composition selon l'une des revendications précédentes, dans laquelle le groupe de jonction comprend au moins 3 groupes fonctionnels, de préférence au moins 4, choisis parmi :

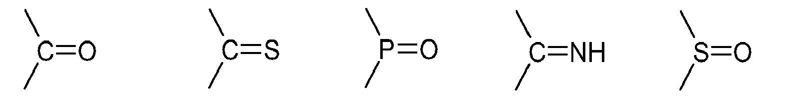

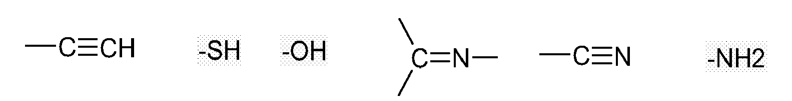

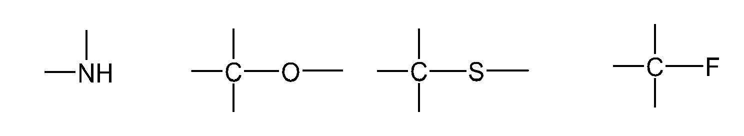

4. Composition selon l'une des revendications précédentes, dans laquelle le groupe de jonction (A) est choisi parmi les familles suivantes, étant entendu que toutes les formes tautomériques sont inclues :
- (i) les aminopyrimidones de formule : 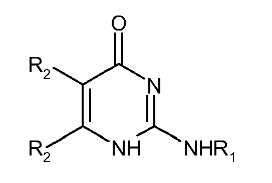
- (ii) les ureïdopyrimidones de formule : 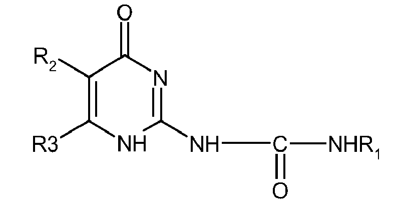
- (iii) les acylaminopyridines et notamment :
- les monoacylaminopyridines de structure : 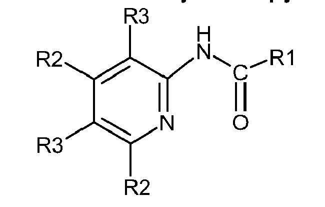
- les di(acylamino)pyridines et plus particulièrement les 2,6-di(acylamino)pyridines de structure : 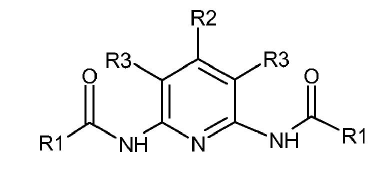
- (iv) les aminopyrimidines, et notamment :
- les composés aminopyrimidines : 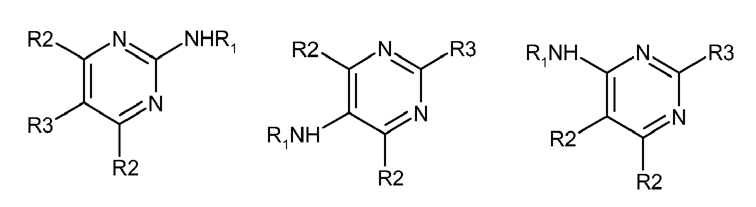
- les composés diaminopyrimidines de structure : 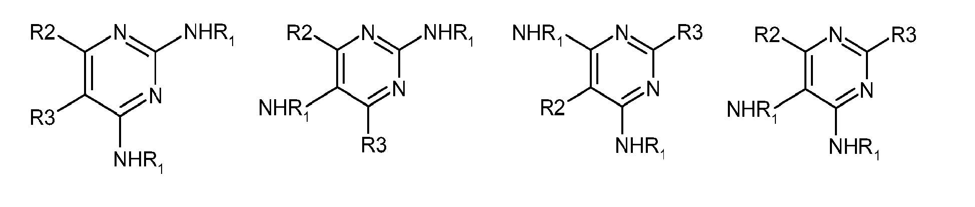
- les composés triaminopyrimidines;
- (v) les uréïdotriazines, et notamment les mono-, di- et tri-uréïdotriazines, et en particulier les uréïdoaminotriazines de structure : 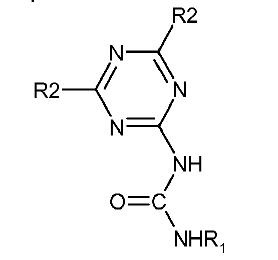
- (vi) les (acylamino)triazines, et notamment les mono-, di- et tri-acylamino triazines, éventuellement amino (mono-, di- ou tri-amino) et en particulier :
- les di(acylamino)triazines de structure : 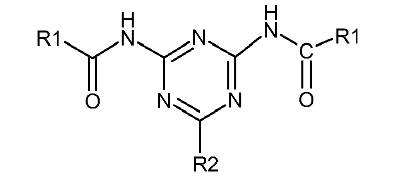
- les acylamino, amino-triazines, (mono ou di- acylamino, et mono- ou di-amino) et notamment les composés de structure : 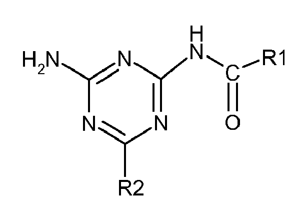
- les acylaminotriazines de structure : 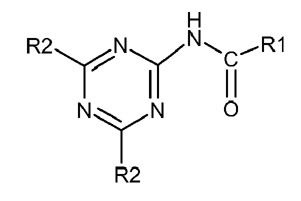
- les tri-acylamino triazines,
- (vii) les aminotriazines et notamment :
- les monoaminotriazines,
- les 2,6-diamino-s-triazines de structure : 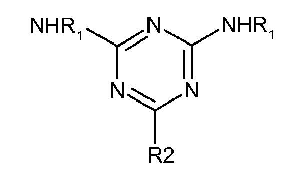
- les composés triamino-s-triazines de structure : 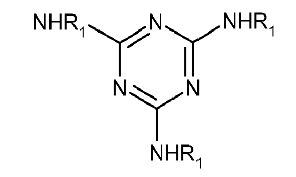
- (viii) les acylaminotriazoles de structure : 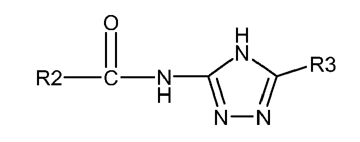
- (ix) les composés de la famille de l'acide urazoyl benzoïque de structure : 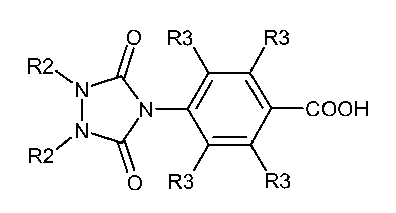
- (x) les phtalhydrazides de structure : 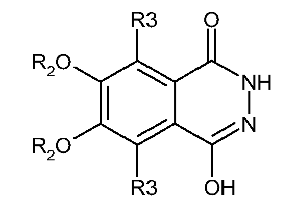
- (xi) les uraciles de structure : 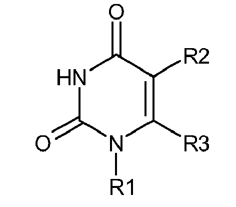
- (xii) les thymines de structure : 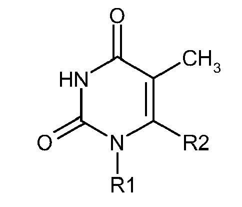
- (xiii) les succinimides de structure : 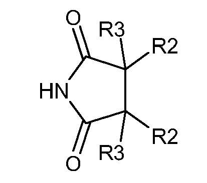
- (xiv) les glutarimides de structure : 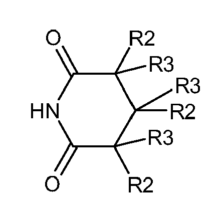
- (xv) les composés de la famille de l'acide cyanurique de structure : 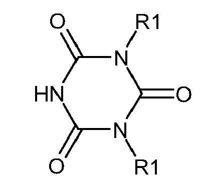
- (xvi) les maléimides : 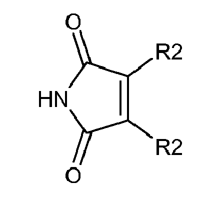
- (xvii) les composés de la famille de l'acide barbiturique, de structure : 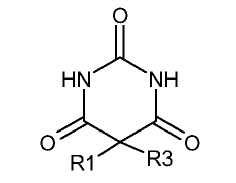
- (xviii) les composés de structure : 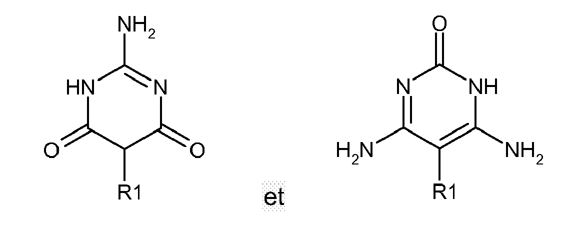
- (xix) les composés de la famille de l'acide triméllitique, de formule : 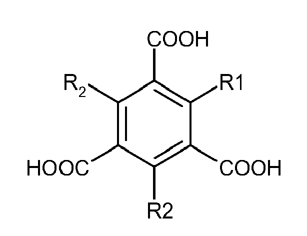
- (xx) les uréïdopyridines, notamment les mono- ou di-uréïdopyridines, et en particulier ceux de formule : 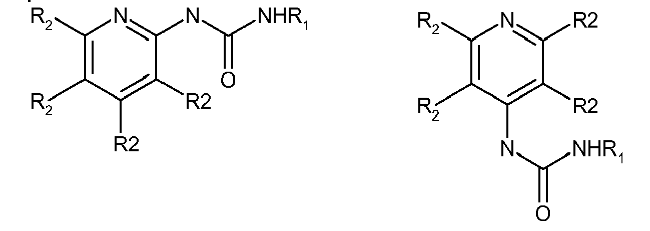 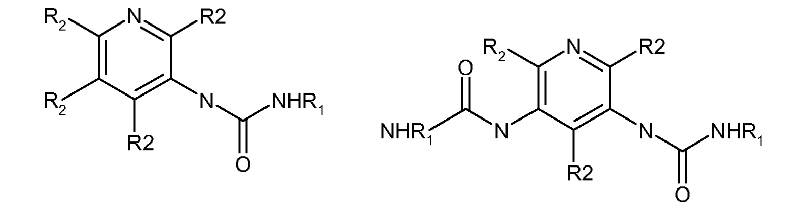 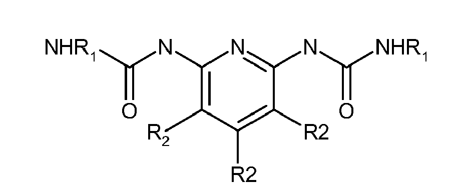
- (xxi) les carbamoylpyridines, de formule : 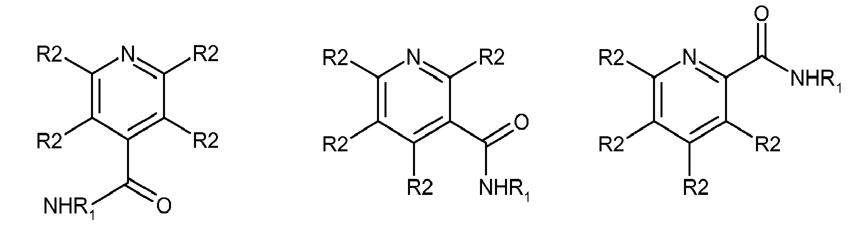
- (xxii) les adénines de formule : 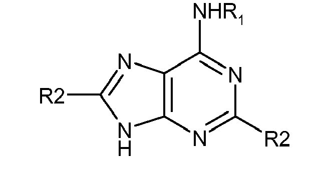
- (xxiii) les guanines de formule : 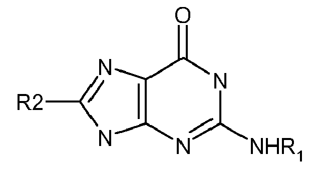
- (xxiv) les cytidines de formule : 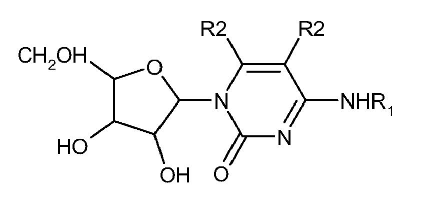 dans lesquelles la signification des radicaux est la suivante :
- (a) les radicaux R¹, identiques ou différents, représentent H, halogène et/ou un groupe carboné (notamment alkyle) monovalent, linéaire, ramifié ou cyclique, en C₁-C₆₀₀₀, saturé ou non, éventuellement aromatique, pouvant contenir un ou plusieurs hétéroatomes tels que O, S, N, P, Cl, Br, Cl, Br, F; ou une combinaison de ces significations;
- (b) les radicaux R2, identiques ou différents au sein d'une même formule, représentent H, halogène (-BR, -Cl, -F), -OH, -N(R)2 (avec R étant H ou un radical alkyle, linéaire ou ramifié en C1-C12, de préférence en C1-C4 et mieux un radical méthyle ou éthyle); ou un groupe hydrocarboné monovalent, linéaire, ramifié ou cyclique, en C1-C6000, saturé ou non, éventuellement aromatique, pouvant contenir un ou plusieurs hétéroatomes tels que O, S, N, P, F; ou une combinaison de ces significations;
- (c) les radicaux R³, identiques ou différents au sein d'une même formule, représentent H ou un groupe hydrocarboné monovalent, linéaire, ramifié ou cyclique, en C₁-C₆₀₀₀, saturé ou non, éventuellement aromatique, pouvant contenir un ou plusieurs hétéroatomes tels que O, S, N, P, F; ou une combinaison de ces significations;
étant entendu qu'au moins un, notamment un ou deux, des groupes R¹ et/ou R² est le point d'accroche du groupe de jonction A sur le squelette polymérique - POL-.

5. Composition selon la revendication 4, dans laquelle :
- (i) R₁ est un groupement : -C₄H₉, phényle ; 1,4-nitrophényle, ou . 1,2-éthylène, 1,6-héxylène, 1,4-butylène, 1 ,6-(2,4,4-triméthylhexylène), 1,4-(4-méthylpentylène), 1,5-(5-méthylhexylène), 1,6-(6-méthylheptylène), 1,5-(2,2,5-triméthylhexylène) ou 1,7-(3,7-diméthyloctylène); -isophorone-, 4,4'-méthylène biscyclohexylène, tolylène , 2-méthyl-1,3-phénylène, 4-méthyl-1,3-phénylène, 4,4-biphénylèneméthylène, et de préférence : -isophorone-, -(CH₂)₂-, -(CH₂)₆-, -CH₂CH(CH₃)-CH₂-C(CH₃)₂-CH₂-CH₂, 4,4'-méthylène biscyclohexylène, 2-méthyl-1,3-phénylène; et/ou
- (ii) les radicaux R² sont choisis parmi H, CN, NH₂ ou bien :
- un groupe alkyle en C₁-C₃₀ ;
- un groupe cycloalkyle en C₄-C₁₂ ;
- un groupe aryle en C₄-C₁₂ ;
- un groupe aryl(C₄-C₁₂) alkyle en C₁-C₃₀
- un groupe alcoxy en C₁-C₄;
- un groupe arylalcoxy, en particulier un groupe aryle (C₁-C₄) alcoxy ;
- un hétérocycle en C₄-C₁₂
- un groupe thioalcoxy,
- un groupe sulfoxy,
ou leurs mélanges
ces groupes étant éventuellement substitués par une fonction amino, ester et/ou hydroxy; et/ou
- (iii) le radical R³ est un groupe cycloalkyle en C₄-C₁₂; un groupe alkyle linéaire ou ramifié en C₁-C₃₀ ou un groupe aryle en C₄-C₁₂; éventuellement substitués par une fonction amino, ester et/ou hydroxy; et/ou
- ledit point d'accroche est porté par R¹ et/ou R² et de préférence lorsque le point d'accroche est unique, il est porté par le groupe R¹.

6. Composition selon l'une des revendications précédentes, dans laquelle le groupe de jonction (A) est choisi parmi :
(a) les groupes de jonction (A) complémentaires et identiques c'est-à-dire autocomplémentaires, et notamment :
- les aminopyrimidones, les uréïdopyrimidones,
- les composés de la famille de l'acide triméllitique, ou de l'acide urazoyl benzoïque,
- les acylaminopyridines, les uréïdopyridines, les carbamoylpyridines,
- les acylaminotriazines, les uréidotriazines et notamment les uréïdoaminotriazines, les diamino-triazines,
- les acylaminotriazoles,
- les phtalhydrazides,
- les composés de formule : 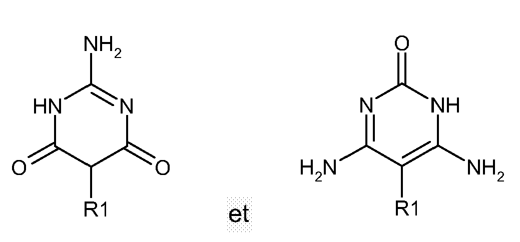
dans lesquelles R¹ est H ou un groupe hydrocarboné monovalent, linéaire, ramifié ou cyclique, en C₁-C₆₀₀₀, saturé ou non, éventuellement aromatique, pouvant contenir un ou plusieurs hétéroatomes tels que O, S, N, P, F.
(b) les groupes de jonction (A) complémentaires mais différents, et notamment :
- adénine complémentaire de guanine,
- cytidine complémentaire de thymine,
- triamino-s-triazine complémentaire de uracile ou de succinimide ou de glutarimide ou d'acide cyanurique ou de thymine ou de maléimide ou de (di)aminopyrimidine ou d'acide barbiturique;
- acylamino-amino-s-triazine complémentaire de uracile ou de succinimide ou de glutarimide ou d'acide cyanurique ou de thymine ou de maléimide ou de (di)aminopyrimidine ou d'acide barbiturique.

7. Composition selon l'une des revendications précédentes, dans laquelle le groupe de jonction est choisi parmi les groupes capables d'établir au moins trois liaisons H avec eux-mêmes (auto-complémentaire), notamment au moins quatre liaisons H avec eux-mêmes, et en particulier :
- les uréïdopyrimidones;
- les uréïdopyridines, les carbamoylpyridines;
- les acylamino-s-triazines et notamment les acyl-diamino-s-triazines;
- les uréidotriazines ;
- les phtalhydrazides;
- les composés de formule : 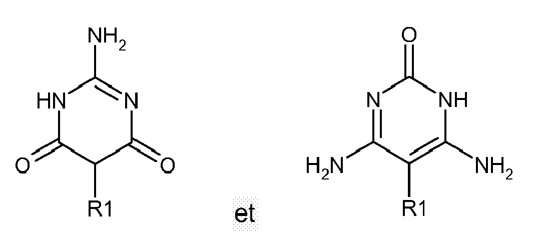 Dans lesquels les radicaux R1, R2 et R3 ont les significations données ci-dessus.

8. Composition selon l'une des revendications précédentes, dans laquelle le groupe de jonction est choisi parmi les groupes suivants:
- la 2-uréïdopyrimidone;
- la 6-méthyl, 2-uréïdopyrimidone;
- la diacyl de 2,6-diamino-s-triazine;
- l'uréido-s-triazine;
- les composés de formule : 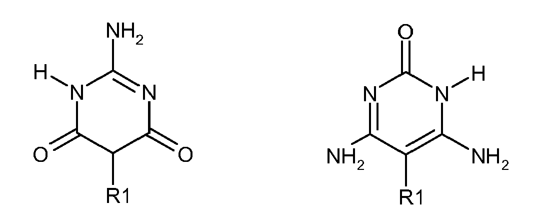
dans lesquelles R¹ est H ou un groupe hydrocarboné monovalent, linéaire, ramifié ou cyclique, en C₁-C₆₀₀₀, saturé ou non, éventuellement aromatique, pouvant contenir un ou plusieurs hétéroatomes tels que O, S, N, P, F.

9. Composition selon l'une des revendications précédentes, dans laquelle le copolymère comporte 1 à 12 groupes de jonction (A), de préférence 2 à 10, notamment 3 à 8, voire 4 à 6 groupes de jonction, par chaîne de copolymère.

10. Composition selon l'une des revendications précédentes, dans laquelle l'un au moins des monomères (a), (b1) et/ou (b2) comprend au moins deux groupes de jonction (A), capables de former au moins 3 liaisons H, de préférence au moins 4 liaisons H et plus préférentiellement 4 liaisons H.

11. Composition selon l'une des revendications précédentes, dans laquelle le monomère qui comporte le ou les groupes de jonction (A) est le monomère (a) et/ou (b2).

12. Composition selon l'une des revendications précédentes, dans laquelle le monomère qui comporte le ou les groupes de jonction (A) est le monomère (a).

13. Composition selon l'une des revendications précédentes, dans laquelle le monomère (a) est de formule Y=C=N-R^{a}-N=C=Y' ou B-C(O)-NH-R^{a}-NH-C(O)-B' avec :
- Y, Y', identique ou différent, représentant O ou S; de préférence Y=Y' et encore mieux Y=Y'=O;
- B et B' sont, indépendamment l'un de l'autre, choisis parmi : 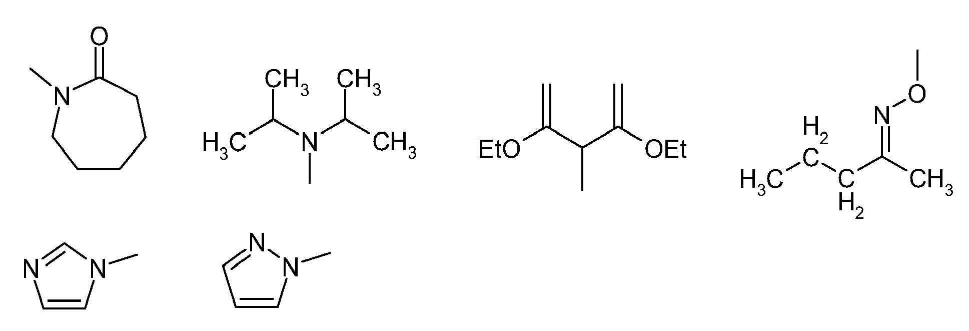
- R^{a} est un radical divalent, aliphatique, saturé ou non, linéaire ou ramifié, cyclique ou non, aromatique ou non, et qui comprend 1 à 40 atomes de carbone, comprenant éventuellement un ou plusieurs hétéroatomes choisis parmi O, S et/ou N, et/ou éventuellement substitué par un ou plusieurs atomes de fluor et/ou radicaux hydroxyle, et leurs mélanges.

14. Composition selon la revendication 13, dans laquelle le radical R^{a} est un groupe alkyle linéaire ou ramifié en C₁-C₃₀ ou un groupe cycloalkyle en C₄-C₁₂ ou un groupe aryle en C₄-C₁₂; éventuellement substitués par une fonction ester et/ou amide.

15. Composition selon la revendication 14, dans laquelle R^{a} est de structure :
- (CH₂)_{c}-, -(CRH)_{c} ou -(CRR')_{c} dans lesquelles R et R', identiques ou différents, représentent un groupe alkyle linéaire ou ramifié en C₁-C₃₀ et c est un entier de 1 à 20, de préférence 1 à 12,
ou bien de structure : 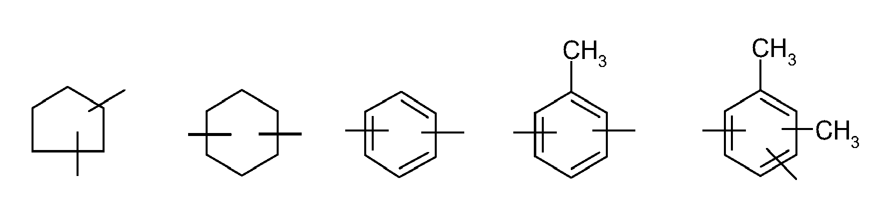 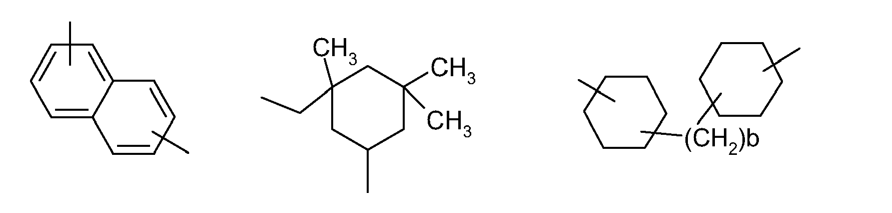 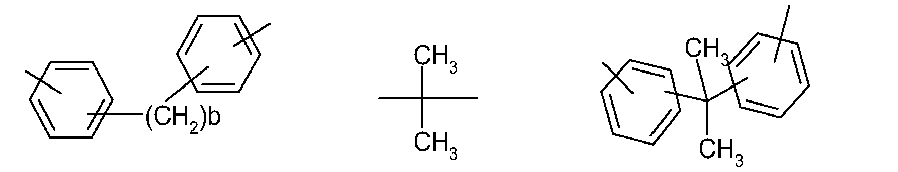 dans lesquelles b est un entier compris entre 0 et 3;
ainsi que toutes les combinaisons de ces structures.

16. Composition selon la revendication 15, dans laquelle R^{a} est choisi parmi les radicaux suivants: 1,2-éthylène, 1,6-héxylène, 1,4-butylène, 1,6-(2,4,4-triméthylhexylène), 1,4-(4-méthylpentylène), 1,5-(5-méthylhexylène), 1,6-(6-méthylheptylène), 1,5-(2,2,5-triméthylhexylène), 1,7-(3,7-diméthyloctylène); 4,4'- methylenebis(cyclohexyl 1,4-cyclohexylène; 2,4-tolylène, 2,6-tolylène; 1,5-naphtylène; 4,4'-methylenebis(phenyl); tétraméthyl xylylène; le radical divalent dérivé de l'isophorone.

17. Composition selon l'une des revendications précédentes, dans laquelle les monomères (a) sont choisis parmi les composés suivants:
1,4-diisocyanatobutane;
1,6-hexamethylenediisocyanate ou 1,6-diisocyanatohexane
1,5-diisocyanato-2-méthyl-pentane
1,4-diisocyanato-4-méthyl-pentane,
1,6-diisocyanato-2,2,4-triméthylhexane,
1,6-diisocyanato-2,4,4-triméthylhexane,
1,5-diisocyanato-5-méthylhexane,
3(4)-isocyanatométhyl-1-méthylcyclohexyl-isocyanate
1,6-diisocyanato-6-méthyl-heptane,
1,5-diisocyanato-2,2,5-triméthylhexane,
1,7-diisocyanato-3,7-diméthyloctane,
1-isocyanato-1,2,2-triméthyl-3-(2-isocyanato-éthyl)-cyclopentane,
1-isocyanato-*n*-butyl-3-(4-isocyanatobut-1-yl)-cyclopentane ;
1-isocyanato-1,2-diméthyl-3-éthyl-3-isocyanatométhylcyclopentane;
1-isocyanato-1-méthyl-4-(4-isocyanatobut-2-yl)-cyclohexane,
1-isocyanato-1,4-diméthyl-4-isocyanatométhyl-cyclohexane,
1-isocyanato-1,3-diméthyl-3-isocyanatométhyl-cyclohexane,
1,3-bis(isocyanatomethyl)cyclohexane
isophoronediisocyanate;
4,4'- methylenebis(cyclohexyl isocyanate)
1,4-diphenylene diisocyanate; tolylène 2,4-diisocyanate; tolylène 2,6-diisocyanate;
1,3-bis(isocyanatomethyl)benzène
4,4'-methylenebis(phényl isocyanate)
naphtalènediisocyanate;
tetramethyl-1,3-xylylenediisocyanate.

18. Composition selon l'une des revendications 1 à 12, dans laquelle le monomère (a) est de formule Y=C=N-R^{a}-N=C=Y' ou B-C(O)-NH-R^{a}-NH-C(O)-B' avec :
- Y, Y', identique ou différent, représentant O ou S; de préférence Y=Y' et encore mieux Y=Y'=O;
- B et B' sont, indépendamment l'un de l'autre, choisis parmi: 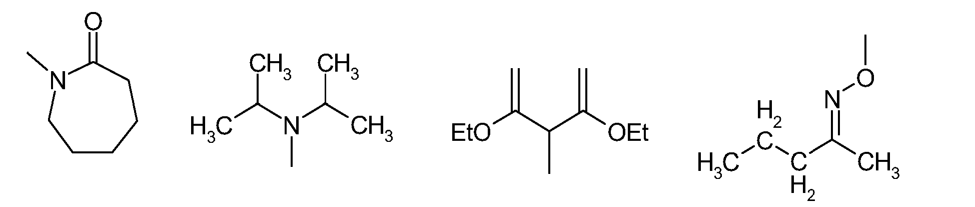 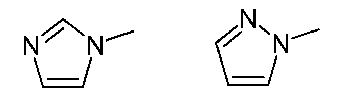
- R^{a} est un radical divalent polymérique, notamment de type homopolymère ou copolymère, choisi parmi :
- les copolymères éthyléniques tels que les polyoléfines comportant des motifs choisis parmi 1,2-butadiène; 1,4-butadiène; isoprène; éthylène; propylène; 1,2-butylène, 1,4-butylène; isobutylène; les copolymères (méth)acryliques, les copolymères (méth)acrylamides, les copolymères vinyliques, les copolymères allyliques et leurs mélanges.
- les polyéthers, perfluorés ou non, de type polyoxyde d'éthylène, polyoxyde de propylène, et leurs copolymères polyoxyde d'éthylène/polyoxyde de propylène, les polyoxydes de tétraméthylène et les perfluoropolyéthers ; les polythioéthers;
- les polyesters et notamment les polyesters à base d'acide adipique ou d'acide téréphtalique; la polycaprolactone; le poly(2-méthyl-1,3-propylène-adipate), le poly-(2-méthyl-1,3-propylène)-glutarate; les polyesters sulfoniques ;
- les polylactides ;
- les polyamides ;
- les polyoxazolines tels que la poly(2-méthyloxazoline) ou la poly(2-éthyloxazoline) ;
- les copolymères de siloxane, tels que par exemple les polysiloxanes constitués de motifs - Si(R⁴)(R⁵)O - où (R⁴) et (R⁵), identiques ou différents, représentent H ou un radical carboné, linéaire ou ramifié, cyclique ou non, saturé ou non, ou aromatique, de préférence un alkyle en C₁-C₁₂ pouvant éventuellement comporter un ou plusieurs, de préférence 1 à 5 hétéroatomes, identiques
ou différents, choisis parmi : O, N, S, P, F et Si, de préférence O, N et S, et notamment les polydiméthylsiloxanes (PDMS), et les poly(méthylphénylsiloxanes) ;
- les copolymères de ces différents types de polymères comme par exemple les copolymères polysiloxane / polyoxyde d'éthylène,
- les polyacétals ;
- les polycarbonates perfluorés ou non ;
- et leurs mélanges.

19. Composition selon la revendication 18, dans laquelle R^{a} a une masse molaire moyenne en poids (Mw) comprise entre 500 et 30 000, plus particulièrement entre 700 et 25 000 et mieux de 800 à 15 000.

20. Composition selon l'une des revendications 18 à 19, dans laquelle R^{a} est choisi parmi les polymères fonctionnels de type :
- polyesters, plus particulièrement à base d'acide adipique et/ou d'acide téréphtalique; le poly(2-méthyl-1,3-propylène-adipate) et le poly-(2-méthyl-1,3-propylène)glutarate;
- polyéthers et notamment les polyoxydes de tétraméthylène,
- les copolymères de siloxane; et
- les poly(éthylène-butylène) et les polybutadiènes.

21. Composition selon l'une des revendications précédentes, dans laquelle le monomère (a) comprend au moins un groupe de jonction (A), et est de formule : 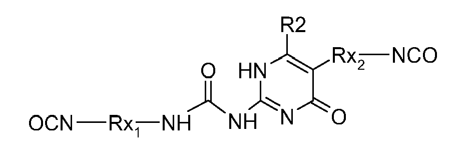 dans laquelle R_{X1}, Rx₂, identiques ou différents, représentent un radical carboné divalent choisi parmi un groupe alkyle linéaire ou ramifié en C₁-C₃₀ ou un groupe cycloalkyle en C₄-C₁₂ ou un groupe aryle en C₄-C₁₂; comprenant éventuellement 1 à 8 hétéroatomes choisis parmi O, N, S, F, Si et P; éventuellement substitués par une fonction ester, amide, ou par un radical alkyle en C₁-C₁₂ ; ou un mélange de ces groupes;
R2 étant tel que défini précédemment pour le groupe de jonction (A).

22. Composition selon l'une des revendications précédentes, dans laquelle le monomère (a) est de formule : 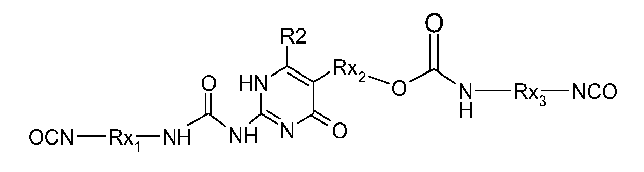 dans laquelle Rx₁, Rx₂, Rx₃ identiques ou différents, représentent un radical carboné divalent choisi parmi un groupe alkyle linéaire ou ramifié en C₁-C₃₀ ou un groupe cycloalkyle en C₄-C₁₂ ou un groupe aryle en C₄-C₁₂; ou leur mélanges,
et R2 est un radical alkyle en C1-C32.

23. Composition selon l'une des revendications précédentes, dans laquelle le monomère (a) est de formule : 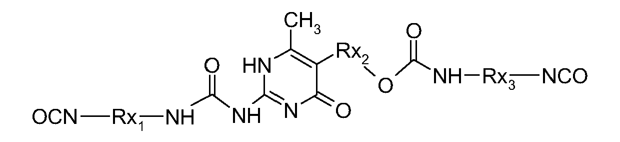 et les radicaux Rx1, Rx2 et Rx3, indépendamment l'un de l'autre sont choisis parmi les radicaux suivants : méthylène, 1,2-éthylène, 1,6-héxylène, 1,4-butylène, 1,6-(2,4,4-triméthylhexylène), 1,4-(4-méthylpentylène), 1,5-(5-méthylhexylène); 1,6-(6-méthylheptylène); 1,5-(2,2,5-triméthylhexylène), 1,7-(3,7-diméthyloctylène); 4,4'-méthylènebiscyclohexylène; 2-méthyl-1,3-phénylène; 4-méthyl-1,3-phénylène ; 4,4'-biphénylèneméthylène, 1,2-tolylène, 1,4-tolylène, 2,4-tolylène, 2,6-tolylène; 1,5-naphtylène; 4,4'-methylenebis(phényl); tétraméthyl xylylène; le radical divalent dérivé de l'isophorone.

24. Composition selon l'une des revendications précédentes, dans laquelle le monomère (a) est le suivant :

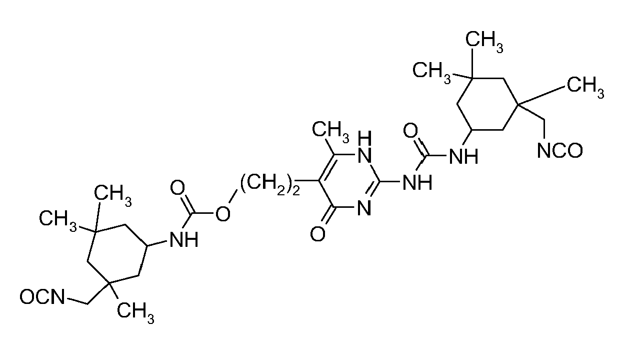

25. Composition selon l'une des revendications précédentes, dans laquelle le monomère (b1) est de formule HX-R^{b1}-X'H, avec :
- X et X', identiques ou différents, choisis parmi O, S, NH ou NR, R représentant un groupe alkyle en C₁-C₆. ; de préférence, X et/ou X' = O et préférentiellement X=X'=O;
- R^{b1} représente un radical divalent polymérique, notamment de type homopolymère ou copolymère, comme par exemple choisi parmi les copolymères fonctionnels dithiol, diamines, diols, aminoalcool, les fonctions alcool, amine et/ou thiol étant portées aux extrémités de la chaîne ou le long du squelette.

26. Composition selon la revendication 25, dans laquelle R^{b1} est un radical polymérique choisi parmi les radicaux de type :
- polyéthers, perfluorés ou non, de type polyoxyde d'éthylène, polyoxyde de propylène, et leurs copolymères polyoxyde d'éthylène/polyoxyde de propylène, les polyoxydes de tétraméthylène; les perfluoropolyéthers et les polythioéthers;
- polylactides;
- polyesters, notamment à base d'acide adipique ou d'acide téréphtalique; en particulier la polycaprolactone; le poly(2-méthyl-1,3-propylène-adipate), le poly-(2-méthyl-1,3-propylène)-glutarate; les polyesters sulfoniques;
- polyamides ;
- polyoxazolines tels que la poly(2-méthyloxazoline), la poly(2-éthyloxazoline);
- les copolymères de siloxane, tels que par exemple les polysiloxanes constitués de motifs - Si(R⁴)(R⁵)O- où (R⁴) et (R⁵), identiques ou différents, représentent H ou un radical carboné, linéaire ou ramifié, cyclique ou non, saturé ou non, ou aromatique, de préférence un alkyle en C₁ - C₁₂ pouvant éventuellement comporter un ou plusieurs, de préférence 1 à 5 hétéroatomes, identiques
ou différents, choisis parmi : O, N, S, P, F et Si, de préférence O, N et S, et notamment les polydiméthylsiloxanes (PDMS), et les poly(méthylphénylsiloxanes) ;
- les polyacétals ;
- les copolymères éthyléniques et notamment les copolymères (méth)acryliques, les copolymères (méth)acrylamides, les copolymères vinyliques, les copolymères allyliques; ainsi conviennent à l'invention les copolymères vinyliques/(méth)acrylates, vinyliques/(méth)acrylamides, vinyliques/(méth)acrylates/méthacrylamides, oléfiniques/vinyliques et les (méth)acrylates /(méth)acrylamides;
- les polyoléfines comportant des motifs choisis parmi 1,2-butadiène; 1,4-butadiène; isoprène; éthylène; propylène; 1,2-butylène, 1,4-butylène; isobutylène;
- les polycarbonates perfluorés ou non ;
- les copolymères de ces différents types de polymères comme par exemple les copolymères polysiloxane / polyoxyde d'éthylène,
- et leurs mélanges.

27. Composition selon l'une des revendications précédentes, dans laquelle le monomère (b1) a une masse molaire moyenne en poids (Mw) comprise entre 500 et 30 000, plus particulièrement entre 700 et 25 000 et mieux de 800 à 15 000.

28. Composition selon l'une des revendications 25 à 27, dans laquelle R^{b1} est choisi parmi les polymères fonctionnels de type :
- polyesters, plus particulièrement à base d'acide adipique et/ou d'acide téréphtalique; et notamment le poly(2-méthyl-1,3-propylène-adipate) et le poly-(2-méthyl-1,3-propylène)glutarate;
- polyéthers et notamment les polyoxydes de tétraméthylène,
- les copolymères de siloxane; et
- les poly(éthylène-butylène) et les polybutadiènes.

29. Composition selon l'une des revendications précédentes, dans laquelle le monomère (b2) est de formule HX-R^{b2}-X'H, avec :
- X et X', identiques ou différents, choisis parmi O, S, NH ou NR, R représentant un groupe alkyle en C₁-C₆; de préférence, X et/ou X' = O et préférentiellement X=X'=O.
- le radical divalent R^{b2} représente un radical carboné divalent ayant de 1 à 40 atomes de carbone, ramifié ou non, cyclique ou non, saturé ou non, aromatique
ou non, comprenant éventuellement un ou plusieurs hétéroatomes choisi(s) parmi O, S, P et N, et/ou éventuellement substitué par un ou plusieurs atomes de fluor et/ou silicium.

30. Composition selon la revendication 29, dans laquelle R^{b2} comprend :
- un radical alkylène ayant 1 à 40 atomes de carbone ou cycloalkylène ayant 3 à 16 atomes de carbone, éventuellement substitué, par un radical alkyle en C₁-C₁₂ comprenant éventuellement 1 à 8 hétéroatomes choisis parmi O, N, S, F, Si et P tel que méthylène, éthylène, propylène, n-butylène, isobutylène, tertiobutylène, n-hexylène, n-octylène, n-dodécylène, n-octadécylène, n-tétradécylène, n-docosanylène; éthyl-2-hexylène, cyclohexylène, cyclohexyleméthylène, isophorone;
- un radical arylène en C₁ à C₃₀ tel qu'un radical phénylène -C₆H₄-(ortho, méta
ou para) éventuellement substitué par un radical alkyle en C₁-C₁₂ comprenant éventuellement 1 à 25 hétéroatomes choisis parmi O, N, S, F, Si et P;
- un radical benzylène -C₆H₄-CH₂- éventuellement substitué par un radical alkyle en C₁-C₁₂ comprenant éventuellement 1 à 8 hétéroatomes choisis parmi O, N, S, F, Si et P;
- un radical alkylarylène ou arylalkylène en C₁ à C₃₀, de préférence en C₂ à C₁₂,
- un radical de formule : -O-CO-O-, -CO-O-, -OCO -, -O-CO-NH-, anhydride, - NH-CO-NH-, NHCO;
- un radical -Si(R⁴)(R⁵)O- où R⁴ et R⁵, identiques ou différents, représentent H ou un radical hydrocarboné, linéaire ou ramifié, cyclique ou non, saturé ou non, ou aromatique, de préférence un alkyle en C₁-C₁₂ pouvant éventuellement comporter un ou plusieurs, de préférence 1 à 5, hétéroatomes identiques ou différents choisis parmi O, N, S, P, F et Si, de préférence O, N et S ;
- un radical oxyalkylène ou aminoalkylène, notamment un radical oxyde d'alkylène de formule -(R"'O)_{y}R^{iv} avec R"' représentant un alkyle linéaire ou ramifié en C₂-C₄, R^{iv} est l'hydrogène ou un radical alkyle, linéaire ou ramifié, en C₁à C₃₀ et y est compris entre 1 et 500 inclus, de préférence de 1 à 250 ;
- un mélange de ces radicaux.

31. Composition selon l'une des revendications 29 à 30, dans laquelle R^{b2} est choisi parmi :
- un radical alkylène ayant 1 à 40 atomes de carbone ou cycloalkylène ayant 3 à 16 atomes de carbone, éventuellement substitué, par un radical alkyle en C₁-C₁₂ comprenant éventuellement 1 à 8 hétéroatomes choisis parmi O, N, S, F, Si et P tel que méthylène, éthylène, propylène, n-butylène, n-pentylène, isobutylène, tertiobutylène, n-hexylène, n-octylène, n-dodécylène, n-octadécylène, n-tétradécylène, n-docosanylène; éthyl-2-hexylène, cyclohexylène, cyclohexyleméthylène, isophorone;
- un radical arylène en C₁ à C₃₀ tel qu'un radical phénylène -C₆H₄-(ortho, méta
ou para) éventuellement substitué par un radical alkyle en C₁-C₁₂ comprenant éventuellement 1 à 25 hétéroatomes choisis parmi O, N, S, F, Si et P;
- un radical alkylarylène ou arylalkylène en C₁ à C₃₀, de préférence en C₂ à C₁₂, et notamment un radical benzylène -C₆H₄-CH₂- éventuellement substitué par un radical alkyle en C₁-C₁₂ comprenant éventuellement 1 à 8 hétéroatomes choisis parmi O, N, S, F, Si et P;
- un radical oxyalkylène ou aminoalkylène, notamment un radical oxyde d'alkylène de formule -(R"'O)_{y}R^{iv} avec R"' représentant un alkyle linéaire ou ramifié en C₂-C₄, R^{iv} est l'hydrogène ou un radical alkyle, linéaire ou ramifié, en C₁à C₃₀ et y est compris entre 1 et 500 inclus, de préférence de 1 à 250 ;
- un mélange de ces radicaux.

32. Composition selon l'une des revendications 29 à 31, dans laquelle R^{b2} est choisi parmi :
- un radical alkylène ayant 1 à 12 atomes de carbone ou cycloalkylène ayant 3 à 6 atomes de carbone, éventuellement substitué, par un radical alkyle en C₁-C₁₂ tel que méthylène, éthylène, propylène, n-butylène, isobutylène, tertiobutylène, n-pentylène, n-hexylène, n-octylène, n-dodécylène, n-octadécylène, cyclohexylène, cyclohexyleméthylène, isophorone;
- un radical alkylarylène ou arylalkylène en C₁ à C₃₀, de préférence en C₂ à C₁₂, éventuellement substitué par un radical alkyle en C₁-C₁₂ tel que par exemple un radical benzylène -C₆H₄-CH₂- ou benzylène diméthylène.

33. Composition selon l'une des revendications précédentes, dans laquelle le monomère (b2) a une masse molaire moyenne en poids (Mw) comprise entre 60 et 1000, plus particulièrement entre 70 et 700 et mieux de 80 à 500.

34. Composition selon l'une des revendications précédentes, dans laquelle le monomère (b2) est choisi parmi :
- l'aminoéthanol, l'aminopropanol; le 4-aminobutanol; le 1-éthylaminobutan-2-ol; l'amino-2-méthyl-2-propanol; le méthyl-4-amino-4-pentan-2-ol ;
- la 1,2-éthylènediamine; la 1,2-propylènediamine; la 1,3-propylènediamine; le 1,4-diaminobutane; le 1,5-diaminopentane; le 1,6-diaminohexane; la 2,6-toluènediamine;
- le 1,4-butanediol; le 1,6-hexanediol; le 1,2-éthanediol; le 1,2-propanediol; le 1,3-propanediol;
- le néopentylglycol; le di(éthylèneglycol) de formule HO-(CH₂CH₂O)₂H; les poly(oxyde d'éthylène)dihydroxylés ; les poly(oxyde de propylène) dihydroxylés; les copolymères poly(oxyde d'éthylène /oxyde de propylène) dihydroxylés ;
- le 1,2-benzènediméthanol, le 1,4-benzènediméthanol; le 1,4-diméthylolcyclohexane ;
- le 1,2-benzènethiol;
- leurs mélanges.

35. Composition selon l'une des revendications 1 à 29, dans laquelle le monomère (b2) comprend au moins un groupe de jonction (A), et est choisi parmi les composés de formule : 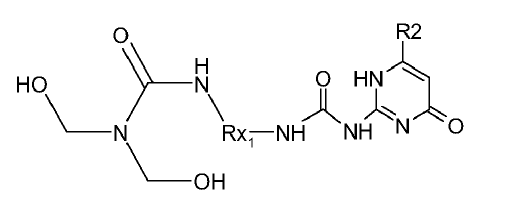 dans laquelle R2 et Rx1 sont tels que définis précédemment.

36. Composition selon la revendication 1, dans laquelle le copolymère comprend au moins un groupe ionisable.

37. Composition selon la revendication 36, dans laquelle les groupes ionisables sont choisis parmi, seuls ou en mélange, :
-i) les groupes anionisables et leurs sels, tels que les groupes comportant une fonction acide choisie parmi :
- le radical carboxylique : -COOH,
- le radical sulfonique : -SO₃H,
- le radical : -OSO₃H
- le radical phosphonique : -(O)P(OH)₂
- le radical phosphorique: -OP(O)(OH)₂
- ou leurs formes salifiées organiques ou minérales;
-ii) les groupes cationisables et leurs sels, tels que les groupes comportant une fonction choisie parmi :
-a) les radicaux amine de formule -N(R^{v})(R^{vi}) ou leurs sels organiques ou minéraux, avec R^{v} et R^{vi} représentant, indépendamment l'un de l'autre :
(i) un atome d'hydrogène,
(ii)un groupement alkyle linéaire, ramifié ou cyclique, saturé ou insaturé, éventuellement aromatique, comprenant de 1 à 30 atomes de carbone et pouvant comprendre 1 à 10 hétéroatomes choisis parmi O, N, S, et P, notamment un groupement méthyle, éthyle, propyle, isopropyle, n-butyle, t-butyle, isobutyle, lauryle, stéaryle;
(iii) un groupement oxyde d'alkylène de formule -(R^{Vii}O)ᵣR^{Viii} avec R^{vii} représentant un alkyle linéaire ou ramifié en C₂-C₄, R^{viii} est l'hydrogène ou un radical alkyle, linéaire ou ramifié, en C₁ à C₃₀ et r est compris entre 1 et 250 inclus ;
(iv) R^{v} et R^{vi} peuvent former avec l'atome d'azote un cycle saturé ou insaturé, éventuellement aromatique, comprenant au total 5, 6, 7 ou 8 atomes, et notamment 4, 5 ou 6 atomes de carbone et/ou 2 à 4 hétéroatomes choisi parmi O, S et N; ledit cycle pouvant en outre être fusionné avec un ou plusieurs autres cycles, saturés ou insaturés, éventuellement aromatiques, comprenant chacun 5, 6 ou 7 atomes, et notamment 4, 5, 6 ou 7 atomes de carbone et/ou 2 à 4 hétéroatomes choisi parmi O, S et N ;
-b) un groupement -R^{ix}-N-R^{x}- dans lequel R^{ix} et R^{x} forment avec l'atome d'azote un cycle, saturé ou insaturé, éventuellement aromatique, comprenant au total 5, 6, 7 ou 8 atomes, et notamment 4, 5 ou 6 atomes de carbone et/ou 2 à 4 hétéroatomes choisi parmi O, S et N; ledit cycle pouvant être fusionné avec un ou plusieurs autres cycles, saturés ou insaturés, éventuellement aromatiques, comprenant chacun 5, 6 ou 7 atomes, et notamment 4, 5, 6, 7 ou 8 atomes de carbone et/ou 2 à 4 hétéroatomes choisi parmi O, S et N, par exemple, le groupe ionisable peut constituer un cycle aromatique ou non comportant un groupement amine tertiaire cationisable ou peut représenter un hétérocycle aromatique ou non, contenant un azote tertiaire, cationisable.
-c) un groupement guanidino ou amidino respectivement de formule : 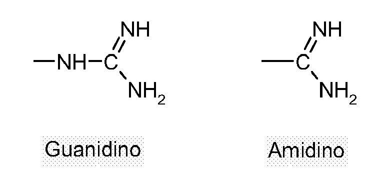
-d) les groupements ammonium quaternaire de formule : 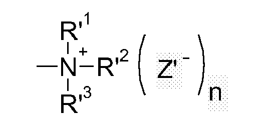 dans laquelle :
les groupes R'¹, R'² et R'³ désignent, indépendamment l'un de l'autre, un groupe alkyle linéaire ou ramifié en C₁ à C₂₀,
n désigne un nombre allant de 0 à 1,
et Z'- représente un ion halogénure tel que Br ou Cl⁻ (n=1), ou encore un ion CH₃SO₃⁻ ou un ion sulfate SO₄²⁻ (n = 1/2) ou leurs mélanges;
- e) et leurs mélanges.

38. Composition selon l'une des revendications 36 à 37, dans laquelle le groupe ionisable est choisi parmi :
- les groupes anionisables suivants: les groupes monovalents -COOH, - CH₂COOH, -(CH₂)₂COOH, -(CH₂)₃COOH, -(CH₂)SO₃H, -(CH₂)₂SO₃H, - (CH₂)₃SO₃H, -O(CH₂)₃SO₃H; les groupes divalents -C(COOH)(CH₃)- et -CH₂-C(COOH)(CH₃)-CH₂- ; pour lesquels les neutralisants peuvent être choisis parmi NaOH, KOH, Ca(OH)₂, NH₄OH, la triéthylamine, la butylamine, l'amino-2-méthyl-2-propanol, la triéthanolamine, la diméthylamino-2-propanol, la lysine ou la 3-(diméthylamino)propylamine;
- les groupes cationisables suivants: les groupes monovalents -(CH₂)₂-N(CH₃)₂,
- N(CH₃)₂, -(CH₂)₃-N(CH₃)₂, -O-(CH₂)₃-N(CH₂CH₃)₂, -(CH₂)₂-N(CH₂CH₃)₂; les groupes divalents -(CH₂)₂-N(CH₃)-(CH₂)₂- et -(CH₂)₃-N(CH₃)-(CH₂)₃- ; pour lesquels les neutralisants peuvent être choisis parmi HCl, l'acide propionique, l'acide acétique, l'acide citrique et l'acide tartrique.

39. Composition selon l'une des revendications 36 à 38, dans laquelle le groupe ionisable est présent sur au moins une partie des monomères de type (b1) et/ou (b2), et/ou sur au moins une partie des groupes de jonction (A), préférentiellement sur au moins une partie des monomères (b2).

40. Composition selon l'une des revendications 36 à 39, dans laquelle l'un au moins des groupes de jonction (A) porte au moins un groupe ionisable; en particulier, l'un au moins des radicaux R¹, R² et/ou R³ desdits groupes de jonction (A) porte un groupe ionisable.

41. Composition selon l'une des revendications 36 à 39, dans laquelle au moins une partie des monomères (b1) porte le groupe ionisable et est choisi parmi les polyesters sulfoniques ou les copolymères éthyléniques à base d'anhydride maléique.

42. Composition selon l'une des revendications 36 à 39, dans laquelle au moins une partie des monomères (b2) porte le groupe ionisable.

43. Composition selon la revendication 42, dans laquelle le monomère (b2) portant le groupe ionisable est choisi parmi les monomères anionisables comportant des fonctions carboxylique (-COOH), et/ou sulfonique (-SO₃H), notamment ceux de formule : 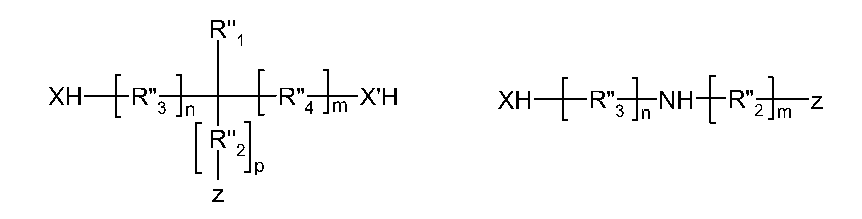 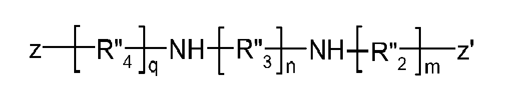 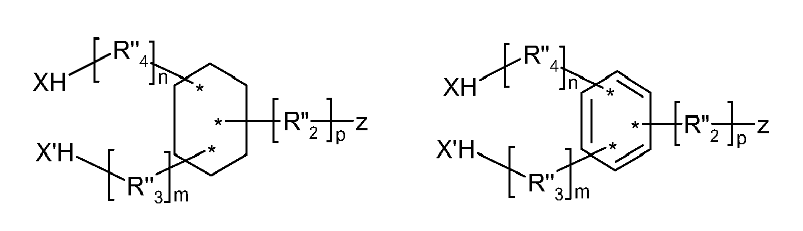 dans lesquelles :
- R"₁ représente H ou un groupe alkyle ayant de 1 à 40 atomes de carbone, ramifié ou non, cyclique ou non, saturé ou non, aromatique ou non, et/ou comprenant éventuellement un ou plusieurs hétéroatomes choisi(s) parmi O, S, P et N, et/ou éventuellement substitué par un ou plusieurs atomes de fluor ou silicium ; le ou les hétéroatomes, quand ils sont présents, peuvent être intercalés dans la chaîne dudit groupe, ou bien ledit groupe peut être substitué par un ou plusieurs groupes les comprenant tels que les groupes hydroxyle ou amino (NH₂, NHR' ou NR'R" avec R' et R" identiques ou différents représentant un alkyle linéaire ou ramifié en C₁-C₂₂, notamment méthyle ou éthyle);
- R"₂, R"₃ et R"₄, identiques ou différents, représentent un groupe alkylène (alkyle divalent) ayant de 1 à 40 atomes de carbone, ramifié ou non, cyclique ou non, saturé ou non, aromatique ou non, et/ou comprenant éventuellement un ou plusieurs hétéroatomes choisi(s) parmi O, S, P et N, et/ou éventuellement substitué par un ou plusieurs atomes de fluor ou silicium ; le ou les hétéroatomes, quand ils sont présents, peuvent être intercalés dans la chaîne dudit groupe, ou bien ledit groupe peut être substitué par un ou plusieurs groupes les comprenant tels que les groupes ester, amide, hydroxyle ou amino (NH₂, NHR' ou NR'R" avec R' et R" identiques ou différents représentant un alkyle linéaire ou ramifié en C₁-C₂₂, notamment méthyle ou éthyle).
- X et X', identiques ou différents, représentent O, S, NH, NR où R est un radical alkyle linéaire ou ramifié, en C₁-C₆; de préférence X et X' représentent O ;
- Z, Z', identiques ou différents, représentent une fonction acide carboxylique (-COOH) ou acide sulfonique (-SO₃H).
- n, p, m et q sont, indépendamment l'un de l'autre, égal à 0 ou 1.

44. Composition selon la revendication 43, dans laquelle les monomères anionisables (b2) sont choisis parmi les composés de formule: 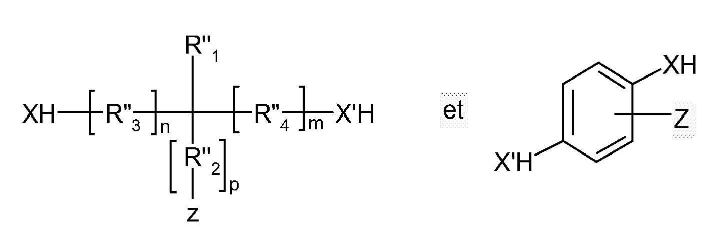 dans lesquelles X, X', R"1, R"2, R"3, R"4, m, n, p et Z sont tels que définis dans la revendication 43, et plus particulièrement les composés de formule : 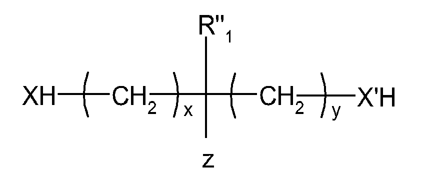 pour lesquels R"1 représente un groupe alkyle ayant de 1 à 22 atomes de carbone, de préférence CH₃, et x et y identiques ou différents, sont compris entre 1 et 5 inclus.

45. Composition selon la revendication 44, dans laquelle les monomères anionisables (b2) sont choisis parmi l'acide diméthylolpropionique, l'acide diméthylaminopropionique, la N-éthylsulfonique-diméthanolamine, la N-éthylsulfonique-diéthanolamine, l'acide diolbenzène sulfonique.

46. Composition selon la revendication 42, dans laquelle le monomère (b2) portant le groupe ionisable est choisi parmi les monomères cationisables ou amphotères, de formule : 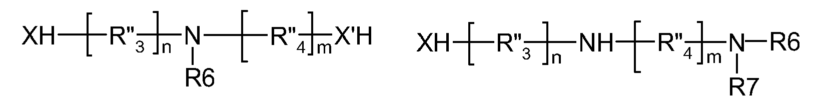 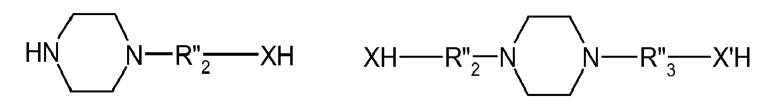 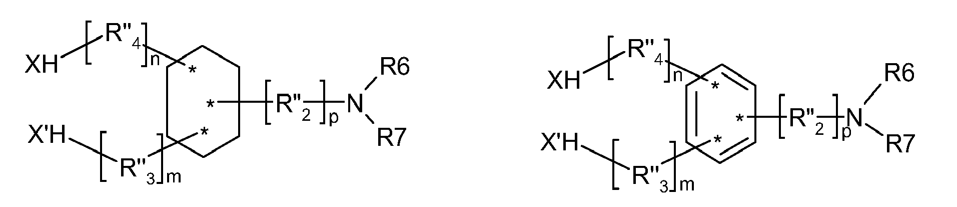 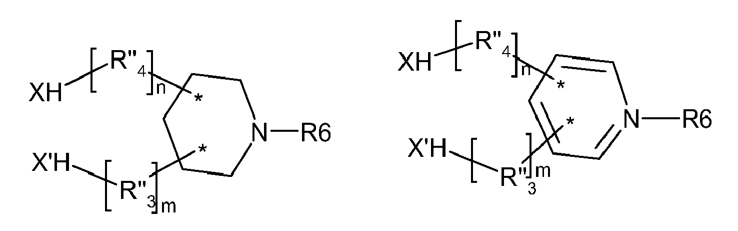 dans lesquelles :
- R"₂, R"₃ et R"₄, identiques ou différents, représentent un groupe alkylène (alkyle divalent) ayant de 1 à 40 atomes de carbone, ramifié ou non, cyclique ou non, saturé ou non, aromatique ou non, et/ou comprenant éventuellement un ou plusieurs hétéroatomes choisi(s) parmi O, S, P et N, et/ou éventuellement substitué par un ou plusieurs atomes de fluor ou silicium ; le ou les hétéroatomes, quand ils sont présents, peuvent être intercalés dans la chaîne dudit groupe, ou bien ledit groupe peut être substitué par un ou plusieurs groupes les comprenant tels que les groupes ester, amide, hydroxyle ou amino (NH₂, NHR' ou NR'R" avec R' et R" identiques ou différents représentant un alkyle linéaire ou ramifié en C₁-C₂₂, notamment méthyle ou éthyle).
- X et X', identiques ou différents, représentent O, S, NH, NR où R est un radical alkyle linéaire ou ramifié, en C₁-C₆ ; de préférence X et X' représentent O ;
- n, p et m sont, indépendamment l'un de l'autre, égal à 0 ou 1;
- R₆ et R₇ représentent un groupe alkyle en C₁ à C₂₂, linéaire ou ramifié, de préférence méthyl, éthyl, lauryl, béhényl.

47. Composition selon la revendication 46, dans laquelle le monomère (b2) cationisable est choisi parmi les monomères de formule : 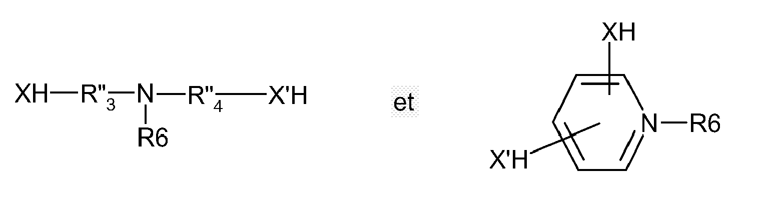 dans lesquelles X, X', R"3, R"4 et R6 sont définis dans la revendication 46.

48. Composition selon la revendication 47, dans laquelle le monomère (b2) cationisable est choisi parmi la N-méthyldiéthanolamine, la N-tert-butyldiéthanolamine, la N-éthyldiéthanolamine et la diaminopyridine.

49. Composition selon la revendication 42, dans laquelle le monomère (b2) cationisable ou amphotère est de formule : 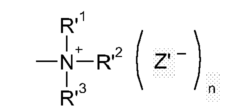 dans laquelle R'1, R'2n R'3 et Z' et n sont tels que définis ci-dessus.

50. Composition selon l'une des revendications 42 à 49, dans laquelle :
- le groupe ionisable représente 0,1 à 50% en poids du poids total du copolymère selon l'invention, de préférence 0,5 à 35% en poids, et notamment 1 à 15% en poids par rapport au poids total du polymère; et/ou
- le ou les monomères portant le/les groupes ionisables représentent 3 à 20% en poids, de préférence 6 à 17% en poids, voire 8 à 15% en poids, du poids total du copolymère final.

51. Composition selon l'une des revendications précédentes, dans laquelle le copolymère comprend, à titre optionnel, des monomères de structure YCN-R₁₀ avec Y représentant O ou S; de préférence O; et R₁₀ représentant un radical carboné, notamment alkyle, ayant de 1 à 40 atomes de carbone, linéaire ou ramifié, cyclique ou non, saturé ou insaturé, aromatique ou non, comprenant éventuellement un ou plusieurs hétéroatomes choisi(s) parmi O, S, P et N, et/ou éventuellement substitué par un ou plusieurs atomes de fluor ou silicium.

52. Composition selon la revendication 51, dans laquelle R₁₀ porte au moins un groupe de jonction (A).

53. Composition selon l'une des revendications 50 à 51, dans laquelle YCN-R₁₀ est de formule : 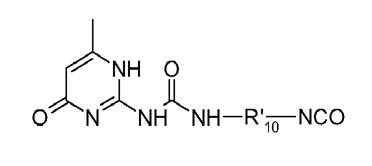 dans laquelle R'10 représente un radical carboné divalent choisi parmi un groupe alkyle linéaire ou ramifié en C₁-C₃₀, un groupe cycloalkyle en C₄-C₁₂ ou un groupe aryle en C₄-C₁₂; comprenant éventuellement mais non préférentiellement, 1 à 8 hétéroatomes choisis parmi O, N, S, F, Si et P; ainsi que les mélanges de ces significations.

54. Composition selon l'une des revendications précédentes, dans laquelle le copolymère comprend, à titre optionnel, des monomères de structure HX-R₁₁ avec :
- X représentant O, S, NH ou NR, R représentant un groupe alkyle en C₁-C₆; de préférence, X = O ; et
- R₁₁ représentant un radical carboné, notamment alkyle, ayant de 1 à 40 atomes de carbone, linéaire ou ramifié, cyclique ou non, saturé ou insaturé, aromatique ou non, comprenant éventuellement un ou plusieurs hétéroatomes choisi(s) parmi O, S, P et N, et/ou éventuellement substitué par un ou plusieurs atomes de fluor ou silicium; le ou les hétéroatomes, quand ils sont présents, peuvent être intercalés dans la chaîne dudit radical, ou bien ledit radical pouvant être substitué par un ou plusieurs groupes les comprenant tels que les groupes esters et/ou amides.

55. Composition selon l'une des revendications précédentes, dans laquelle le copolymère a un squelette polymérique ayant un degré de polymérisation allant de 3 à 20000, et de préférence de 5 à 10000 et mieux de 10 à 5000.

56. Composition selon l'une des revendications précédentes, dans laquelle le copolymère a une masse moléculaire moyenne en nombre Mn comprise entre 1 000 et 3 000 000, de préférence entre 5000 et 1 000 000, mieux entre 8 000 et 500 000.

57. Composition selon l'une des revendications précédentes, dans laquelle le rapport molaire entre la totalité des monomères de formule YCN-Ra-NCY' et la totalité des monomères de formule HX-Rb-X'H (à savoir les monomères (b1) et (b2)) peut être de 0,5 à 2, de préférence de 0,6 à 1,5 et mieux de 0,7 à 1,3; préférentiellement, ce rapport est de 1.

58. Composition selon l'une des revendications précédentes, dans laquelle les monomères de type HX-R^{b1}-X'H représentent 10 à 95% en poids, notamment 12 à 85% en poids, préférentiellement 15 à 80% en poids, du poids total du polymère final.

59. Composition selon l'une des revendications précédentes, dans laquelle les monomères de type HX-R^{b2}-X'H représentent 1 à 30% en poids, notamment 2 à 25% en poids, préférentiellement 3 à 20% en poids, du poids total du polymère final.

60. Composition selon l'une des revendications précédentes, dans laquelle le copolymère est présent sous forme soluble ou dispersible dans le milieu physiologiquement acceptable.

61. Composition selon l'une des revendications précédentes, dans laquelle le milieu physiologiquement acceptable comprend un milieu solvant des copolymères comprenant au moins un composé choisi parmi l'eau, les alcools, les polyols, les esters, les huiles carbonées, les huiles de silicone, les huiles de silicone fluorées, et leurs mélanges.

62. Composition selon l'une des revendications précédentes, dans laquelle le milieu physiologiquement acceptable comprend en outre au moins un constituant choisi parmi les cires, les gommes, les tensioactifs, les épaississants, les gélifiants hydrophiles ou lipophiles, les actifs cosmétiques, les conservateurs, les antioxydants, les parfums, les agents nacrants, les charges, les neutralisants, les émulsionnants, les co-émulsionnants, les pigments, les nacres, les colorants hydrosolubles, les colorants liposolubles, les polymères notamment filmogènes.

63. Composition selon l'une des revendications précédentes, dans laquelle les copolymères, seuls ou en mélange, sont présents en une quantité de 0,01 % à 90% en poids, de préférence de 0,1 à 85% en poids, et mieux de 0,5 à 80% en poids, par rapport au poids total de la composition.

64. Composition selon l'une des revendications précédentes, se présentant sous forme d'une composition cosmétique comprenant un milieu cosmétiquement acceptable.

65. Composition selon l'une des revendications précédentes, se présentant sous la forme d'un produit de soin, de nettoyage et/ou de maquillage de la peau du corps ou du visage, des lèvres, des cils, des ongles et des cheveux; d'un produit solaire ou autobronzant; d'un produit d'hygiène corporelle; d'un produit capillaire, notamment de soin, de nettoyage, de coiffage, de mise en forme, ou de coloration des cheveux.

66. Procédé de traitement cosmétique, notamment de maquillage, de soin, de nettoyage ou de coloration, des matières kératiniques, notamment de la peau du corps ou du visage, des ongles, des cils, des cheveux, comprenant l'application sur lesdites matières d'une composition cosmétique telle que définie à l'une des revendications précédentes.

67. Procédé pour améliorer à la fois la persistance d'au moins un effet apporté après dépôt par une composition cosmétique et l'adhésion de ladite composition cosmétique appliquée sur les matières kératiniques, consistant à ajouter à ladite composition une quantité efficace d'au moins un copolymère tel que défini à l'une des revendications 1 à 59.
